# EUROPEAN PATENT APPLICATION

(11) **EP 4 821 950 A1**
(43) Date of publication of application: **14.10.2026**
(21) Application number: 24899124.2
(22) Date of filing: 29.04.2024
(51) Int. Cl.: C07K 16/46, C07K 19/00, C12N 15/62, C12N 15/85, C12N 5/10, C12N 15/13, A61K 47/68, A61K 39/395, A61K 45/06

(54) **ANTI-MET/EGFR BISPECIFIC ANTIBODY AND DRUG CONJUGATE THEREOF**

(30) Priority: 06.12.2023 CN 202311671052
(71) Applicant: Novatim Immune Therapeutics (Zhejiang) Co., Ltd, Huzhou, Zhejiang 313399 (CN)
(72) Inventor: WU, Guoxiang, Huzhou, Zhejiang 313399 (CN); CAI, Xianghai, Huzhou, Zhejiang 313399 (CN); HONG, Yu, Huzhou, Zhejiang 313399 (CN); JIANG, Yingying, Huzhou, Zhejiang 313399 (CN); ZHANG, Chao, Huzhou, Zhejiang 313399 (CN)
(74) Representative: Lavoix
(86) International application number: PCT/CN2024/090671
(87) International publication number: WO 2025/118472

(57) **Abstract**

Provided is an anti-MET nanobody and an anti-EGFR nanobody, and a bispecific antibody comprising the two nanobodies. Also provided is a drug conjugate constructed on the basis of the nanobodies and the bispecific nanobody.

## Description

### Technical field

The present invention relates to the field of biomedicine. Particularly, the present invention relates to an anti-MET/EGFR bispecific antibody and a drug conjugate thereof.

### Background

Currently, ADC (Antibody-Drug Conjugate) technology is a hotspot in the research of cancer treatment. It utilizes monoclonal antibodies to specifically bind to targets, deliver small-molecule drugs to lesion tissues, and release these drugs to exert therapeutic effects, thereby killing tumor cells and treating cancers. Tumor genesis is associated with multiple factors, single-target therapies are prone to causing drug resistance and damage to normal tissue cells. Therefore, single-target ADCs cannot fully meet the needs of cancer treatment, and there is an urgent need to develop dual-target ADC drugs to reduce toxicity to normal tissues and alleviate drug resistance.

MET (Mesenchymal-Epithelial Transition Factor) and EGFR (Epidermal Growth Factor Receptor) are classic targets in tumor therapy, both belonging to receptor tyrosine kinase proteins. They are closely related to tumor development, metastasis, drug resistance, and other pathological processes. MET amplification is one of the factors contributing to tumor cell resistance to EGFR inhibitors. For example, in metastatic colorectal cancer resistant to Cetuximab or Panitumumab, MET gene amplification occurs, and inhibiting MET can effectively suppress tumor growth in Cetuximab-resistant PDX mouse models. Given the association between EGFR and MET in tumor progression, simultaneous inhibition of MET and EGFR has become one of the strategies in anti-tumor drug development.

In existing technologies, almost all ADCs employ traditional antibodies, such as IgG1 and IgG4 conjugated with toxins. Currently, the available MET-EGFR dual-target antibody is Amivantamab, used for treating non-small cell lung cancer with exon 20 insertion mutations in EGFR. AstraZeneca has disclosed the MET-EGFR dual-target ADC AZD9592 in patent application (US20230183358A1), which is currently in Phase 1 clinical trials. Additionally, the MET-EGFR ADC disclosed in Sorrento's patent application WO2018069851A2 is in the preclinical research stage. Although traditional antibodies are widely used and exhibit good targeting capabilities, their large molecular size limits tumor penetration and results in slow distribution. In contrast, nanobodies, composed only of heavy chain dimers including VHH, CH2, and CH3 regions, have smaller molecular weights and theoretically would exhibit stronger diffusion capabilities in tumor tissues, which is beneficial for ADC drugs to enter cells and release toxins.

Furthermore, following deficiencies are also present in existing technologies:
1) Low product purity: currently, the drug-to-antibody ratio (DAR) of commercially available ADC drugs such as Tisotumab Vedotin-tftv, Brentuximab Vedotin, Disitamab Vedotin, and Enfortumab Vedotin-ejfv, mainly ranges from 3 to 5. For these ADCs, cysteine-based methods are used to conjugate antibodies and toxins. Since the used antibodies are traditional IgG1 antibodies which contain four pairs of disulfide bonds in the hinge region, up to 8 toxins can be conjugated using the cysteine conjugation method. As a result, the conjugation mixture includes products of DAR2, DAR4, DAR6, and DAR8, making the process being difficult to be controlled and the products complex. For example, in AbbVie's patent application US20150337042, the EGFR-targeted ADC product exhibits 8 HIC peaks mainly distributed at DAR2 and DAR4, with DAR4 product purity at 35.28%, which increases to 45.88% after purification. The DAR4 product of ABBV-399 disclosed in AU2017268342B2 accounts for only 26%.
2) Toxic side effects: EGFR is widely expressed in normal tissues, with particularly high expression in the skin and thyroid. Therefore, inhibiting EGFR can easily cause damage to normal tissues. Current development of EGFR antibodies and ADCs focuses on reducing affinity and using lower-affinity EGFR antibodies for toxin conjugation to minimize toxicity to normal tissues. However, excessively low affinity may affect killing efficacy, requiring extensive exploration to achieve a balance.

Therefore, there is a need in the field to develop a nanobody-based MET-EGFR dual-target ADC drug with smaller molecular-weight.

### Summary of the invention

The purpose of the present invention is to provide an anti-MET/EGFR bispecific antibody and a drug conjugate thereof.

In the first aspect of the present invention, it provides a nanobody targeting MET, wherein the VHH chain of the nanobody comprises the following complementarity determination regions CDR1, CDR2 and CDR3:
a CDR1 as shown in SEQ ID NO: 13,
a CDR2 as shown in SEQ ID NO: 14, and
a CDR3 as shown in SEQ ID NO: 15.

In another preferred embodiment, the CDR region of the nanobody VHH chain comprises an amino acid sequence having at least 80%, preferably at least 90%, more preferably at least 95% sequence identity to any of the above sequences.

In another preferred embodiment, any one of the above amino acid sequences further includes a derivative sequence which is optionally added, deleted, modified, and/or substituted with at least one amino acid, and can retain the binding affinity to MET.

In another preferred embodiment, the number of added, deleted, modified and/or substituted amino acids is 1-3, preferably 1-2, more preferably 1.

In another preferred embodiment, the VHH chain of the nanobody further comprises a framework region (FR).

In another preferred embodiment, the CDR1, CDR2 and CDR3 are separated by framework regions FR1, FR2, FR3 and FR4 of the VHH chain.

In another preferred embodiment, the framework region (FR) is of human origin, murine origin, rabbit origin or camel origin.

In another preferred embodiment, the nanobody binds to human, murine or cynomolgus MET.

In another preferred embodiment, the nanobody is capable of being internalized by a cell expressing MET antigen.

In another preferred embodiment, the nanobody is capable of blocking the binding between MET and HGF.

In another preferred embodiment, the VHH chain of the nanobody targeting MET has an amino acid sequence having ≥85%, ≥90%, ≥95%, ≥96%, ≥97%, ≥98%, or ≥99% homology to the amino acid sequence shown in SEQ ID NO: 1.

In another preferred embodiment, the VHH chain of the nanobody targeting MET has an amino acid sequence as shown in SEQ ID NO: 1.

In the second aspect of the present invention, it provides an antibody targeting MET, which comprises one or more VHH chain(s) of the nanobody targeting MET according to the first aspect of the present invention.

In another preferred embodiment, the VHH chain of the nanobody targeting MET has an amino acid sequence as shown in SEQ ID NO: 1.

In another preferred embodiment, the antibody is a monomer, a bivalent antibody, and/or a multivalent antibody.

In another preferred embodiment, the antibody is an animal-derived antibody, a humanized antibody, a chimeric antibody, or a chimeric antigen receptor antibody (CAR).

In another preferred embodiment, the CDR region of the humanized antibody comprises changes of 1, 2, or 3 amino acids.

In another preferred embodiment, the animal is a non-human mammal, and preferably is a mouse, a goat, a rabbit or a camel.

In another preferred embodiment, the antibody is a double-chain antibody or a single-chain antibody.

In another preferred embodiment, the antibody is a monoclonal antibody.

In another preferred embodiment, the antibody is a partially or fully humanized antibody.

In another preferred embodiment, the antibody is a heavy chain antibody which comprises heavy chain constant regions CH2 and CH3 (an Fc fragment).

In another preferred embodiment, the heavy chain constant region is derived from an Fc fragment of IgG, and preferably an Fc fragment of human IgG.

In another preferred embodiment, the amino acid sequence of the heavy chain antibody is as shown in SEQ ID NO: 2.

In another preferred embodiment, any one of the above amino acid sequences further includes a derivative sequence which is optionally added, deleted, modified, and/or substituted with at least one amino acid, and can retain the binding affinity to MET.

In another preferred embodiment, the number of added, deleted, modified, and/or substituted amino acids is not more than 40%, preferably 20%, and more preferably 10% of the total number of amino acids in the initial amino acid sequence.

In another preferred embodiment, the antibody binds to human, murine or cynomolgus MET.

In another preferred embodiment, the antibody is capable of being internalized by a cell expressing MET antigen.

In another preferred embodiment, the antibody is capable of blocking the binding between MET and HGF.

In the third aspect of the present invention, it provides a nanobody targeting EGFR, wherein the VHH chain of the nanobody comprises the following complementarity determination regions CDR1, CDR2 and CDR3:
a CDR1 as shown in SEQ ID NO: 16,
a CDR2 as shown in SEQ ID NO: 17, and
a CDR3 as shown in SEQ ID NO: 18.

In another preferred embodiment, the CDR region of the nanobody VHH chain comprises an amino acid sequence having at least 80%, preferably at least 90%, more preferably at least 95% sequence identity to any of the above sequences.

In another preferred embodiment, any one of the above amino acid sequences further includes a derivative sequence which is optionally added, deleted, modified, and/or substituted with at least one amino acid, and can retain the binding affinity to EGFR.

In another preferred embodiment, the number of added, deleted, modified and/or substituted amino acids is 1-3, preferably 1-2, more preferably 1.

In another preferred embodiment, the VHH chain of the nanobody further comprises a framework region (FR).

In another preferred embodiment, the CDR1, CDR2 and CDR3 are separated by framework regions FR1, FR2, FR3 and FR4 of the VHH chain.

In another preferred embodiment, the framework region (FR) is of human origin, murine origin, rabbit origin or camel origin.

In another preferred embodiment, the nanobody binds to human, murine or cynomolgus EGFR.

In another preferred embodiment, the nanobody is capable of being internalized by a cell expressing EGFR antigen.

In another preferred embodiment, the nanobody does not blocking the binding between EGFR and EGF.

In another preferred embodiment, the VHH chain of the nanobody targeting EGFR has an amino acid sequence having ≥85%, ≥90%, ≥95%, ≥96%, ≥97%, ≥98%, or ≥99% homology to the amino acid sequence shown in SEQ ID NO: 3.

In another preferred embodiment, the VHH chain of the nanobody targeting EGFR has an amino acid sequence as shown in SEQ ID NO: 3.

In the fourth aspect of the present invention, it provides an antibody targeting EGFR, which comprises one or more VHH chain(s) of the nanobody targeting EGFR according to the third aspect of the present invention.

In another preferred embodiment, the VHH chain of the nanobody targeting EGFR has an amino acid sequence as shown in SEQ ID NO: 3.

In another preferred embodiment, the antibody is a monomer, a bivalent antibody, and/or a multivalent antibody.

In another preferred embodiment, the antibody is an animal-derived antibody, a humanized antibody, a chimeric antibody, or a chimeric antigen receptor antibody (CAR).

In another preferred embodiment, the CDR region of the humanized antibody comprises changes of 1, 2, or 3 amino acids.

In another preferred embodiment, the animal is a non-human mammal, and preferably is a mouse, a goat, a rabbit or a camel.

In another preferred embodiment, the antibody is a double-chain antibody or a single-chain antibody.

In another preferred embodiment, the antibody is a monoclonal antibody.

In another preferred embodiment, the antibody is a partially or fully humanized antibody.

In another preferred embodiment, the antibody is a heavy chain antibody which comprises heavy chain constant regions CH2 and CH3 (an Fc fragment).

In another preferred embodiment, the heavy chain constant region is derived from an Fc fragment of IgG, and preferably an Fc fragment of human IgG.

In another preferred embodiment, the amino acid sequence of the heavy chain antibody is as shown in SEQ ID NO: 4.

In another preferred embodiment, any one of the above amino acid sequences further includes a derivative sequence which is optionally added, deleted, modified, and/or substituted with at least one amino acid, and can retain the binding affinity to EGFR.

In another preferred embodiment, the number of added, deleted, modified, and/or substituted amino acids is not more than 40% of the total number of amino acids in the initial amino acid sequence, preferably 20%, and more preferably 10%.

In another preferred embodiment, the antibody binds to human, murine or cynomolgus EGFR.

In another preferred embodiment, the antibody is capable of being internalized by a cell expressing EGFR antigen.

In another preferred embodiment, the antibody does not block the binding between EGFR and EGF.

In the fifth aspect of the present invention, it provides a multi-specific antibody which comprises: the VHH chain of the nanobody targeting MET according to the first aspect of the present invention, the antibody targeting MET according to the second aspect of the present invention, the VHH chain of the nanobody targeting EGFR according to the third aspect of the present invention, the antibody targeting EGFR according to the fourth aspect of the present invention, or a combination thereof.

In another preferred embodiment, the multi-specific antibody is a bispecific antibody targeting MET and EGFR.

In another preferred embodiment, the multi-specific antibody comprises one or more antigen-binding chains, and each of the one or more antigen-binding chains independently has the structure shown in formula (I) from N-terminus to C-terminus:

V1-L1-V2-H1-Fc1-L2-V3 (I)

wherein,
V1, V2, and V3 are each independently absent, the VHH chain of the nanobody targeting MET, or the VHH chain of the nanobody targeting EGFR, and at least one of V1, V2, and V3 is the VHH chain of the nanobody targeting MET or EGFR;
L1 and L2 are each independently absent or a linker peptide;
H1 is absent or an immunoglobulin hinge region;
Fc1 is an immunoglobulin Fc fragment;
"-" represents a peptide bond.

In another preferred embodiment, the bispecific antibody is a dimer composed of a first antigen-binding chain and a second antigen-binding chain.

In another preferred embodiment, the bispecific antibody is a homodimer or a heterodimer.

In another preferred embodiment, the bispecific antibody has the structure shown in formula II from N-terminus to C-terminus:

V1-L1-V2-H1-Fc1-L2-V3 I

V4-L3-V5-H2-Fc2-L4-V6 (II);

wherein,
V1, V2, V3, V4, V5, and V6 are each independently absent, the VHH chain of the nanobody targeting MET, or the VHH chain of the nanobody targeting EGFR,
at least one of V1, V2, and V3 is the VHH chain of the nanobody targeting MET, and at least one of V4, V5, and V6 is the VHH chain of the nanobody targeting EGFR;
L1, L2, L3, L4, L5, and L6 are each independently absent or a linker peptide;
H1 and H2 are each independently absent or an immunoglobulin hinge region;
Fc1 and Fc2 are each independently an immunoglobulin Fc fragment;
"∥" represents a disulfide bond or a knob-into-hole connection;
"-" represents a peptide bond.

In another preferred embodiment, the linker peptide is a flexible linker peptide.

In another preferred embodiment, the linker peptide is represented by (G4S)n, wherein n is an integer selected from 1-6, preferably 2, 3, 4 or 5.

In another preferred embodiment, Fc1 and/or Fc2 are derived from an Fc fragment of IgG, preferably from an Fc fragment of human IgG.

In another preferred embodiment, H1 and/or H2 are derived from a hinge region of IgG, preferably from a hinge region of human IgG.

In another preferred embodiment, H1 and/or H2 are truncated by 1-7 amino acids at the N-terminus compared with the hinge region of wild-type immunoglobulin (such as human IgG), for example, 3, 4 or 5 amino acids.

In another preferred embodiment, the sequences of H1 and H2 are each independently as shown in SEQ ID NO: 23 or SEQ ID NO: 24.

In another preferred embodiment, the sequence of H2 is as shown in SEQ ID NO: 24.

In another preferred embodiment, the bispecific antibody has the structure shown in the following formula IIa from N-terminus to C-terminus:

V1-L1-V2-H1-Fcl ∥

V4-L3-V5-H2-Fc2 (IIa)

wherein,
one of V1 and V2 is the VHH chain of the nanobody targeting MET, and the other is absent or the VHH chain of the nanobody targeting EGFR;
one of V4 and V5 is the VHH chain of the nanobody targeting EGFR, and the other is absent or the VHH chain of the nanobody targeting MET;
the remaining elements are as defined in formula (II).

In another preferred embodiment, V1 is the VHH chain of the nanobody targeting MET, and V2 is absent.

In another preferred embodiment, V1 is the VHH chain of the nanobody targeting MET, and V2 is the VHH chain of the nanobody targeting EGFR.

In another preferred embodiment, V4 is the VHH chain of the nanobody targeting EGFR, and V5 is the VHH chain of the nanobody targeting MET.

In another preferred embodiment, V4 is the VHH chain of the nanobody targeting MET, and V5 is the VHH chain of the nanobody targeting EGFR.

In another preferred embodiment, the bispecific antibody has the structure shown in the following formula IIb from N-terminus to C-terminus:

V1-H1-Fc1 II

V4-H2-Fc2 (IIb)

wherein,
V1 is the VHH chain of the nanobody targeting MET;
V4 is the VHH chain of the nanobody targeting EGFR;
the remaining elements are as defined in formula (II).

In another preferred embodiment, the bispecific antibody has the structure shown in the following formula IIc from N-terminus to C-terminus:

V1-H1-Fc1-L2-V3 ∥

V4-H2-Fc2-L4-V6 (IIc)

wherein,
V1 and V4 are each independently the VHH chain of the nanobody targeting MET;
V3 and V6 are each independently absent or the VHH chain of the nanobody targeting EGFR, and at least one of V3 and V6 is the VHH chain of the nanobody targeting EGFR;
the remaining elements are as defined in formula (II).

In another preferred embodiment, the amino acid sequence of the antigen-binding chain is selected from the group consisting of:
(a) the amino acid sequence shown in any one of SEQ ID NOs: 2, 5-12;
(b) a derivative sequence formed by adding one or more amino acids, substituting one or more amino acids, or deleting 1-30 amino acids in the amino acid sequence of (a), wherein the derivative sequence retains the antigen-specific binding ability of its source sequence.

In another preferred embodiment, the amino acid sequence of the first antigen-binding chain is selected from the group consisting of:
(a) the amino acid sequence shown in any one of SEQ ID NOs: 2, 6, 11 and 12;
(b) a derivative sequence formed by adding one or more amino acids, substituting one or more amino acids, or deleting 1-30 amino acids in the amino acid sequence of (a), wherein the derivative sequence retains the antigen-specific binding ability of its source sequence.

In another preferred embodiment, the amino acid sequence of the second antigen-binding chain is selected from the group consisting of:
(a) the amino acid sequence shown in any one of SEQ ID NOs: 5, 7-12;
(b) a derivative sequence formed by adding one or more amino acids, substituting one or more amino acids, or deleting 1-30 amino acids in the amino acid sequence of (a), wherein the derivative sequence retains the antigen-specific binding ability of its source sequence.

In another preferred embodiment, the bispecific antibody comprises a first antigen-binding chain shown in SEQ ID NO: 2 and/or a second antigen-binding chain shown in SEQ ID NO: 5 or 7.

In another preferred embodiment, the multi-specific antibody further comprises an antigen-binding region targeting an additional antigen, preferably the antigen-binding region is a nanobody.

In another preferred embodiment, the multi-specific antibody has an affinity KD value for human MET of ≤10⁻⁸ M, more preferably ≤10⁻⁹ M.

In another preferred embodiment, the multi-specific antibody has an affinity KD value for human EGFR of ≤10⁻⁵ M, more preferably ≤10⁻⁶ M.

In a sixth aspect of the present invention, it provides a recombinant protein, wherein the recombinant protein comprises:
(i) the nanobody targeting MET according to the first aspect of the present invention, the antibody targeting MET according to the second aspect of the present invention, the nanobody targeting EGFR according to the third aspect of the present invention, the antibody targeting EGFR according to the fourth aspect of the present invention, or the multi-specific antibody according to the fifth aspect of the present invention; and
(ii) an optional tag sequence to assist expression and/or purification.

In another preferred embodiment, the tag sequence is selected from a group consisting of: a 6His tag, a GGGS tag, and a FLAG tag.

In the seventh aspect of the present invention, it provides a CAR construct, wherein the antigen-binding domain of the CAR construct comprises the nanobody targeting MET according to the first aspect of the present invention, the nanobody targeting EGFR according to the third aspect of the present invention, or a combination thereof.

In the eighth aspect of the present invention, it provides a recombinant immune cell, wherein the immune cell expresses an exogenous CAR construct according to the seventh aspect of the present invention.

In another preferred embodiment, the immune cell is selected from a NK cell and a T cell.

In another preferred embodiment, the immune cell is derived from human or a non-human mammal (such as a mouse).

In the ninth aspect of the present invention, it provides an immunoconjugate which comprises:
(a) an antibody moiety, which is the nanobody targeting MET according to the first aspect of the present invention, the antibody targeting MET according to the second aspect of the present invention, the nanobody targeting EGFR according to the third aspect of the present invention, the antibody targeting EGFR according to the fourth aspect of the present invention, or the multi-specific antibody according to the fifth aspect of the present invention; and
(b) a coupling moiety coupled to the nanobody moiety, which is selected from the group consisting of a detectable label, a drug, and a combination thereof.

In another preferred embodiment, the immunoconjugate is an antibody-drug conjugate.

In another preferred embodiment, the antibody moiety and the coupling moiety are coupled through a chemical bond or a linker.

In another preferred embodiment, the detectable label is a chemical marker, a biological marker, or a combination thereof.

In another preferred embodiment, the chemical marker is an isotope, an immunotoxin, and/or a chemical drug.

In another preferred embodiment, the biological marker is a biotin, an avidin or an enzyme marker.

In another preferred embodiment, the drug is a small-molecule drug, a biological factor, or a combination thereof.

In another preferred embodiment, the drug is a cytotoxic drug (toxin).

In another preferred embodiment, the toxin is selected from the group consisting of:
auristatin (e.g., auristatin E, auristatin F, MMAE and MMAF), chlortetracycline, maytansinoid, ricin, ricin A-chain, combretastatin, duocarmycin, dolastatin, adriamycin, daunorubicin, paclitaxel, cisplatin, cc1065, ethidium bromide, mitomycin, etoposide, tenoposide, vincristine, vinblastine, colchicine, dihydroxy anthrax dione, actinomycin, diphtheria toxin, pseudomonas exotoxin (PE) A, PE40, abrin, abrin A chain, modeccin A chain, α-octococcus, gelonin, mitogellin, retstrictocin, phenomycin, enomycin, curicin, crotonin, calicheamicin, Sapaonaria officinalis inhibitors, glucocorticoids, and a combination thereof.

In another preferred embodiment, the coupling moiety is MMAE.

In another preferred embodiment, the coupling moiety is a detectable marker.

In another preferred embodiment, the detectable marker comprises a radionuclide, and the radionuclide comprises:
(i) a diagnostic isotope, which is selected from the group consisting of Tc-99m, Ga-68, F-18, I-123, I-125, I-131, In-111, Ga-67, Cu-64, Zr-89, C-11, Lu-177, Re-188, and a combination thereof; and/or
(ii) a therapeutic isotope, which is selected from the group consisting of Lu-177, Y-90, Ac-225, As-211, Bi-212, Bi-213, Cs-137, Cr-51, Co-60, Dy-165, Er-169, Fm-255, Au-198, Ho-166, I-125, I-131, Ir-192, Fe-59, Pb-212, Mo-99, pd-103, P-32, K-42, Re-186, Re-188, Sm-153, Ra223, Ru-106, Na24, Sr89, Tb-149, Th-227, Xe-133 Yb-169, Yb-177, and a combination thereof.

In another preferred embodiment, the coupling moiety is selected from the group consisting of a fluorescent or luminescent marker, a radioactive marker, MRI (magnetic resonance imaging) or CT (electronic computer X-ray tomography technique) contrast agent, or an enzyme capable of producing a detectable product, a radionuclide, a biotoxin, a cytokine (such as IL-2, etc.), an antibody, an Fc fragment of an antibody, an scFv fragment of an antibody, a gold nanoparticle/nanorod, a viral particle, a liposome, a magnetic nanoparticle, a prodrug activating enzyme (such as DT-diaphorase (DTD) or biphenyl hydrolase-like protein (BPHL)), chemotherapeutic agent (such as cisplatin) and any form of nanoparticles, and the like.

In another preferred embodiment, the detection is *in vivo* detection or *in vitro* detection.

In another preferred embodiment, the immunoconjugate is used for diagnosing and/or treating a tumor that expresses MET protein.

In another preferred embodiment, the immunoconjugate is an antibody-drug conjugate, which has a molecular formula as shown in the following: 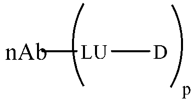 wherein,
nAb is the nanobody targeting MET according to the first aspect of the present invention, the antibody targeting MET according to the second aspect of the present invention, the nanobody targeting EGFR according to the third aspect of the present invention, the antibody targeting EGFR according to the fourth aspect of the present invention, or the multi-specific antibody according to the fifth aspect of the present invention;
LU is a chemical bond or a linker;
D is a drug;
p is the average number of drugs conjugated in the antibody-drug conjugate, and p is a value selected from 1-10.

In another preferred embodiment, the average number of drugs conjugated in the antibody-drug conjugate is 2-6, preferably 3-4, and more preferably 3.6-4.0.

In another preferred embodiment, LU is a maleimidocaproyl-valine-citrulline (MC-Val-Cit) linker.

In the tenth aspect of the present invention, it provides a pharmaceutical composition which comprises:
(i) the nanobody targeting MET according to the first aspect of the present invention, the antibody targeting MET according to the second aspect of the present invention, the nanobody targeting EGFR according to the third aspect of the present invention, the antibody targeting EGFR according to the fourth aspect of the present invention, the multi-specific antibody according to the fifth aspect of the present invention, the recombinant protein according to the sixth aspect of the present invention, the recombinant immune cell according to the eighth aspect of the present invention, or the immunoconjugate according to the ninth aspect of the present invention;
(ii) a pharmaceutically acceptable carrier.

In another preferred embodiment, the pharmaceutical composition includes a single drug, a compound drug, or a synergistic drug.

In another preferred embodiment, the pharmaceutical composition further comprises other biologically active substances, such as drugs for treating tumors.

In another preferred embodiment, the administration method of the pharmaceutical composition is selected from the group consisting of: subcutaneous injection, intradermal injection, intramuscular injection, intravenous injection, intraperitoneal injection, microneedle injection, oral administration, or oral and nasal spraying and aerosol inhalation.

In another preferred embodiment, the dosage form of the pharmaceutical composition is selected from the group consisting of: liquid, solid, or gel.

In another preferred embodiment, the pharmaceutical composition is a liquid preparation.

In another preferred embodiment, the pharmaceutical composition is an injection.

In the tenth aspect of the present invention, it provides a use of an active ingredient selected from the group consisting of: the nanobody targeting MET according to the first aspect of the present invention, the antibody targeting MET according to the second aspect of the present invention, the nanobody targeting EGFR according to the third aspect of the present invention, the antibody targeting EGFR according to the fourth aspect of the present invention, the multi-specific antibody according to the fifth aspect of the present invention, the recombinant protein according to the sixth aspect of the present invention, the recombinant immune cell according to the eighth aspect of the present invention, or the immunoconjugate according to the ninth aspect of the present invention, and a combination thereof, wherein the active ingredient is used for (a) preparation of a detection reagent, detection plate or kit; and/or (b) preparation of a medicament for the prevention and/or treatment of a disease.

In another preferred embodiment, the detection reagent, detection plate or kit is used in:
(1) detection of MET and/or EGFR protein in a sample; and/or
(2) detection of tumor cells expressing MET protein and/or EGFR protein.

In another preferred embodiment, the detection reagent, detection plate or kit is used for diagnosis of MET and/or EGFR related diseases.

In another preferred embodiment, the disease is a MET and/or EGFR related disease.

In another preferred embodiment, the disease includes a cancer.

In another preferred embodiment, the cancer comprises a solid tumors and a hematologic cancer.

In another preferred embodiment, the cancer is selected from the group consisting of: colon cancer, renal chromophobe cell carcinoma, renal papillary cell carcinoma, mesothelioma, pancreatic cancer, prostate cancer, ovarian germ cell cancer, thyroid cancer, gastric cancer, esophageal cancer, lung cancer (such as lung adenocarcinoma and non-small cell lung cancer), breast cancer (such as triple-negative breast cancer), malignant glioma, liver cancer, bladder cancer, endometrial cancer, cervical cancer, leukemia, myeloma, osteosarcoma, angiosarcoma, and a combination thereof.

In another preferred embodiment, the cancer is selected from the group consisting of: lung cancer, colorectal cancer, pancreatic cancer, breast cancer, renal cancer, and gastric cancer.

In the twelfth aspect of the present invention, it provides a polynucleotide which encodes a polypeptide selected from the group consisting of:
(1) the nanobody targeting MET according to the first aspect of the present invention, the antibody targeting MET according to the second aspect of the present invention, the nanobody targeting EGFR according to the third aspect of the present invention, the antibody targeting EGFR according to the fourth aspect of the present invention, the multi-specific antibody according to the fifth aspect of the present invention;
(2) the recombinant protein according to the sixth aspect of the present invention; or
(3) the CAR construct according to the seventh aspect of the present invention.

In another preferred embodiment, the polynucleotide includes RNA, DNA or cDNA.

In another preferred embodiment, the polynucleotide comprises a nucleotide sequence selected from the group consisting of SEQ ID NOs: 19, 20, 21, 22, and a combination thereof.

In the thirteenth aspect of the present invention, it provides a vector, which contains the polynucleotide according to the twelfth aspect of the present invention.

In another preferred embodiment, the vector includes: bacterial plasmid, phage, yeast plasmid, plant cell virus, mammalian cell virus such as adenovirus, retrovirus, or other vectors.

In the fourteenth aspect of the present invention, it provides a host cell, wherein the host cell comprises the vector according to the thirteenth aspect of the present invention or the genome thereof is integrated with the polynucleotide according to the twelfth aspect of the present invention.

In the fifteenth aspect of the present invention, it provides a method for preparing a recombinant polypeptide, which comprises:
(a) culturing the host cell according to the fourteenth aspect of the present invention under conditions suitable for expression;
(b) isolating the recombinant polypeptide from the culture, wherein the recombinant polypeptide is the nanobody targeting MET according to the first aspect of the present invention, the antibody targeting MET according to the second aspect of the present invention, the nanobody targeting EGFR according to the third aspect of the present invention, the antibody targeting EGFR according to the fourth aspect of the present invention, the multi-specific antibody according to the fifth aspect of the present invention, or the recombinant protein of the sixth aspect of the invention.

In the sixteenth aspect of the present invention, it provides a method for treating a disease, comprising: administering to a subject in need the nanobody targeting MET according to the first aspect of the present invention, the antibody targeting MET according to the second aspect of the present invention, the nanobody targeting EGFR according to the third aspect of the present invention, the antibody targeting EGFR according to the fourth aspect of the present invention, the multi-specific antibody according to the fifth aspect of the present invention, or the recombinant protein of the sixth aspect of the invention, or a combination thereof.

In another preferred embodiment, the method further comprises: administering additional drugs or therapies to the subject in need for a combined treatment.

In another preferred embodiment, the additional drugs or therapies include: an anti-tumor immunotherapy drug, tumor targeted drug, tumor chemotherapy drug, and tumor radiotherapy.

It should be understood that, within the scope of the present invention, the technical features specifically described above and below (such as the Examples) can be combined with each other, thereby constituting a new or preferred technical solution which needs not be described one by one.

### DESCRIPTION OF DRAWINGS

The following drawings are used to illustrate specific embodiments of the present invention and are not intended to limit the scope of the present invention as defined by the claims.
Figure 1 shows the screening process for nanobodies.
Figure 2 shows the binding of MET antibody KY301-01 to recombinant human MET antigen (by ELISA).
Figure 3 shows the binding of MET antibody KY301-01 to recombinant human MET antigen (A), mouse MET antigen (B) and cynomolgus MET antigen (C) (by ELISA).
Figure 4 shows that MET antibody KY301-01 can bind to CHO-K1-MET cells (CHO-K1 cells expressing human MET protein).
Figure 5 shows that MET antibody KY301-01 can be internalized in ASPC-1 (A), HCT116 (B), NCI-H1993 (C) and EBC-1 (D) cells.
Figure 6 shows that MET antibody KY301-01 can block the binding of HGF to MET antigen.
Figure 7 shows that the MET antibody-MMAE conjugate can effectively kill NCI-H1975 (A), HCT116 (B), BxpC-3 (C), MDA-MB-468 (D), NCI-H1993 (E), HT29 (F), MDA-MB-231 (G) and 786O cells (H).
Figure 8 shows that the MET antibody-MMAE product can effectively inhibit the growth of HCT116 xenograft tumors.
Figure 9 shows that the MET antibody-MMAE product can effectively inhibit the growth of NCI-H1975 tumor cells in the NCG tumor-bearing mouse model.
Figure 10 shows that the EGFR antibody KY303-58 can bind to the recombinant human EGFR antigen (by ELISA).
Figure 11 shows the binding of the EGFR antibody KY303-58 to the recombinant human EGFR antigen (A), mouse EGFR antigen (B) and cynomolgus EGFR antigen (C) (by ELISA).
Figure 12 shows that EGFR antibody KY303-58 does not bind to CHO-K1 cells.
Figure 13 shows that EGFR antibody KY303-58 can be internalized in NCI-H1975 (A) and HCT116 (B) cells.
Figure 14 shows that EGFR antibody KY303-58 cannot block the binding of EGF to EGFR.
Figure 15 shows that EGFR antibody-MMAE conjugate can effectively kill HCT116 (A), NCI-H1975 (B), NCI-H1993 (C), MDA-MB-468 (D), MDA-MB-231 (E), HT29 (F) and BxpC-3 (G).
Figure 16 shows that the EGFR antibody-MMAE product can effectively inhibit the growth of NCI-H1975 tumor cells in the NCG tumor-bearing mouse model.
Figure 17 shows that the EGFR antibody-MMAE product can effectively inhibit the growth of HCT116 tumor cells in the NCG tumor-bearing mouse model.
Figure 18 shows 8 forms of the MET-EGFR bispecific antibody.
Figure 19 shows that the MET-EGFR antibody can bind to the recombinant human MET antigen (by ELISA).
Figure 20 shows that the MET-EGFR antibody can bind to the recombinant human EGFR antigen (by ELISA).
Figure 21 shows that the MET-EGFR antibody can bind to the recombinant human, mouse and cynomolgus MET and EGFR antigens.
Figure 22 shows that the binding of the MET-EGFR antibody to cells is specific.
Figure 23 shows that the 8 MET-EGFR antibody-MMAE products can effectively kill HCT116 cells.
Figure 24 shows that BH01-BK58, DH01-DK58, and BH01-DK58 at a concentration of 1.25 µg/mL have significant killing effects on HCT116 cells.
Figure 25 shows that 8 MET-EGFR antibody-MMAE products can effectively kill NCI-H1975 cells.
Figure 26 shows that BH01-BK58, DH01-DK58, and BH01-DK58 at a concentration of 1.25 µg/mL have significant killing effects on NCI-H1975 cells.
Figure 27 shows the association-dissociation kinetic curves of BH01-BK58, BH01-DK58, and the control antibody AZ-RAA22/B09 with MET and EGFR antigens.
Figure 28 shows that BH01-BK58-MMAE and BH01-DK58-MMAE are mainly DAR4 products.
Figure 29 shows that BH01-BK58-MMAE and BH01-DK58-MMAE can effectively kill HT29 (A), BxPC-3 (B), HS746T (C), and SNU-5 (D) cells.
Figure 30 shows that BH01-BK58-MMAE and BH01-DK58-MMAE can effectively inhibit the tumor growth in B-NDG mice inoculated with HCT116 cells, and the tumor-inhibiting effect of BH01-DK58-MMAE is better than that of BH01-BK58-MMAE.
Figure 31 shows that BH01-BK58-MMAE and BH01-DK58-MMAE can effectively inhibit the tumor growth in B-NDG mice inoculated with NCI-H1975 cells, and the tumor-inhibiting effect of BH01-DK58-MMAE is better than that of BH01-BK58-MMAE.
Figure 32 shows that BH01-BK58-MMAE and BH01-DK58-MMAE are well-tolerated at a dose of 10 mg/kg or less.
Figure 33 shows that BH01-BK58-MMAE has certain toxicity to male C57BL/6 mice at doses of 16 mg/kg and 20 mg/kg, while BH01-DK58-MMAE is still well-tolerated at high doses.
Figures 34A-34D show that the internalization ability of BH01-DK58 is stronger than that of single-target antibodies and the control antibody.
Figure 35 shows the protein levels of MET and EGFR in 9 types of tumor cells.
Figure 36 shows the changes in protein levels and phosphorylation levels of MET and EGFR in NCI-H1975, EBC-1, and HCC827 cells after 24-hour treatment with 1 mg/mL KY-0301.
Figure 37 shows that the tumor-killing effect of BH01-DK58-MMAE is better than that of single-target ADCs (KY 301-01-MMAE and KY303-58-MMAE).

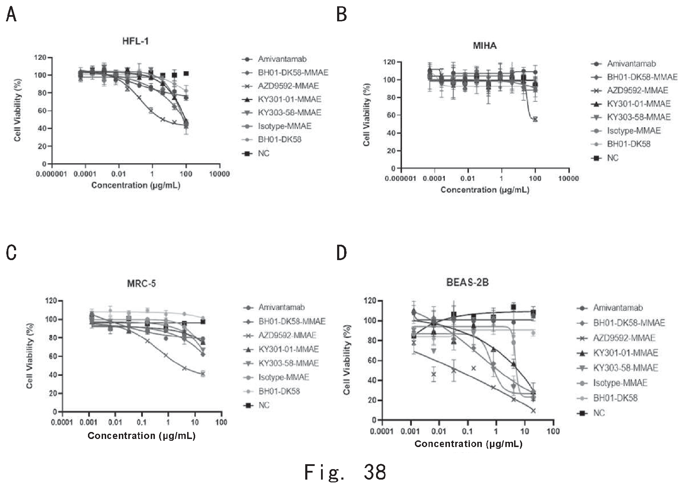

Figure 38 shows that the toxicity of BH01-DK58-MMAE to normal cells is weaker than that of the control antibody-MMAE conjugate.
Figure 39 shows that BH01-DK58-MMAE can induce G2/M phase arrest in HCT116 cells.
Figure 40 shows the structural schematic diagram of KY-0301 (i.e., BH01-DK58-MMAE).
Figure 41 shows that KY-0301 can significantly inhibit the growth of xenograft tumors of human lung cancer cells EBC-1.
Figure 42 shows that KY-0301 can significantly inhibit the growth of xenograft tumors of human colorectal cancer cells HT29.
Figure 43 shows that KY-0301 has significant anti-tumor efficacy against NCI-H1975 xenograft tumors in high-tumor-burden models and relapse models.
Figure 44 shows that KY-0301 can effectively inhibit tumor growth in lung cancer PDX mouse models.
Figure 45 shows that KY-0301 can effectively inhibit tumor growth in pancreatic cancer PDX mouse models.
Figure 46 shows that the inhibitory efficiency of KY-0301 against xenograft tumors of human colorectal cancer cells HCT116 is better than that of AZD9592-MMAE.
Figure 47 shows that the inhibitory efficiency of KY-0301 against xenograft tumors of human lung cancer cells EBC-1 is better than that of AZD9592-Dxd.

### EMBODIMENTS

Through extensive and intensive research, the inventors have developed an anti-MET/EGFR bispecific antibody and a drug conjugate thereof for the first time. Specifically, the present invention provides a nanobody against MET and a nanobody against EGFR, as well as a bispecific antibody comprising the two nanobodies of the present invention. The bispecific nanobody of the present invention targets both MET and EGFR simultaneously, and has characteristics, such as small molecular weight and rapid penetration into tumor tissues. It has better toxin-delivery ability compared with traditional antibodies. On this basis, the present invention has been completed.

### MET

MET, also known as the HGF (hepatocyte growth factor) receptor, consists of three parts: the extracellular region, the transmembrane region, and the intracellular region. The extracellular region contains the Sema (Semaphorin) domain, the PSI (Plexin Semaphorin Integrin) domain, and the IPT (Immunoglobulin Plexin Transcription) domain. The SPH domain of HGF interacts with the SEMA domain of MET, inducing MET activation. The intracellular region of MET includes the juxtamembrane region, the tyrosine kinase catalytic domain, and the C-terminal tail. When HGF binds to MET, MET dimerizes, and the tyrosines Y1234 and Y1235 in the tyrosine kinase catalytic domain undergo autophosphorylation, which also induces the phosphorylation of Y1349 and Y1356 in the C-terminal tail, recruiting effector molecules such as GRB2, SRC, PI3K and GV1 to activate downstream signaling pathways. Serine 975 and tyrosine Y1003 in the juxtamembrane region are important sites for negatively regulating MET activity. After the phosphorylation of tyrosine Y1003, the ubiquitin ligase c-CBL is recruited to ubiquitinate MET, promoting MET degradation and reducing the signaling through MET signaling pathway. The downstream signaling pathways of MET include the RAS/MAPK proliferation and survival signaling pathway, the PI3K/AKT/mTOR cell movement and invasion signaling pathway, and the SRC/FAK mechanical sensing signaling pathway. When gene amplification, mutation, or overexpression of MET occurs, MET is over-activated, which induces tumor genesis or promotes drug resistance and invasion of tumor. For example, MET amplification is an indicator of poor prognosis in non-small cell lung cancer, liver cancer, gastric cancer, and triple-negative breast cancer. In NSCLC cells resistant to EGFR inhibitors, MET gene amplification occurs, affecting the treatment effect. The exon 14 skipping mutation of MET exists in about 4% of non-small cell lung cancers and is associated with poor prognosis in immunotherapy.

### EGFR

EGFR is a member of the HER (Human Epidermal Growth Factor Receptor) protein family, also known as HER1. Similar to MET in structure, it is divided into three parts: the extracellular region, the transmembrane region, and the intracellular region. The extracellular region of EGFR has four domains (I, II, III, and IV). Domains I and III bind ligands such as GF, TGF-α, and HB-EGF through the leucine-rich structure, and domains II and IV are rich in cysteine. The intracellular region of EGFR is divided into the juxtamembrane region, the tyrosine kinase region, and the C-terminal tail, containing 5 autophosphorylation sites: Y992, Y1045, Y1068, Y1148, and Y117. After the ligand binds to EGFR, EGFR forms a dimer and undergoes autophosphorylation, activating downstream RAS/RAF/MAPK, AKT, and JAK/STAT signaling pathways to regulate cell physiological functions such as growth, proliferation, and differentiation. Studies have shown that EGFR overexpression and mutation are related to the occurrence, development, and drug resistance of many tumors. For example, an increase in the expression level of EGFR is a predictive indicator for head and neck cancer, ovarian cancer, cervical cancer, bladder cancer, esophageal cancer, etc. The so-called "classical" mutations of EGFR, the deletion of exon 19 and the single-amino acid substitution L858R in exon 21, exist in about 85% of EGFR-mutated NSCLC.

As two important proto-oncogenes, there is an interrelationship between MET and EGFR. The expression levels of MET and EGFR in tumor tissues are often higher than those in normal tissues, and they tend to be co-expressed in a variety of tumor cells. For example, data from the GEPIA database show that in colon cancer, renal chromophobe cell carcinoma, renal papillary cell carcinoma, mesothelioma, pancreatic cancer, prostate cancer, ovarian germ cell cancer, and thyroid cancer, the linear correlation coefficient between MET and EGFR expression is above 0.5, indicating a high correlation.

**Table 1 Relationship and co-expression of MET and EGFR in certain tumor cells**

| Cancer type | COAD | KICH | KIRP | MESO | PAAD | PRAD | TGCT | THCA |
|---|---|---|---|---|---|---|---|---|
| R | 0.54 | 0.54 | 0.52 | 0.53 | 0.59 | 0.61 | 0.62 | 0.6 |
| p-value | 0 | 2.4e-06 | 0 | 1.2e-07 | 0 | 0 | 6.7e-16 | 0 |

### Nanobody

As used herein, the terms "the anti-MET nanobody of the present invention" and "anti-MET nanobody" are used interchangeably, both referring to the nanobody according to the first aspect of the present invention that specifically recognizes and binds to MET (including human MET), and particularly preferred the nanobody whose amino acid sequence of the VHH chain is as shown in SEQ ID NO: 1.

As used herein, the terms "the anti-EGFR nanobody of the present invention" and "anti-EGFR nanobody" are used interchangeably, both referring to the nanobody according to the first aspect of the present invention that specifically recognizes and binds to EGFR (including human EGFR), and particularly preferred the nanobody whose amino acid sequence of the VHH chain is as shown in SEQ ID NO: 3.

The term "antibody" or "immunoglobulin" as used herein refers to a heterotetrameric glycoprotein having the same structural feature of about 150,000 daltons consisting of two identical light chains (L) and two identical heavy chains (H). Each light chain is linked to a heavy chain by a covalent disulfide bond, and the numbers of disulfide bonds between the heavy chains of different immunoglobulin isoforms are different. Each heavy and light chain also has regularly spaced intrachain disulfide bonds. One end of each heavy chain has a variable region (VH) followed by a plurality of constant regions. There is a variable region (VL) at one end of each chain and a constant region at the other end; the constant region of the light chain corresponds to the first constant region of the heavy chain; the variable region of the light chain corresponds to the variable region of the heavy chain. An interface is formed between the variable regions of the light and heavy chains by particular amino acid residues.

As used herein, the terms "nanobody", "VHH" have the same meaning and refer to the heavy chain variable region of a monoclonal antibody. A nanobody (VHH) is the smallest antigen-binding fragment with complete function. Usually, the antibody with natural deletion of light chain and heavy chain constant region 1(CH1) is obtained first, and then the variable region of the antibody heavy chain is cloned to construct a nanobody (VHH) composed of only one heavy chain variable region.

As used herein, the term "heavy chain antibody" refers to an antibody that comprises only heavy chains. In the blood of camelids, a part of the antibodies are "heavy chain antibodies" lacking light chains. The heavy chain antibody of the present invention comprises a heavy chain variable region (VHH) and heavy chain constant regions CH2 and CH3. The heavy chain antibody of the present invention may be an antibody isolated from animals (such as camel-derived) that naturally lacks the light chain and the heavy chain constant region 1 (CH1); or may be a recombinant antibody obtained by recombining the nanobody (VHH) of the present invention with a heavy chain constant region. The heavy chain antibody of the present invention may comprise a constant region derived from, for example, IgG1, IgG2, IgG3 or IgG4, preferably a constant region derived from IgG1.

As used herein, the term "variable" means that some certain portions of the variable region of an antibody differ in sequence and contribute to the binding and specificity of each particular antibody to its particular antigen. However, the variability is not evenly distributed throughout the antibody variable region. It is concentrated in three regions in the light and heavy chain variable regions called complementarity determining regions (CDRs) or hypervariable regions. The more conserved portions of the variable regions are referred as framework regions (FRs). The variable regions of the natural heavy and light chains each comprises four FR regions, which are in a substantially β-folded configuration, and are linked by three CDRs that form the linker ring and, in some cases, form a partial β-folded structure. The CDRs in each chain stand close together through FR regions and form the antigen-binding site of the antibody together with the CDRs of the other chain (see Kabat et al., NIH Publ. No. 91-3242, Vol. I, 647-669 (1991)). Constant regions are not directly involved in the binding of the antibodies to the antigens, but they exhibit different effector functions, such as antibody-dependent cellular cytotoxicity involved in antibodies.

In a preferred embodiment of the present invention, the heavy chain variable region of the antibody comprises three complementarity determining regions, CDR1, CDR2, and CDR3. There are currently several methods for CDR division, including the IMGT method, Kabat method, Chothia method, VBASE2 method, etc. In one embodiment, the CDRs mentioned in the present invention are divided using the IMGT method.

In a preferred embodiment of the present invention, the heavy chain of the antibody comprises the above-mentioned heavy chain variable region and the heavy chain constant region.

The invention also provides other proteins or fusion expression products comprising the antibody of the present invention. Specifically, the present invention includes any protein or protein conjugate and fusion expression product (i.e., immunoconjugate and fusion expression product) having a heavy chain containing variable regions, as long as the variable region is the same as or has at least 90% homology with the variable regions of the heavy chain of the antibody of the present invention, preferably at least 95% homology.

In the present invention, the terms "the recombinant protein of the present invention", "the fusion protein of the present invention", or "the polypeptide of the present invention" can be used interchangeably and all refer to an polypeptide specifically binding to MET and/or EGFR protein, e.g., a protein or polypeptide with the VHH chain of the nanobody of the present invention. They can contain or do not contain starting methionine.

The variable regions of the heavy chain of the antibody of the present invention are of particular interest because at least part of them involves binding antigens. Therefore, the present invention includes molecules with heavy chain variable regions of antibodies with CDRs, as long as their CDR has more than 90% (preferably more than 95%, most preferably more than 98%) homology with the CDR identified here.

### Anti-MET nanobody and anti-EGFR nanobody

The present invention provides a nanobody targeting MET, wherein the VHH chain of the nanobody comprises the following complementarity determination regions CDR1, CDR2 and CDR3:
a CDR1 as shown in SEQ ID NO: 13,
a CDR2 as shown in SEQ ID NO: 14, and
a CDR3 as shown in SEQ ID NO: 15.

In one embodiment, the VHH chain of the nanobody targeting MET has an amino acid sequence as shown in SEQ ID NO: 1.

The present invention further provides a nanobody targeting EGFR, wherein the VHH chain of the nanobody comprises the following complementarity determination regions CDR1, CDR2 and CDR3:
a CDR1 as shown in SEQ ID NO: 16,
a CDR2 as shown in SEQ ID NO: 17, and
a CDR3 as shown in SEQ ID NO: 18.

In one embodiment, the VHH chain of the nanobody targeting EGFR has an amino acid sequence as shown in SEQ ID NO: 3.

Preferably, any one of the above amino acid sequences further includes a derivative sequence which is optionally added, deleted, modified, and/or substituted with at least one amino acid, and can retain the binding affinity to MET and/or EGFR.

In another preferred embodiment, the sequence formed by adding, deleting, modifying, and/or substituting at least one amino acid has preferably at least 80% amino acid sequence homology, preferably at least 85%, preferably at least 90%, and preferably at least 95%.

Wherein, the animal is preferably a mammal, such as a mouse, or a llama.

In preferred embodiments, the present invention discloses a variety of llama-derived and humanized nanobodies targeting MET and/or EGFR with high specificity and high affinity. These nanobodies merely consist of a heavy chain which comprises a heavy chain variable region (VHH) amino acid sequence and optionally constant regions CH2 and CH3.

### Bispecific Antibodies

The present invention also includes a recombinant protein (or fusion protein) comprising the MET and/or EGFR nanobody of the present invention. A preferred fusion protein is a multi-specific antibody, such as a bispecific antibody.

The bispecific antibody of the present invention specifically binds to MET and EGFR antigens. In a preferred embodiment, the affinity of the bispecific antibody of the present invention for MET (e.g., human MET) is significantly higher than that for EGFR (e.g., human EGFR). For example, when the affinity is represented by the equilibrium dissociation constant (KD value) for the antigen, the ratio of the KD value for EGFR to the KD value for MET (KD_{EGFR}/KD_{MET}) of the bispecific antibody of the present invention is ≥50, preferably ≥100, and more preferably ≥150.

The present invention includes not only intact antibodies, but also immunologically active fragments of antibody fragments or fusion proteins formed by antibodies and other sequences. Therefore, the present invention also includes fragments, derivatives and analogs of the antibodies.

The antibody of the present invention may be a double-chain or a single-chain antibody, and can be selected from an animal derived antibody, a chimeric antibody, and a human-animal chimeric antibody, preferably a humanized antibody.

The antibody derivative described in the present invention can be a single chain antibody, and/or an antibody fragment, such as Fab, Fab ', (Fab') 2 or other known antibody derivatives in the field, as well as any one or more of IgA, IgD, IgE, IgG, and IgM antibodies or other subtypes of antibodies.

The antibodies of the present invention can be homodimers or heterodimers. As used herein, a "homodimer" refers to an antibody having two antigen-binding chains with the same amino acid sequence. As used herein, a "heterodimer" refers to an antibody having two antigen-binding chains with different amino acid sequences. When the antibody of the present invention is a heterodimer, it can comprise one or more specific binding sites on each chain. For example, one chain can comprise binding domains (such as VHH) targeting both Met and EGFR, while the other chain can only comprise a binding domain (such as VHH) targeting Met.

The bispecific antibodies of the present invention can be produced by the "knob-into-hole" strategy. Briefly, the selected amino acids that form the constant-region connecting site in IgG can be mutated at positions that affect the interaction between constant regions to promote the formation of heterodimers. An amino acid with a small side chain (hole) is introduced into the constant region of one chain, and an amino acid with a large side chain (knob) is introduced into the constant region of the other chain. Due to the preferential interaction between the heavy chain with a "hole" and the antigen-binding chain with a "knob", a heterodimer is formed between the two chains.

In each antigen-binding chain of the bispecific antibody of the present invention, the VHH portion can be connected to the Fc fragment of IgG through a hinge region. In one embodiment, the EGFR-targeting VHH domain is connected to the Fc fragment via a short-arm hinge. Compared with the wild-type IgG1 hinge EPKSSDKTHTCPPCP (SEQ ID NO: 23), this short-arm hinge can be truncated by 1-7 amino acids at the N-terminus. In one embodiment, the short-arm hinge is truncated by 4 amino acids, and the truncated hinge is mutated to ADKTHTCPPCP (SEQ ID NO: 24). The design of the short-arm hinge helps to reduce the swinging range of the antibody at the EGFR end, thereby reducing the binding priority of the EGFR antibody to the antigen.

As used herein, the terms "fragment", "derivative" and "analog" refer to the polypeptides basically maintaining the same biological function or activity of the antibody of the present invention. The polypeptide fragments, derivatives or analogs of the present invention may be (i) a polypeptide with one or more conservative or non-conservative amino acid residues (preferably the conservative amino acid residues) being substituted, while such substituted amino acid residues may or may not be encoded by genetic code, or (ii) a polypeptide having substituted group(s) in one or more amino acid residues, or (iii) a polypeptide formed by fusion of the matured polypeptide with another compound (such as the compound that prolongs the half-life of the polypeptide, such as polyethylene glycol), or (iv) a polypeptide formed with additional amino acid sequence fused to said polypeptide sequence (such as, leader sequence, secretion sequence, or a sequence or a protein sequence used to purify the polypeptide, or a fusion protein formed with 6His tag). According to the subject application, these fragments, derivatives and analogs are within the scope commonly known by the skilled person.

The antibody of the present invention refers to a polypeptide having MET and/or EGFR protein binding activity and comprising the above-mentioned CDR regions. The term also includes variant forms of polypeptides comprising the CDR regions described above that have the same function as the antibody of the present invention. These variant forms include, but are not limited to, deletions of one or more amino acids (typically 1-50, preferably 1-30, more preferably 1-20, most preferably 1-10) , insert ions and/or substitutions, and the addition of one or several amino acids (typically at most 20, preferably at most 10, more preferably at most 5) at the C-terminus and/or N-terminus. For example, in the art, the protein's functions are usually unchanged when an amino acids is substituted by a similar or analogous one. Also, for example, the addition of one or several amino acids at the C-terminus and/or the N-terminus will not normally alter the function of the protein. The term also includes active fragments and active derivatives of the antibody of the present invention.

The variant forms of the antibody include homologous sequences, conserved variants, allelic variants, natural mutants, induced mutants, proteins encoded by a DNA capable of hybridizing to the coding DNA of the antibody of the present invention under high or low stringency conditions, and a polypeptide or protein obtained using an antiserum against the antibody of the present invention.

In the present invention, "conservative variants thereof the antibody of the present invention" refers to polypeptides that comprises at most 10, preferably at most 8, more preferably at most 5, most preferably at most 3 amino acids replaced by amino acids with the same or similar properties compared with the amino acid sequence of the antibody of the present invention. These conservatively variant polypeptides are preferably produced by amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides a polynucleotide molecule encoding the antibody or a fragment thereof or a fusion protein thereof. The polynucleotides of the present invention can be in a form of DNA or RNA. DNA forms include cDNA, genomic DNA, or synthetic DNA. DNA can be single-stranded or double-stranded. DNA can be the coding strand or the non-coding strand.

The polynucleotides encoding the mature polypeptides of the present invention comprise coding sequences encoding only the mature polypeptide; coding sequences of the mature polypeptide and various additional coding sequences; coding sequences (and optionally additional coding sequences) of the mature polypeptide, and non-coding sequences.

The term "polynucleotide encoding a polypeptide" may include a polynucleotide that encodes the polypeptide, or a polynucleotide that also includes additional coding and/or non-coding sequences.

The present invention also relates to polynucleotides that hybridize to the sequences as described above and having at least 50%, preferably at least 70%, more preferably at least 80% identical between the two sequences. In particular, the present invention relates to polynucleotides that can hybridize to the polynucleotides of the present invention under stringent conditions. In the present invention, "stringent conditions" means: (1) hybridization and elution at lower ionic strength and higher temperature, such as 0.2 x SSC, 0.1% SDS, 60°C; or (2) hybridization adding a denaturant, such as 50% (v/v) formamide, 0.1% calf serum/0.1% Ficoll, 42 °C, or the like; or (3) hybridization only occurs when the identity between the two sequences is at least 90%, more preferably 95% or more. And the polypeptide encoded by the hybridizable polynucleotide has the same biological function and activity as the mature polypeptide.

The whole length of the nucleotide sequence or the fragment thereof of the antibody of the present invention can be obtained via PCR amplification, recombinant method or artificial synthesis. One feasible method is to synthesize relevant sequences by artificial method, especially when the fragment is short in length. Usually, several small fragments are synthesized first, and then are linked together to obtain a fragment with a long sequence. In addition, the sequence coding the heavy chain and the expression label (e.g. 6His) can be fused together to form a fusion protein.

### Preparation of the antibody

The sequence of the DNA molecule for the antibody or a fragment thereof according to the present invention can be obtained by conventional techniques, for example, methods such as PCR amplification or genomic library screening. In addition, the sequences encoding the nanobody and a constant region can be fused together, to form a heavy chain antibody.

Once a relevant sequence is obtained, the relevant sequence can be obtained in bulk using a recombination method. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

At present, DNA sequences encoding the protein of the invention (or fragments thereof, or derivatives thereof) can be completely obtained by chemical synthesis. The DNA sequence can then be introduced into a variety of existing DNA molecules (or vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as an animal cell. Preferred animal cells comprise, but are not limited to, CHO-S, HEK-293 cells.

In general, under conditions suitable for expression of the antibody according to the present invention, the host cell obtained is cultured. Then, the antibody according to the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained can be identified by conventional means. For example, the binding specificity of a monoclonal antibody can be determined by immunoprecipitation or an in vitro binding assay (such as radioimmunoassay (RIA) or enzyme-linked immunosorbent assay (ELISA)). The binding affinity of a monoclonal antibody, for example, can be determined by using Scatchard analysis, Munson et al., Anal. Biochem, 107:220 (1980).

The antibody of the present invention can be expressed intracellularly or on the cell membrane, or be secreted out of the cell. If desired, recombinant proteins can be isolated and purified by various separation methods using their physical, chemical and other properties. These methods are well known to those skilled in the art. Examples of such methods include, but are not limited to, conventional renaturation treatments, treatment with a protein precipitant (salting-out method), centrifugation, osmosis cell disruption, super-treatment, ultracentrifugation, molecular sieve chromatography (gel filtration), adsorption chromatography, ion-exchange chromatography, high performance liquid chromatography (HPLC) and various other liquid chromatography techniques and combinations of these methods.

### Immunoconjugate

The present invention also provides an immunoconjugate based on the antibody of the present invention (ADC), such as a nanobody-drug conjugate (NDC).

Typically, the antibody-drug conjugate comprises the antibody and an effector molecule, wherein the antibody is conjugated, preferably chemically conjugated to the effector molecule. Preferably, the effector molecule is a therapeutically active drug. In addition, the effector molecule may be one or more of a toxic protein, a chemotherapeutic drug, a small-molecule drug or a radionuclide.

The antibody of present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that forms a covalent bond between the effector molecule and the antibody, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g., a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a linker between a drug and an antibody. The linker can be a degradable or non-degradable linker. Typically, the degradable linker is easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g., lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g., linkers that are hydrolyzed at a pH of below 5.5, such as hydrazone linkers) and linkers that are degraded under reducing conditions (e.g., disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g., iodo-, bromo- or chloro-substituted) amide; a halogenated (e.g., iodo-, bromo- or chloro-substituted) ester; a halogenated (e.g., iodo-, bromo- or chloro-substituted) methyl ketone, a benzyl halide (e.g., iodide, bromide or chloride); vinyl sulfone, pyridyl disulfide; a mercury derivative such as 3,6-di-(mercurymethyl)dioxane, wherein the counter ion is an acetate, a chloride, or a nitrate; and polymethylene dimethyl sulfide thiosulfonate. The linker may include, for example, a maleimide linked to an antibody via thiosuccimide.

In one embodiment, a maleimidocaproyl-valine-citrulline (MC-Val-Cit) linker is used in the ADC of the present invention.

A drug may be any cytotoxic, cytostatic or immunosuppressive drug. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA groove binding reagents, DNA alkylation reagents and tubulin inhibitors, and typical cytotoxic drugs include, for example, auristatins, camptothecins, duocarmycins, etoposides, maytansines and maytansinoids (e.g., DM1 and DM4), taxanes, benzodiazepines, or benzodiazepine containing drugs (e.g., pyrrolo[1,4]benzodiazepines (PBDs), indolinobenzodiazepines, and oxazolidinobenzodiazepines) and vinca alkaloids.

In the present invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, as described in Bioconjugation Technology (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

The present invention further provides a method for preparing an ADC, which may further comprise: under conditions sufficient to form an antibody-drug conjugate (ADC), connecting an antibody to a drug-linker compound.

In certain embodiments, the method according to the present invention comprises: under conditions sufficient to form an antibody-linker conjugate, connecting an antibody to a linker compound. In these embodiments, the method according to the present invention further comprises: under conditions that the drug moiety is sufficient to be covalently linked to the antibody via a linker, connecting the antibody-linker conjugate to the drug moiety.

The ADCs prepared from the bispecific antibodies provided by the present invention have a uniform drug-to-antibody ratio (DAR). In some embodiments, the drug-to-antibody ratio is in the range of 2-4, such as 2-2.5, 2.5-3, 3-3.2, 3.2-3.4, 3.4-3.6, 3.6-3.8 and 3.8-4.

### Application

The present invention also provides uses of the antibodies of the present invention, such as for the preparation of diagnostic agents or for the preparation of drugs for the prevention and/or treatment of MET- and/or EGFR-related diseases. The MET- and/or EGFR-related diseases include genesis, growth and/or metastasis of tumor, diseases related to tumor-drug-resistance, inflammation, metabolism-related diseases, etc.

The uses of the antibodies, ADCs, CAR-Ts, etc. of the present invention include (but are not limited to): diagnosing, preventing and/or treating genesis, growth and/or metastasis of tumors, especially tumors with high expression of MET and/or EGFR. The tumors include (but are not limited to): colon cancer, renal chromophobe cell carcinoma, renal papillary cell carcinoma, mesothelioma, pancreatic cancer, prostate cancer, ovarian germ cell cancer, thyroid cancer, gastric cancer, esophageal cancer, lung cancer (such as lung adenocarcinoma and non-small cell lung cancer), breast cancer (such as triple-negative breast cancer), malignant glioma, liver cancer, bladder cancer, endometrial cancer, cervical cancer, leukemia, myeloma, angiosarcoma, and a combination thereof.

### Pharmaceutical composition

The present invention further provides a composition. In the preferred embodiments, the composition is a pharmaceutical composition comprising the antibody, or an active fragment, a fusion protein or an ADC thereof, or a corresponding CAR-T cell, and a pharmaceutically acceptable carrier. In general, these substances may be formulated in a non-toxic, inert and pharmaceutically acceptable aqueous carrier medium. The formulated pharmaceutical composition may be administered by conventional routes, including (but not limited to): intratumoral, intraperitoneal, intravenous, or topical administration.

The antibody of the present invention can also be used for cell therapy by expressing the nucleotide sequence in the cell. For example, the antibody is used for chimeric antigen receptor T cell immunotherapy (CAR-T) and the like.

The pharmaceutical composition according to the present invention can be directly used for binding to a MET and/or EGFR protein molecule, and thus can be used for preventing and treating diseases such as tumors. In addition, other therapeutic agents can also be used at the same time.

The pharmaceutical composition according to the present invention comprises a safe and effective amount (e.g. 0.001-99 wt %, preferably 0.01-90 wt %, preferably 0.1-80 wt %) of the monoclonal antibody according to the present invention (or a conjugate thereof) and a pharmaceutically acceptable carrier or excipient. Such carriers include (but are not limited to): saline, buffers, glucose, water, glycerol, ethanol, and a combination thereof. Pharmaceutical preparations should correspond to the administration modes. The pharmaceutical composition according to the present invention can be prepared in the form of an injection, for example, by a conventional method using physiological saline or an aqueous solution containing glucose and other adjuvants. A pharmaceutical composition, for example, an injection and a solution, should be prepared under aseptic conditions. The administration amount of an active ingredient is a therapeutically effective amount, for example, about 1 µg per kilogram of body weight to about 5 mg per kilogram of body weight daily. In addition, the polypeptide according to the present invention may also be used in combination with an additional therapeutic agent.

When a pharmaceutical composition is used, a safe and effective amount of Immune conjugate is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 20 mg per kilogram of body weight. Of course, a specific amount should also depend on the factors such as administration route and physical conditions of a patient, which fall into the skills of skilled physicians.

For ADCs, since the nanobody-drug conjugate provided by the present invention can target specific cell populations and bind to specific proteins (antigens) on the cell surface, the drug can be released into cells in an active form through internalization of conjugates or infiltration of drugs. Therefore, the nanobody-drug conjugate of the present invention can be used to treat target diseases. The above-mentioned antibody-drug conjugate can be administered to a subject (such as a human) in a therapeutically effective amount via a suitable route. Subjects in need of treatment can be patients at risk of or suspected to have a disorder related to the activity or expression level of a specific antigen. Such patients can be identified through routine physical examinations.

When treating with the nanobody-drug conjugate of the present invention, it can be delivered by conventional methods in the art. For example, it can be introduced into cells by using liposomes, hydrogels, cyclodextrins, biodegradable nanocapsules, or bioadhesive microspheres. Alternatively, the nucleic acid or vector can be delivered locally by direct injection or by using an infusion pump.

### Main advantages of the present invention include:

1) The nanobodies of the present invention are characterized by their small molecular weight and rapid penetration into tumor tissues. The nanobody ADCs constructed from them have better toxin-delivery capabilities compared with traditional antibodies.
2) The MET-EGFR dual-target ADC of the present invention employs the nanobody which has only two pairs of disulfide bonds in the hinge region. By the random cysteine coupling method, a maximum of 4 toxins can be coupled. Thus the DAR value is uniform, and the purity of the unpurified DAR4 ADC after coupling can reach over 90%, which is beneficial for large-scale production.
3) In the present invention, the EGFR antibody has been optimized to have moderate affinity. The affinity of the bispecific antibody of the present invention for MET is significantly higher than that for EGFR. Meanwhile, the EGFR end of the MET-EGFR dual-target ADC is designed to have a short-arm hinge, which to some extent reduces the binding priority of the EGFR antibody to the antigen, improving the safety of the MET-EGFR dual-target ADC. The acute toxicity experiment in mice shows that the short-arm hinge design at the EGFR end can effectively reduce the toxic side effects in mice.
4) The drug resistance of tumor cells to EGFR inhibitors is often related to the amplification of the MET gene. Inhibiting both MET and EGFR simultaneously can enhance the tumor-killing effect and reduce tumor drug resistance.

The invention is further illustrated below in conjunction with specific embodiments. It should be understood that the examples are not intended to limit the scope of the invention. The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989), or in accordance with the conditions recommended by the manufacturer. Unless otherwise stated, percentages and parts are by weight.

The cell lines used in the examples include:
Tumor cell
Lung cancer: NCI-H1975, NCI-H1993, EBC-1, HCC827
Colorectal cancer: HCT116, HT29
Pancreatic cancer: BxPC-3, ASPC-1
Breast cancer: MDA-MB-231, MDA-MB-468
Renal cancer: 786-O
Gastric cancer: HS746T, SNU-5

### Non-tumor cell

Hamster ovarian cells: CHO-K1, CHO-K1-MET (CHO-K1 cell expressing human MET protein), CHO-K1-EGFR (CHO-K1 cell expressing human EGFR protein)
Human normal tissue cells: HFL-1 (human lung fibroblast), MIHA cell (human normal liver cell), MRC-5 (human embryonic lung fibroblast), BEAS-2B (human normal bronchial epithelial cell)

### Example 1. Immunization of llama and screening of MET nanobody

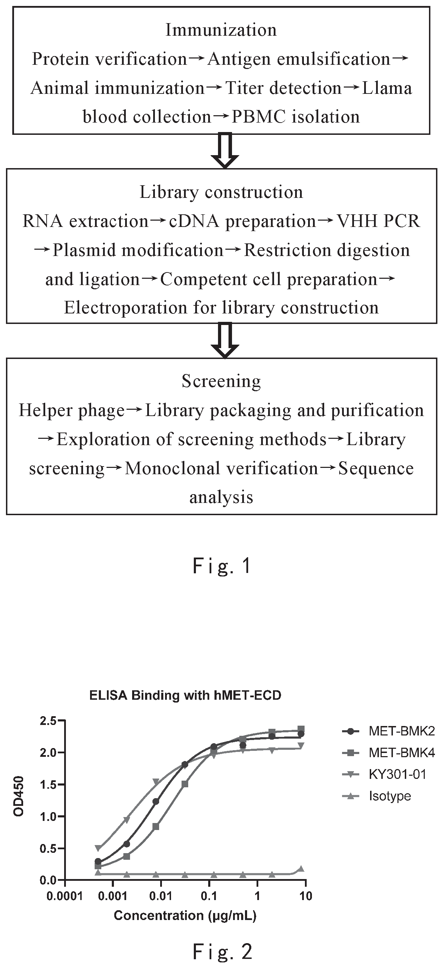

The recombinant human MET antigen was used to immunize llama. After 4 times of antigen immunization, the serum titer of llama reached the library standard, the mRNA in the llama PBMC was extracted, and the cDNAs were obtained by reverse transcription. The antibody screening was carried out through phage display technology. The nanobody screening process is shown in Fig. 1.

### Example 2. Expression and purification of MET nanobody

Through the phage screening technique, the MET nanobody sequence KY301-VHH01 was obtained, and the amino acid sequence and nucleotide sequence thereof are shown in Table 2. Then KY301-VHH01 was fused to the IgG1 Fc sequence and expressed, and the fusion-expressed MET nanobody was named as KY301-01.

The CDR sequences (IMGT) of KY301-01 are shown below:
CDR1: GRTDSTYA (SEQ ID NO: 13);
CDR2: ISWSGGST (SEQ ID NO: 14);
CDR3: AADHRTGSTRFRTAQYDYDY (SEQ ID NO: 15).

**Table 2 Amino acid sequences and nucleotide sequences of the MET antibody variable region**

| Antibody No. | Amino Acid Sequence | Nucleotide Sequence |
|---|---|---|
| KY301-V HH01 | QVQLVESGGGLVQAGG SLRLSCAASGRTDSTYA MGWFRQAPGKERESVA AISWSGGSTYYADSVKG RFTISRDNAKHTVYLQM NSLKPEDTAVYYCAAD HRTGSTRFRTAQYDYD YWGQGTQVTVSS (SEQ ID NO: 1) | CAAGTGCAGTTGGTCGAATCCGGAGGGGGCTG GTCCAAGCTGGAGGTAGTCTGCGCCTTTCATGTG CTGCATCAGGTCGGACCGATTCAACATACGCTA TGGGATGGTTTCGACAGGCGCCAGGCAAAGAAC GGGAAAGCGTTGCCGCTATCTCCTGGTCCGGCG GCTCAACGTACTATGCCGACAGTGTGAAGGGTA GATTCACGATCAGTCGAGATAATGCCAAACACA CGGTGTACCTTCAAATGAACTCTCTGAAACCGG AGGACACAGCCGTATATTATTGCGCCGCAGACC ATCGGACGGGGAGTACAAGGTTCCGCACCGCAC AATATGATTATGATTACTGGGGGCAAGGAACCC AGGTAACTGTATCAAGT (SEQ ID NO: 19) |
| KY301-01 | QVQLVESGGGLVQAGG SLRLSCAASGRTDSTYA MGWFRQAPGKERESVA AISWSGGSTYYADSVKG RFTISRDNAKHTVYLQM NSLKPEDTAVYYCAAD HRTGSTRFRTAQYDYD YWGQGTQVTVSSEPKSS DKTHTCPPCPAPELLGG PSVFLFPPKPKDTLMISR TPEVTCVVVDVSHEDPE VKFNWYVDGVEVHNA KTKPREEQYNSTYRVVS VLTVLHQDWLNGKEYK CKVSNKALPAPIEKTISK AKGQPREPQVYTLPPSR DELTKNQVSLSCAVKGF YPSDIAVEWESNGQPEN NYKTTPPVLDSDGSFFL VSKLTVDKSRWQQGNV FSCSVMHEALHNHYTQ KSLSLSPGK (SEQ ID NO: 2) | CAAGTGCAGTTGGTCGAATCCGGAGGGGGCTG GTCCAAGCTGGAGGTAGTCTGCGCCTTTCATGTG CTGCATCAGGTCGGACCGATTCAACATACGCTA TGGGATGGTTTCGACAGGCGCCAGGCAAAGAAC GGGAAAGCGTTGCCGCTATCTCCTGGTCCGGCG GCTCAACGTACTATGCCGACAGTGTGAAGGGTA GATTCACGATCAGTCGAGATAATGCCAAACACA CGGTGTACCTTCAAATGAACTCTCTGAAACCGG AGGACACAGCCGTATATTATTGCGCCGCAGACC ATCGGACGGGGAGTACAAGGTTCCGCACCGCAC AATATGATTATGATTACTGGGGGCAAGGAACCC AGGTAACTGTATCAAGTGAGCCCAAGAGTTCCG ATAAGACCCACACGTGTCCCCCCTGTCCCGCCCC CGAGTTGTTGGGCGGACCAAGTGTTTTTTGTTT CCCCCTAAGCCAAAAGATACGCTGATGATTAGT AGAACACCAGAGGTGACATGCGTGGTCGTGGAT GTAAGCCATGAAGATCCCGAGGTGAAGTTTAAC TGGTATGTGGACGGCGTGGAAGTACATAACGCA AAGACAAAACCTAGGGAAGAACAATACAACAG TACTTACAGGGTAGTGTCAGTATTGACTGTCCTG CATCAGGATTGGTTGAACGGCAAAGAGTACAAG TGCAAAGTGTCAAACAAAGCGCTCCCTGCCCCC ATTGAGAAAACCATCAGTAAAGCTAAAGGTCAG CCTCGGGAACCGCAGGTATATACTCTTCCGCCAT CCAGAGATGAACTCACAAAAAATCAGGTAAGTC TTTCTTGCGCGGTTAAAGGCTTTACCCGTCCGA CATTGCAGTAGAATGGGAGAGTAATGGACAGCC AGAGAATAATTACAAGACTACACCTCCGGTGCT CGATAGCGACGGCTCCTTTTTCCTGGTTAGTAAG CTCACCGTGGATAAATCTCGCTGGCAGCAAGGT AACGTCTTCAGCTGCTCAGTCATGCACGAGGCG CTGCACAATCACTACACTCAGAAGAGCCTCAGT |
| | | CTGAGTCCTGGCAAATGA (SEQ ID NO: 20) |

### Example 3. Evaluation of the binding activity of MET antibody to human MET antigen

Method:
1. The plate was coated with recombinant human MET antigen at 4°C overnight, and then washed twice with 200 µL of PBST (0.01% Tween-20);
2. Wells were blocked for 2 hours by adding 300 µL of 3% BSA solution per well, and then washed twice with 200 µL of PBST (0.01% Tween-20);
3. MET antibody was added and incubated at room temperature for 1 hour, and then washed twice with 200 µL of PBST (0.01% Tween-20);
4. 100 µL of anti-Fc-HRP antibody dilution was added and incubated at 37°C for 0.5 hours, and then washed 4 times with 200 µL of PBST (0.01% Tween-20);
5. 100 µL of TMB substrate solution was added for color development for 5 minutes;
6. 50 µL of stop solution was added to quench the reaction, and the absorbance value was read at 450 nm on a microplate reader.

The experimental results are shown in Table 3 and Fig. 2. The results indicate that the MET antibody KY301-01 binds to the MET antigen, and its antigen-binding activity level is comparable to that of MET-BMK2 and MET-BMK4. MET-BMK2 is from SEQ ID NO: 82 in patent US11142578B2, while MET-BMK4 is from the MET monoclonal antibody portion of Amivantamab.

**Table 3 EC₅₀ value of the MET antibody binding to recombinant human MET antigen**

| | KY301-01 | MET-BMK2 | MET-BMK4 |
|---|---|---|---|
| EC₅₀(µg/mL) | 0.002333 | 0.007301 | 0.01818 |

### Example 4. Evaluation of the species-cross binding activity of MET antibody

Method:
1. The plate was coated with recombinant human, mouse and cynomolgus MET antigen at 4°C overnight, respectively, and then washed twice with 200 µL of PBST (0.01% Tween-20);
2. Wells were blocked for 2 hours by adding 300 µL of 3% BSA solution per well, and then washed twice with 200 µL of PBST (0.01% Tween-20);
3. MET antibody was added and incubated at room temperature for 1 hour, and then washed twice with 200 µL of PBST (0.01% Tween-20);
4. 100 µL of anti-Fc-HRP antibody dilution was added and incubated at 37°C for 0.5 hours, and then washed 4 times with 200 µL of PBST (0.01% Tween-20);
5. 100 µL of TMB substrate solution was added for color development for 5 minutes;
6. 50 µL of stop solution was added to quench the reaction, and the absorbance value was read at 450 nm on a microplate reader.

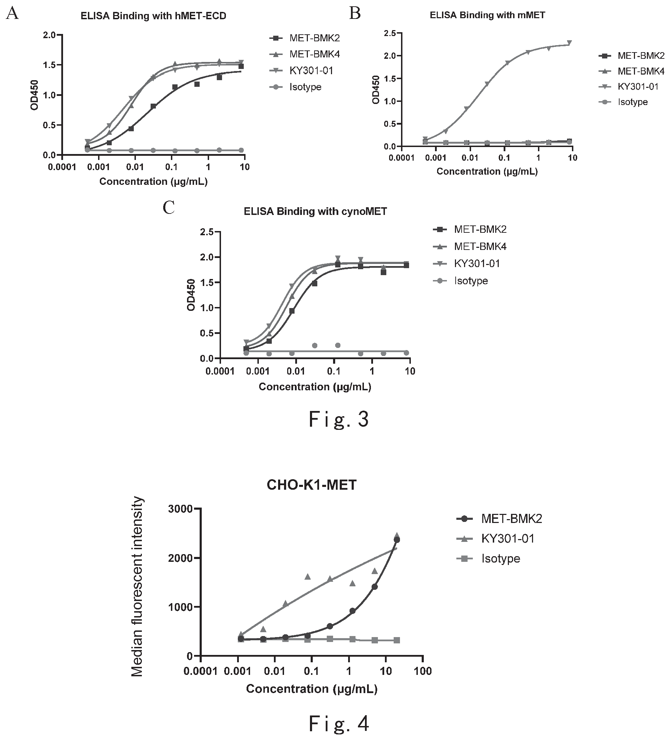

As shown in Fig. 3 and Table 4, the experimental results indicate that the MET antibody KY301-01 is capable of binding to human, mouse, and cynomolgus MET antigens, having the species-cross activity. MET-BMK2 and MET-BMK4 have no binding activity to mouse MET antigen.

**Table 4 EC₅₀ values of MET antibody binding to mouse and cynomolgus MET antigens**

| EC₅₀(µg/mL) | KY301-01 | MET-BMK2 | MET-BMK4 |
|---|---|---|---|
| mMET | 0.01692 | / | / |
| cynoMET | 0.004290 | 0.008757 | 0.005713 |

### Example 5. Evaluation of MET antibody for binding activity at cellular level

Method:
1. CHO-K1-MET cells (CHO-K1 cells expressing human MET protein) were seeded in a 96-well transparent V-bottom plate at a density of 1E5 cells/well, with 100 µL cell suspension per well;
2. 100 µL of diluted antibody solution to be tested was added to each well and incubated at 4°C for 1 hour. The cells were washed twice with 200 µL of PBS containing 1% FBS at 4°C;
3. 100 µL of 1:100 diluted anti-Fc-PE antibody was added to each well, and incubated at 4°C for 0.5 hour;
4. The cells were washed twice with 100 µL of PBS containing 1% FBS, and resuspended in 100 µL of PBS. Fluorescence signals were detected in the PE fluorescence channel, and median fluorescence intensity was observed.

As shown in Fig. 4, the experimental results indicate that the MET antibody binds to CHO-K1-MET cell, and its binding activity is stronger than that of MET-BMK2.

### Example 6. Evaluation of MET antibody for internalization activity in tumor cells

Method:
1. ASPC-1, HCT116, NCI-H1993, and EBC-1 cells were seeded in a 96-well transparent V-bottom plate at a density of 3E5 cells/well with 100 µL cell suspension per well, respectively;
2. 100 µL of diluted antibody solution to be tested was added to each well and incubated at 4°C for 1 hour. The cells were washed twice with 200 µL of culture medium;
3. 200 µL of culture medium was used to resuspend the cells. 100 µL of cell suspension from each well was transferred to a new 96-well V-bottom plate and incubated at 37°C for 2 hours, while the remaining cells in the prior wells were incubated at 4°C for 2 hours;
4. The supernatant was removed by centrifugation, and 100 µL of 1:100 diluted anti-Fc-PE antibody was added to each well and incubated at 4°C for 0.5 hour;
5. The cells were washed twice with 100 µL of PBS containing 1% FBS, and resuspended in 100 µL of PBS. Fluorescence signals were detected in the PE fluorescence channel, and median fluorescence intensity was observed to calculate the internalization rate.

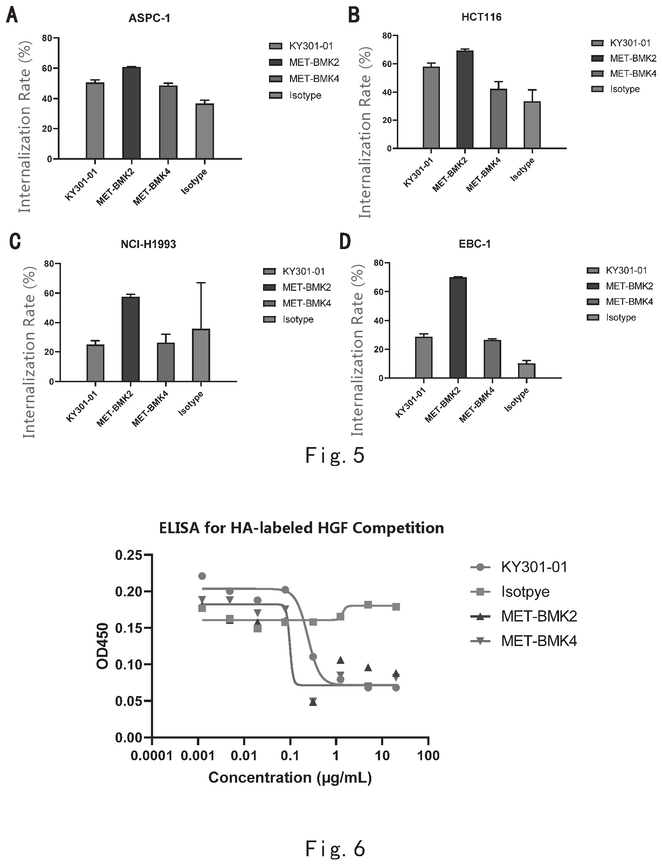

As shown in Fig. 5, the experimental results indicate the internalization of MET antibody in ASPC-1, HCT116, NCI-H1993, and EBC-1 cells, where the internalization rate is cell type-dependent.

### Example 7. Evaluation of the ligand-blocking activity of MET antibody

Method:
1. The plate was coated with recombinant human MET antigen at 4°C overnight, and then washed twice with 200 µL of PBST (0.01% Tween-20);
2. Wells were blocked for 2 hours by adding 300 µL of 3% BSA solution per well, and then washed twice with 200 µL of PBST (0.01% Tween-20);
3. 100 µL of a mixture of MET antibody and different concentrations of HA-labeled HGF ligand was added and incubated at room temperature for 1 hour, and then washed twice with 200 µL of PBST (0.01% Tween-20);
4. 100 µL of anti-HA-HRP antibody dilution was added and incubated at 37°C for 0.5 hours, and then washed 4 times with 200 µL of PBST (0.01% Tween-20);
5. 100 µL of TMB substrate solution was added for color development for 5 minutes;
6. 50 µL of stop solution was added to terminate the reaction, and the absorbance value was read at 450 nm using a microplate reader.

As shown in Fig. 6, the experimental results indicate that KY301-01 has the ability to block HGF.

### Example 8. Conjugation of MET antibody with MMAE

Method:
1. Histidine-acetate buffer (with a pH of 5.5), EDTA-2Na solution, TCEP solution, and MET antibody were sequentially added, so that the final concentration of MET antibody was 2-3 mg/mL, the final concentration of EDTA-2Na was 5 mM, and the amount of TCEP substance was 8 times that of the MET antibody. The mixture was well-mixed and reacted with shaking at 37 °C for 4 h;
2. 9 times the amount of the antibody substance of MMAE and DMSO solution were added, such that the total amount of DMSO accounted for 10% of the total reaction solution volume. The reaction was carried out with shaking at 4 °C for 2 h;
3. 7 times the amount of the antibody substance of L-cysteine was added and mixed well;
4. The above-mentioned reaction product was centrifuged. The supernatant was taken and transferred to a 10 KDa ultrafiltration tube, centrifuged, added with histidine-acetate buffer, and centrifuged again. This process was repeated 18 times to remove the free toxin that was not conjugated to the antibody.
5. The liquid in the ultrafiltration tube was taken out. The ultrafiltration tube was rinsed with histidine-acetate buffer. The rinsing liquid was mixed with the liquid taken-out previously. The absorbance was detected at 280 nm for calculation of antibody concentration.

### Example 9. Cytotoxicity Evaluation of MET Antibody-MMAE Product

Method:
1. NCI-H1975/HCT116/BxPC-3/MDA-MB-468/NCI-H1993/HT29/MDA-MB-231/786O cells were seeded into a 96-well plate with black wall and transparent bottom at 100 µL of cell suspension per well;
2. 10 µL of serially diluted ADC to be tested was added to each well, and the cells were cultured for 6 days;
3. 100 µL of CellTiter-Glo 2.0 Cell Viability Assay was added to each well, and the plate was shaken for mixing for 10 min. Then the fluorescence intensity was detected.

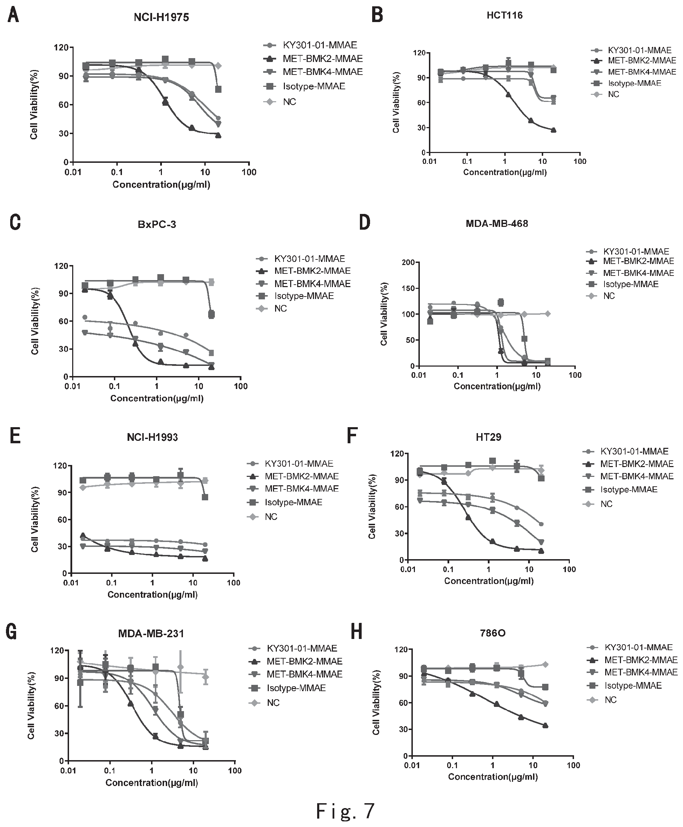

As shown in Fig. 7, the experimental results indicate that the MET antibody-MMAE conjugate shows different sensitivities to different tumor cells, and can effectively inhibit the growth of various tumor cells at a high concentration.

**Table 5 IC50 values (µg/mL) of MET antibody-MMAE conjugate product against different tumor cells**

| Cell Line | KY301-01 | Cell Line | KY301-01 |
|---|---|---|---|
| HCT116 | 6.320 | 786-O | >20 |
| HT29 | >20 | BxPC-3 | >20 |
| NCI-H1975 | 9.961 | NCI-H1993 | >20 |
| MDA-MB-468 | 1.512 | MDA-MB-231 | 3.096 |

### Example 10. MET Antibody-MMAE Product Inhibits the Growth of HCT116 Xenograft Tumors

Method:
1. HCT116 cells were inoculated subcutaneously into the right anterior flank of female NCG mice (aged 5-6 weeks, 18-21 g);
2. When the tumors grew to about 100-150 mm³, the mice were grouped into 3 groups with 5 mice in each group;
3. MET single-target ADC or PBS was administered via tail-vein injection at doses of 6 mg/kg and 2 mg/kg once a week for 2 consecutive weeks;
4. Tumor volume and body weight were measured twice a week, and the relationship between the changes in body weight and tumor volume of tumor-bearing mice and the administration time was recorded.
5. The tumor volume and tumor growth inhibition rate were calculated:
   Tumor volume (V) was calculated as (length × width²)/2.
   Tumor growth inhibition rate (TGI%) was calculated using the following formula: 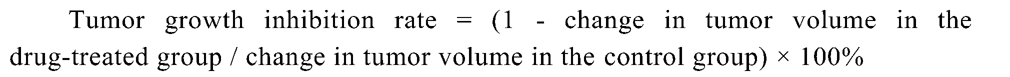 Tumor growth inhibition rate = (1 - change in tumor volume in the drug-treated group / change in tumor volume in the control group) × 100%

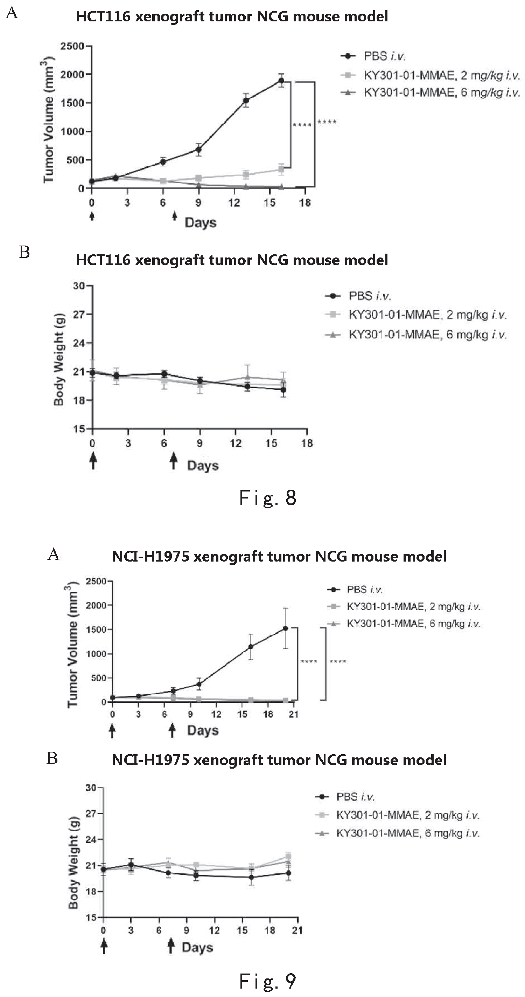

As shown in Fig. 8, the experimental results indicate that in the NCG mouse model inoculated with HCT116 tumor cells, tumor growth was significantly inhibited at a dose of 2 mg/kg, with a tumor inhibition rate of 79.1% on Day 16. When the dose was increased (6 mg/kg), the inhibitory effect of MET single-target ADC on tumor volume was further significantly enhanced, and the tumor inhibition rate reached 98.6%. The above results indicate that MET single-target ADC can effectively inhibit the growth of HCT116 xenograft tumors in a dose-dependent manner. Meanwhile, significant effect on the body weight of the mice was shown in neither the low-dose nor the high-dose group.

### Example 11. MET Antibody-MMAE Product Inhibits the Growth of NCI-H1975 Xenograft Tumors

Method:
1. NCI-H1975 cells were inoculated subcutaneously into the right anterior flank of female NCG mice (aged 5-6 weeks, 18-21 g);
2. When the tumors grew to about 100-150 mm³, the mice were grouped into 3 groups with 5 mice in each group;
3. MET single-target ADC or PBS was administered via tail-vein injection at doses of 6 mg/kg and 2 mg/kg once a week for 2 consecutive weeks;
4. Tumor volume and body weight were measured twice a week, and the relationship between the changes in body weight and tumor volume of tumor-bearing mice and the administration time was recorded.
5. The tumor volume and tumor growth inhibition rate were calculated:
   Tumor volume (V) was calculated as (length × width²)/2.
   Tumor growth inhibition rate (TGI%) was calculated using the following formula: 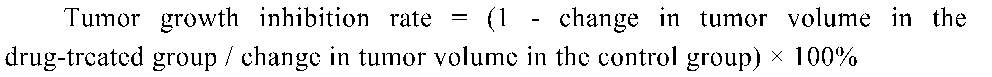 Tumor growth inhibition rate = (1 - change in tumor volume in the drug-treated group / change in tumor volume in the control group) × 100%

As shown in Fig. 9, the experimental results indicate that in the NCG mouse model inoculated with NCI-H1975 tumor cells, tumor growth was significantly inhibited at doses of 2 mg/kg and 6 mg/kg, with tumor inhibition rates of 97.6% and 98.01% on Day 20, respectively. The above results indicate that MET single-target ADC can effectively inhibit the growth of NCI-H1975 xenograft tumors in a dose-dependent manner. Meanwhile, significant effect on the body weight of the mice was shown in neither the low-dose nor the high-dose group.

### Example 12. Immunization of llama and screening, expression and purification of EGFR nanobody

The recombinant human EGFR antigen was used to immunize llama. After 4 times of antigen immunization, the serum titer of llama reached the library standard, the mRNA in the llama PBMC was extracted, and the cDNA was obtained by reverse transcription. The antibody screening was carried out through phage display technology. The nanobody screening process is shown in Fig. 1.

Through phage screening, the EGFR nanobody sequence KY303-VHH58 was obtained, and the amino acid sequence and nucleotide sequence thereof are shown in Table 6. Then KY303-VHH58 was fused to the IgG1 Fc sequence and expressed, and the fusion-expressed EGFR nanobody was named KY303-58.

The CDR sequences (IMGT) of KY303-58 are shown below:
CDR1: GSTFSSYA (SEQ ID NO: 16);
CDR2: ISSGGST (SEQ ID NO: 17);
CDR3: NLHPRVN (SEQ ID NO: 18).

**Table 6 Amino acid sequence and nucleotide sequence of the EGFR antibody variable region**

| Antibody No. | Amino acid sequence of variable region | Nucleotide sequence of variable region |
|---|---|---|
| KY303-V HH58 | EVQVVESGGGLVQAGG SLRLSCAASGSTFSSYA MGWYRQAPGKQRESVA FISSGGSTNVAESVKGRF TISRDNAKNTVYLQMN NLKAEDTAVYYCNLHP RVNWGQGTQVTVSS (SEQ ID NO: 3) | GAGGTCCAGGTTGTCGAGTCAGGAGGAGGTTTG GTTCAAGCGGGCGGATCTCTGAGACTTAGTTGT GCGGCTTCCGGGAGCACCTTCTCAAGTTACGCA ATGGGGTGGTATCGGCAGGCTCCTGGTAAACAA AGGGAATCCGTTGCTTTATTCAAGCGGAGGCT CTACAAATGTCGCGGAGTCTGTCAAAGGCAGAT TTACGATCTCACGCGATAATGCAAAAAACACCG TGTATTTGCAGATGAATAATCTGAAGGCTGAGG ATACCGCCGTATACTATTGCAATTTGCACCCGAG GGTTAACTGGGGGCAAGGGACGCAGGTAACGGT TTCCAGT (SEQ ID NO: 21) |
| KY303-58 | EVQVVESGGGLVQAGG SLRLSCAASGSTFSSYA MGWYRQAPGKQRESVA FISSGGSTNVAESVKGRF TISRDNAKNTVYLQMN NLKAEDTAVYYCNLHP RVNWGQGTQVTVSSEP KSSDKTHTCPPCPAPELL GGPSVFLFPPKPKDTLMI SRTPEVTCVVVDVSHED PEVKFNWYVDGVEVHN AKTKPREEQYNSTYRVV SVLTVLHQDWLNGKEY KCKVSNKALPAPIEKTIS KAKGQPREPQVYTLPPS RDELTKNQVSLSCAVKG FYPSDIAVEWESNGQPE NNYKTTPPVLDSDGSFF LVSKLTVDKSRWQQGN VFSCSVMHEALHNHYT QKSLSLSPGK (SEQ ID NO: 4) | GAGGTCCAGGTTGTCGAGTCAGGAGGAGGTTTG GTTCAAGCGGGCGGATCTCTGAGACTTAGTTGT GCGGCTTCCGGGAGCACCTTCTCAAGTTACGCA ATGGGGTGGTATCGGCAGGCTCCTGGTAAACAA AGGGAATCCGTTGCTTTTATTTCAAGCGGAGGCT CTACAAATGTCGCGGAGTCTGTCAAAGGCAGAT TTACGATCTCACGCGATAATGCAAAAAACACCG TGTATTTGCAGATGAATAATCTGAAGGCTGAGG ATACCGCCGTATACTATTGCAATTTGCACCCGAG GGTTAACTGGGGGCAAGGGACGCAGGTAACGGT TTCCAGTGAGCCCAAGAGTTCCGATAAGACCCA CACGTGTCCCCCCTGTCCGCCCCCGAGTTGTTG GGCGGACCAAGTGTTTTTTGTTTCCCCCTAAGC CAAAAGATACGCTGATGATTAGTAGAACACCAG AGGTGACATGCGTGGTCGTGGATGTAAGCCATG AAGATCCCGAGGTGAAGTTTAACTGGTATGTGG ACGGCGTGGAAGTACATAACGCAAAGACAAAA CCTAGGGAAGAACAATACAACAGTACTTACAGG GTAGTGTCAGTATTGACTGTCCTGCATCAGGATT GGTTGAACGGCAAAGAGTACAAGTGCAAAGTGT CAAACAAAGCGCTCCCTGCCCCCATTGAGAAAA CCATCAGTAAAGCTAAAGGTCAGCCTCGGGAAC CGCAGGTATATACTCTTCCGCCATCCAGAGATG AACTCACAAAAAATCAGGTAAGTCTTTCTTGCG CGGTTAAAGGCTTTTACCCGTCCGACATTGCAGT AGAATGGGAGAGTAATGGACAGCCAGAGAATA ATTACAAGACTACACCTCCGGTGCTCGATAGCG ACGGCTCCTTTTTCCTGGTTAGTAAGCTCACCGT |
| | | GGATAAATCTCGCTGGCAGCAAGGTAACGTCTT CAGCTGCTCAGTCATGCACGAGGCGCTGCACAA TCACTACACTCAGAAGAGCCTCAGTCTGAGTCC TGGCAAATGA (SEQ ID NO: 22) |

### Example 13. Evaluation of the binding activity of EGFR antibody to human EGFR antigen

Method:
1. The plate was coated with recombinant human EGFR antigen at 4°C overnight, and then washed twice with 200 µL of PBST (0.01% Tween-20);
2. Wells were blocked for 2 hours by adding 300 µL of 3% BSA solution per well, and then washed twice with 200 µL of PBST (0.01% Tween-20);
3. EGFR antibody was added and incubated at room temperature for 1 hour, and then washed twice with 200 µL of PBST (0.01% Tween-20);
4. 100 µL of anti-Fc-HRP antibody dilution was added and incubated at 37°C for 0.5 hours, and then washed 4 times with 200 µL of PBST (0.01% Tween-20);
5. 100 µL of TMB substrate solution was added for color development for 5 minutes;
6. 50 µL of stop solution was added to terminate the reaction, and the absorbance value was read at 450 nm using a microplate reader.

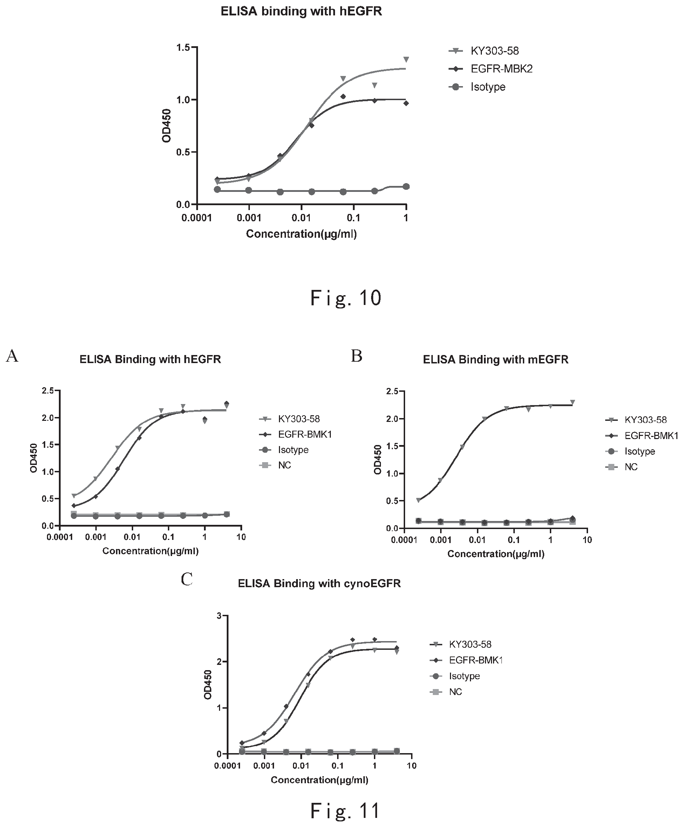

As shown in Fig. 10, the experimental results indicate that the EGFR antibody KY303-58 can bind to the EGFR antigen with an affinity lower than that of the control antibody EGFR-BMK2. This weak affinity to EGFR suggests better safety. EGFR-BMK2 is derived from SEQ ID NO: 34 and SEQ ID NO: 42 of patent US 10047160B2.

**Table 7 EC₅₀ value of the EGFR antibody binding to recombinant human EGFR antigen**

| | KY303-58 | EGFR-BMK2 |
|---|---|---|
| EC50 (µg/mL) | 0.01271 | 0.007996 |

### Example 14. Evaluation of the species-cross activity of EGFR antibody

Method:
1. The plate was coated with recombinant human, mouse and cynomolgus EGFR antigen at 4°C overnight, respectively, and then washed twice with 200 µL of PBST (0.01% Tween-20);
2. Wells were blocked for 2 hours by adding 300 µL of 3% BSA solution per well, and then washed twice with 200 µL of PBST (0.01% Tween-20);
3. EGFR antibody was added and incubated at room temperature for 1 hour, and then washed twice with 200 µL of PBST (0.01% Tween-20);
4. 100 µL of anti-Fc-HRP antibody dilution was added and incubated at 37°C for 0.5 hours, and then washed 4 times with 200 µL of PBST (0.01% Tween-20);
5. 100 µL of TMB substrate solution was added for color development for 5 minutes;
6. 50 µL of stop solution was added to terminate the reaction, and the absorbance value was read at 450 nm using a microplate reader.

As shown in Fig. 11, the experimental results indicate that the EGFR antibody KY303-58 is capable of binding to human, mouse, and cynomolgus EGFR antigens, having the species-cross activity. EGFR-BMK1 has no binding activity to mouse EGFR antigen. EGFR-BMK1 is derived from Cetuximab.

**Table 8 EC₅₀ values of EGFR antibody binding to mouse and cynomolgus EGFR antigens**

| EC₅₀(µg/mL) | KY303-58 | EGFR-BMK1 |
|---|---|---|
| mEGFR | 0.01692 | / |
| cynoEGFR | 0.002586 | 2.439 |

### Example 15. Evaluation of EGFR antibody binding specificity at cellular level

Method:
1. CHO-K1, CHO-K1-EGFR cells (CHO-K1 cells expressing human EGFR protein) were seeded in a 96-well transparent V-bottom plate at a density of 1E5 cells/well, with 100 µL cell suspension per well;
2. 100 µL of diluted antibody solution to be tested was added to each well and incubated at 4°C for 1 hour. The cells were washed twice with 200 µL of PBS containing 1% FBS at 4°C;
3. 100 µL of 1:100 diluted anti-Fc-PE antibody was added to each well, and incubated at 4°C for 0.5 hour;
4. The cells were washed twice with 100 µL of PBS containing 1% FBS, and resuspended in 100 µL of PBS. Fluorescence signals were detected in the PE fluorescence channel, and median fluorescence intensity was observed.

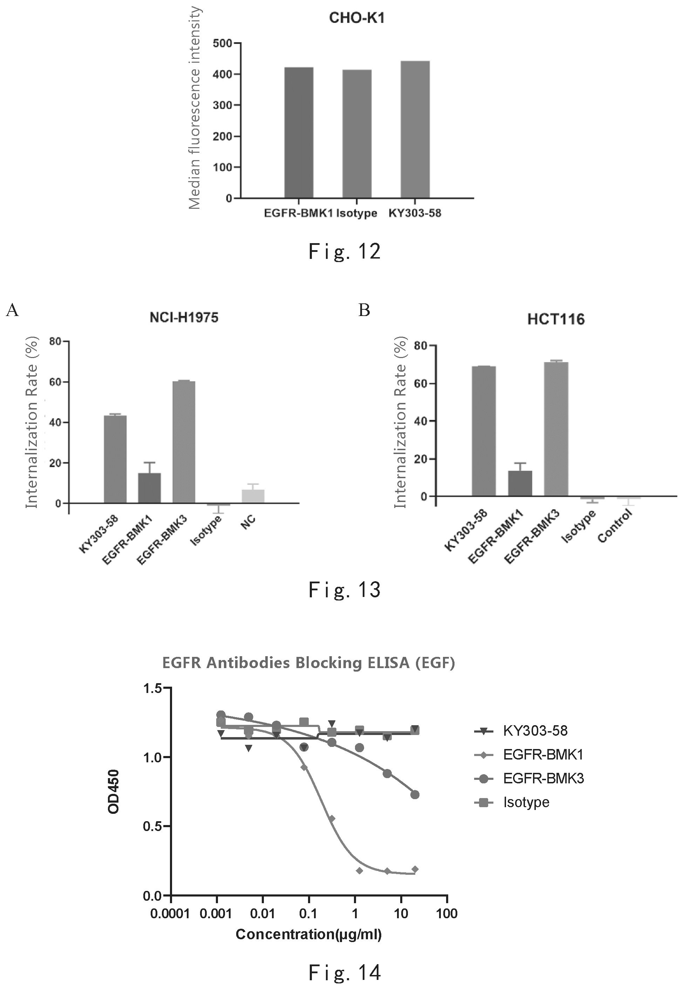

As shown in Fig. 12, the experimental results indicate that the EGFR antibody does not bind to CHO-K1 cells, indicating that the binding of the EGFR antibody at the cellular level is specific.

### Example 16. Evaluation of EGFR antibody internalization activity in tumor cells

Method:
1. HCT116 and NCI-H1975 cells were seeded in a 96-well transparent V-bottom plate at a density of 3E5 cells/well with 100 µL cell suspension per well;
2. 100 µL of diluted antibody solution to be tested was added to each well and incubated at 4°C for 1 hour. The cells were washed twice with 200 µL of culture medium;
3. 200 µL of culture medium was used to resuspend the cells. 100 µL of cell suspension from each well was transferred to a new 96-well V-bottom plate and incubated at 37°C for 2 hours, while the remaining cells in the prior wells were incubated at 4°C for 2 hours;
4. The supernatant was removed by centrifugation, and 100 µL of 1:100 diluted anti-Fc-PE antibody was added to each well and incubated at 4°C for 0.5 hour;
5. The cells were washed twice with 100 µL of PBS containing 1% FBS, and resuspended in 100 µL of PBS. Fluorescence signals were detected in the PE fluorescence channel, and median fluorescence intensity was observed to calculate the internalization rate.

As shown in Fig. 13, the experimental results indicate the internalization of EGFR antibody in both HCT116 and NCI-H1975 cells, where the internalization rate is cell type-dependent.

### Example 17. Evaluation of the ligand-blocking activity of EGFR antibody

Method:
1. The plate was coated with recombinant human EGFR antigen at 4°C overnight, and then washed twice with 200 µL of PBST (0.01% Tween-20);
2. Wells were blocked for 2 hours by adding 300 µL of 3% BSA solution per well, and then washed twice with 200 µL of PBST (0.01% Tween-20);
3. 100 µL of a mixture of EGFR antibody and different concentrations of HA-labeled EGF ligand was added and incubated at room temperature for 1 hour, and then washed twice with 200 µL of PBST (0.01% Tween-20);
4. 100 µL of anti-HA-HRP antibody dilution was added and incubated at 37°C for 0.5 hours, and then washed 4 times with 200 µL of PBST (0.01% Tween-20);
5. 100 µL of TMB substrate solution was added for color development for 5 minutes;
6. 50 µL of stop solution was added to terminate the reaction, and the absorbance value was read at 450 nm using a microplate reader.

As shown in Fig. 14, the experimental results indicate that KY303-58 has no ability to block the binding of EGF and EGFR.

### Example 18. Conjugation of EGFR antibody with MMAE

Method:
1. Histidine-acetate buffer (with a pH of 5.5), EDTA-2Na solution, TCEP solution, and EGFR antibody were sequentially added, so that the final concentration of EGFR antibody was 2-3 mg/mL, the final concentration of EDTA-2Na was 5 mM, and the amount of TCEP substance was 8 times that of the EGFR antibody. The mixture was well-mixed and reacted with shaking at 37 °C for 4 h;
2. 9 times the amount of the antibody substance of MMAE and DMSO solution were added, such that the total amount of DMSO accounted for 10% of the total reaction solution volume. The reaction was carried out with shaking at 4 °C for 2 h;
3. 7 times the amount of the antibody substance of L-cysteine was added and mixed well;
4. The above-mentioned reaction product was centrifuged. The supernatant was taken and transferred to a 10 KDa ultrafiltration tube, centrifuged, added with histidine-acetate buffer, and centrifuged again. This process was repeated 18 times to remove the free toxin that was not conjugated to the antibody.
5. The liquid in the ultrafiltration tube was taken out. The ultrafiltration tube was rinsed with histidine-acetate buffer. The rinsing liquid was mixed with the liquid taken-out previously. The absorbance was detected at 280 nm for calculation of antibody concentration.

### Example 19. Cytotoxicity Evaluation of EGFR Antibody-MMAE Product

Method:
1. NCI-H1975/HCT116/BxPC-3/MDA-MB-468/NCI-H1993/HT29/MDA-MB-231 cells were seeded into a 96-well plate with black wall and transparent bottom at 100 µL of cell suspension per well;
2. 10 µL of serially diluted ADC to be tested was added to each well, and the cells were cultured for 6 days;
3. 100 µL of CellTiter-Glo 2.0 Cell Viability Assay was added to each well, and the plate was shaken for mixing for 10 min. Then the fluorescence intensity was detected.

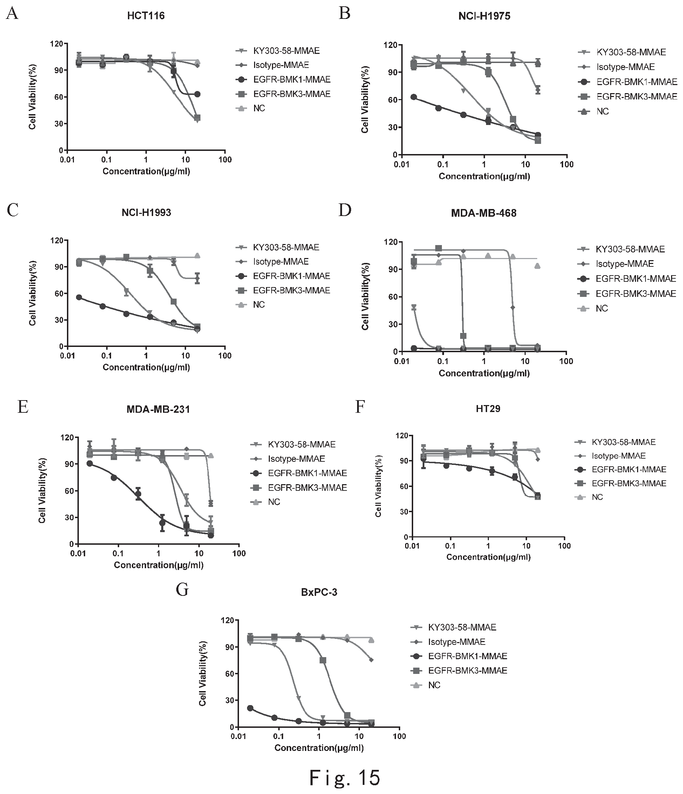

As shown in Fig. 15 and Table 9, EGFR antibody-MMAE can effectively inhibit the growth of various tumor cells.

**Table 9 IC50 values (µg/mL) of EGFR antibody-MMAE conjugate product against different tumor cells**

| Cell Line | KY303-58 | Cell Line | KY303-58 |
|---|---|---|---|
| HCT116 | 5.689 | BxPC-3 | 0.2344 |
| HT29 | 15.11 | NCI-H1993 | 0.3978 |
| NCI-H1975 | 0.4907 | MDA-MB-231 | 3.483 |
| MDA-MB-468 | 0.01033 | | |

### Example 20. EGFR Antibody-MMAE Product Effectively Inhibits the Growth of NCI-H1975 Xenograft Tumors

Method:
1. NCI-H1975 cells were inoculated subcutaneously into the right anterior flank of 15 female NCG mice;
2. When the tumors grew to about 100-150 mm³, the mice were randomly grouped into 3 groups with 5 mice in each group;
3. PBS or EGFR single-target ADC was administered via tail-vein injection once a week for 2 consecutive weeks, where doses of EGFR single-target ADC were 1.5 mg/kg and 3 mg/kg;
4. Tumor volume and body weight were measured twice a week, and the relationship between the changes in body weight and tumor volume of tumor-bearing mice and the administration time was recorded.

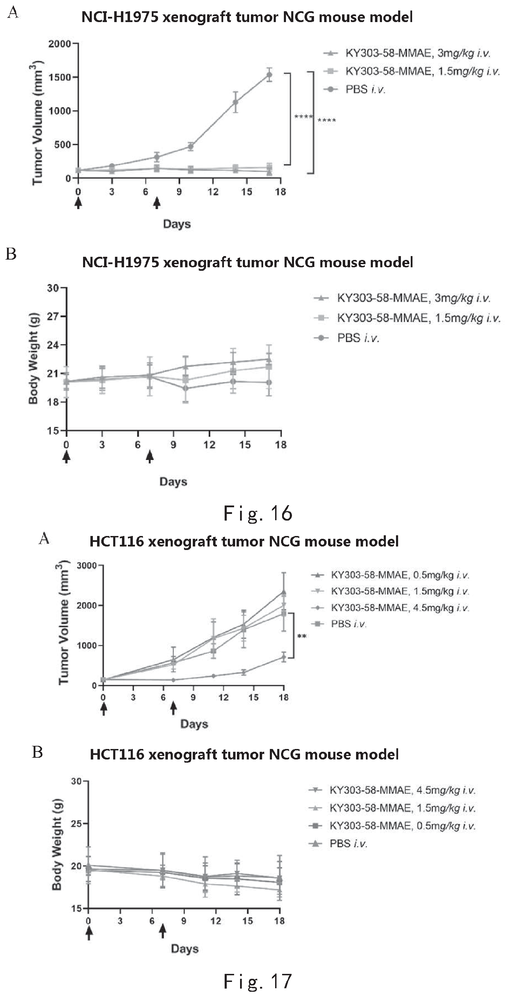

As shown in Fig. 16, the experimental results indicate that at the administration doses of 1.5 mg/kg and 3 mg/kg, the tumor growth inhibition rates on Day 14 were 89.5% and 93.5%, respectively. The above results indicate that EGFR single-target ADC can significantly inhibit the growth of NCI-H1975 xenograft tumors. Meanwhile, significant effect on the body weight of the mice was shown in neither the low-dose nor the high-dose group.

### Example 21. EGFR Antibody-MMAE Product Effectively Inhibits the Growth of HCT116 Xenograft Tumors

Method:
1. HCT116 cells were inoculated subcutaneously into the right anterior flank of 15 female NCG mice;
2. When the tumors grew to about 100-150mm³, the mice were randomly grouped into 3 groups with 5 mice in each group;
3. PBS or EGFR single-target ADC was administered via tail-vein injection once a week for 2 consecutive weeks, where doses of EGFR single-target ADC were 0.5 mg/kg, 1.5 mg/kg and 4.5 mg/kg;
4. Tumor volume and body weight were measured twice a week, and the relationship between the changes in body weight and tumor volume of tumor-bearing mice and the administration time was recorded.

As shown in Fig. 17, the experimental results indicate that at the administration doses of 4.5mg/kg, the tumor growth inhibition rates on Day 18 were 60.2%. The above results indicate that EGFR single-target ADC can inhibit the growth of HCT116 xenograft tumors. Meanwhile, significant effect on the body weight of the mice was shown in none of the low-dose, medium-dose and high-dose groups.

### Example 22. Design of MET-EGFR bispecific antibodies

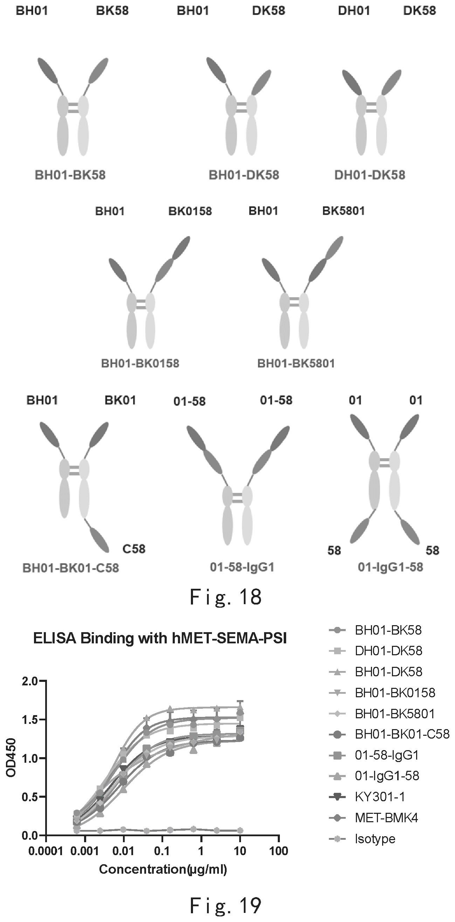

8 forms of MET-EGFR combined bispecific antibodies were designed, namely BH01-BK58, DH01-DK58, BH01-DK58, BH01-BK0158, BH01-BK5801, BH01-BK01-C58, 01-58-IgG1, and 01-IgG1-58. Among them, 01-58-IgG1 and 01-IgG1-58 use wild-type IgG1 FC; whereas the Fc regions of BH, BK, DH, and DK are mutated to form hole chains or knob chains to form a "knob-into-hole" connection between the two Fc chains. BH01-BK58, DH01-DK58, and BH01-DK58 have a "1+1" approximately symmetric "Y"-shaped structure. "DH" and "DK" are the hinge-truncated forms of "BH" and "BK". BH01-BK0158 and BH01-BK5801 have a "1+2" "Y"-shaped structure. Both the BK0158 arm and the BK5801 arm have CDR regions that can bind to MET and EGFR, only differing in the front-back positions. In BH01-BK01-C58, the EGFR-binding arm is located at the other end of the Fc of BH01-BK58. 01-58-IgG1 has a "2+2" approximately symmetric "Y"-shaped structure, that is, each Fab has CDR regions that bind to MET and EGFR. 01-IgG1-58 comprises 4 Fabs, located on both sides of the Fc respectively, and the CDR regions of the Fabs on the same side have the same sequence. The eight forms of the MET-EGFR bispecific antibodies are shown in Fig.18, and the amino acid sequences of the arms are shown in Table 10.

**Table 10 Amino acid sequences of MET-EGFR bispecific antibodies**

| **Name of sequence** | **Amino acid sequence** |
|---|---|
| BH01 | QVQLVESGGGLVQAGGSLRLSCAASGRTDSTYAMGWFRQAPGKERESVAAISW SGGSTYYADSVKGRFTISRDNAKHTVYLQMNSLKPEDTAVYYCAADHRTGSTR FRTAQYDYDYWGQGTQVTVSSEPKSSDKTHTCPPCPAPELLGGPSVFLFPPKPK DTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSR DELTKNQVSLSCAVKGFYPSDIAVEWESNGQPENNYKTTPPVLDSDGSFFLVSK LTVDKSRWQQGNVFSCSVMHEALHNHYTQKSLSLSPGK (SEQ ID NO: 2) |
| BK58 | EVQVVESGGGLVQAGGSLRLSCAASGSTFSSYAMGWYRQAPGKQRESVAFISS GGSTNVAESVKGRFTISRDNAKNTVYLQMNNLKAEDTAVYYCNLHPRVNWGQ GTQVTVSSEPKSSDKTHTCPPCPAPELLGGPSVFLFPPKPKDTLMISRTPEVTCVV VDVSHEDPEVKFNWYVDGVEVHNAKTKPREEQYNSTYRVVSVLTVLHQDWLN GKEYKCKVSNKALPAPIEKTISKAKGQPREPQVYTLPPSRDELTKNQVSLWCLV KGFYPSDIAVEWESNGQPENNYKTTPPVLDSDGSFFLYSKLTVDKSRWQQGNVF SCSVMHEALHNHYTQKSLSLSPGK (SEQ ID NO: 5) |
| DH01 | QVQLVESGGGLVQAGGSLRLSCAASGRTDSTYAMGWFRQAPGKERESVAAISW SGGSTYYADSVKGRFTISRDNAKHTVYLQMNSLKPEDTAVYYCAADHRTGSTR |
| | FRTAQYDYDYWGQGTQVTVSSADKTHTCPPCPAPELLGGPSVFLFPPKPKDTLM ISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSRDELT KNQVSLSCAVKGFYPSDIAVEWESNGQPENNYKTTPPVLDSDGSFFLVSKLTVD KSRWQQGNVFSCSVMHEALHNHYTQKSLSLSPGK (SEQ ID NO: 6) |
| DK58 | EVQVVESGGGLVQAGGSLRLSCAASGSTFSSYAMGWYRQAPGKQRESVAFISS GGSTNVAESVKGRFTISRDNAKNTVYLQMNNLKAEDTAVYYCNLHPRVNWGQ GTQVTVSSADKTHTCPPCPAPELLGGPSVFLFPPKPKDTLMISRTPEVTCVVVDV SHEDPEVKFNWYVDGVEVHNAKTKPREEQYNSTYRVVSVLTVLHQDWLNGKE YKCKVSNKALPAPIEKTISKAKGQPREPQVYTLPPSRDELTKNQVSLWCLVKGF YPSDIAVEWESNGQPENNYKTTPPVLDSDGSFFLYSKLTVDKSRWQQGNVFSCS VMHEALHNHYTQKSLSLSPGK (SEQ ID NO: 7) |
| BK0158 | QVQLVESGGGLVQAGGSLRLSCAASGRTDSTYAMGWFRQAPGKERESVAAISW SGGSTYYADSVKGRFTISRDNAKHTVYLQMNSLKPEDTAVYYCAADHRTGSTR FRTAQYDYDYWGQGTQVTVSSGGGGSGGGGSGGGGSEVQVVESGGGLVQAG GSLRLSCAASGSTFSSYAMGWYRQAPGKQRESVAFISSGGSTNVAESVKGRFTIS RDNAKNTVYLQMNNLKAEDTAVYYCNLHPRVNWGQGTQVTVSSEPKSSDKTH TCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPI EKTISKAKGQPREPQVYTLPPSRDELTKNQVSLWCLVKGFYPSDIAVEWESNGQ PENNYKTTPPVLDSDGSFFLYSKLTVDKSRWQQGNVFSCSVMHEALHNHYTQK SLSLSPGK (SEQ ID NO: 8) |
| BK5801 | EVQVVESGGGLVQAGGSLRLSCAASGSTFSSYAMGWYRQAPGKQRESVAFISS GGSTNVAESVKGRFTISRDNAKNTVYLQMNNLKAEDTAVYYCNLHPRVNWGQ GTQVTVSSGGGGSGGGGSGGGGSQVQLVESGGGLVQAGGSLRLSCAASGRTDS TYAMGWFRQAPGKERESVAAISWSGGSTYYADSVKGRFTISRDNAKHTVYLQM NSLKPEDTAVYYCAADHRTGSTRFRTAQYDYDYWGQGTQVTVSSEPKSSDKTH TCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPI EKTISKAKGQPREPQVYTLPPSRDELTKNQVSLWCLVKGFYPSDIAVEWESNGQ PENNYKTTPPVLDSDGSFFLYSKLTVDKSRWQQGNVFSCSVMHEALHNHYTQK SLSLSPGK (SEQ ID NO: 9) |
| BK01-C5 8 | QVQLVESGGGLVQAGGSLRLSCAASGRTDSTYAMGWFRQAPGKERESVAAISW SGGSTYYADSVKGRFTISRDNAKHTVYLQMNSLKPEDTAVYYCAADHRTGSTR FRTAQYDYDYWGQGTQVTVSSEPKSSDKTHTCPPCPAPELLGGPSVFLFPPKPK DTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSR DELTKNQVSLWCLVKGFYPSDIAVEWESNGQPENNYKTTPPVLDSDGSFFLYSK LTVDKSRWQQGNVFSCSVMHEALHNHYTQKSLSLSPGKGGGGSGGGGSEVQV VESGGGLVQAGGSLRLSCAASGSTFSSYAMGWYRQAPGKQRESVAFISSGGSTN VAESVKGRFTISRDNAKNTVYLQMNNLKAEDTAVYYCNLHPRVNWGQGTQVT VSS- (SEQ ID NO: 10) |
| 01-58-Ig G1 | QVQLVESGGGLVQAGGSLRLSCAASGRTDSTYAMGWFRQAPGKERESVAAISW SGGSTYYADSVKGRFTISRDNAKHTVYLQMNSLKPEDTAVYYCAADHRTGSTR FRTAQYDYDYWGQGTQVTVSSGGGGSGGGGSGGGGSEVQVVESGGGLVQAG GSLRLSCAASGSTFSSYAMGWYRQAPGKQRESVAFISSGGSTNVAESVKGRFTIS RDNAKNTVYLQMNNLKAEDTAVYYCNLHPRVNWGQGTQVTVSSEPKSSDKTH TCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPI EKTISKAKGQPREPQVYTLPPSRDELTKNQVSLTCLVKGFYPSDIAVEWESNGQP ENNYKTTPPVLDSDGSFFLYSKLTVDKSRWQQGNVFSCSVMHEALHNHYTQKS LSLSPGK (SEQ ID NO: 11) |
| 01-IgG1-58 | QVQLVESGGGLVQAGGSLRLSCAASGRTDSTYAMGWFRQAPGKERESVAAISW SGGSTYYADSVKGRFTISRDNAKHTVYLQMNSLKPEDTAVYYCAADHRTGSTR FRTAQYDYDYWGQGTQVTVSSEPKSSDKTHTCPPCPAPELLGGPSVFLFPPKPK DTLMISRTPEVTCVVVDVSHEDPEVKFNWYVDGVEVHNAKTKPREEQYNSTYR |
| | VVSVLTVLHQDWLNGKEYKCKVSNKALPAPIEKTISKAKGQPREPQVYTLPPSR DELTKNQVSLTCLVKGFYPSDIAVEWESNGQPENNYKTTPPVLDSDGSFFLYSK LTVDKSRWQQGNVFSCSVMHEALHNHYTQKSLSLSPGKGGGGSGGGGSEVQV VESGGGLVQAGGSLRLSCAASGSTFSSYAMGWYRQAPGKQRESVAFISSGGSTN VAESVKGRFTISRDNAKNTVYLQMNNLKAEDTAVYYCNLHPRVNWGQGTQVT VSS (SEQ ID NO: 12) |

### Example 23. Transient transfection and expression of MET - EGFR bispecific antibodies

Method:
1. 293T cells were transiently transfected with BH01-BK58 plasmids (co-transfection of BH01 and BK58 double plasmids), DH01-DK58 (co-transfection of DH01 and DK58 double plasmids), BH01-DK58 (co-transfection of BH01 and DK58 double plasmids), BH01-BK0158 (co-transfection of BH01 and BK0158 double plasmids), BH01-BK5801 (co-transfection of BH01 and BK5801 double plasmids), BH01-BK01-C58 (co-transfection of BH01 and BK01C58 double plasmids), 01-58-IgG1 (transfection of a single 01-58-IgG1 plasmid), and 01-IgG1-58 (transfection of a single 01-IgG1-58 plasmid).
2. Cells were lysed, subjected to affinity purification with protein A, and the eluate was collected and concentrated.

**Table 11 Expression levels and purities of 8 MET - EGFR bispecific antibodies**

| **Antibody Name** | **Antibody Subtype** | **Antibody Concentration (mg/ml)** | **Antibody Purity** | |
|---|---|---|---|---|
| | | | **SEC 280nm, %** | **SDS-PAGE, %** |
| BH01-BK58 | hIgG1 Fc | 2.87 | >95 | >95 |
| DH01-DK58 | hIgG1 Fc | 2.58 | >90 | >95 |
| BH01-DK58 | hIgG1 Fc | 2.65 | >95 | >95 |
| BH01-BK0158 | hIgG1 Fc | 2.89 | >90 | >95 |
| BH01-BK5801 | hIgG1 Fc | 2.83 | >90 | >95 |
| BH01-BK01-C58 | hIgG1 Fc | 2.44 | >80 | >95 |
| 01-58-IgG1 | hIgG1 Fc | 3.49 | >95 | >95 |
| 01-IgG1-58 | hIgG1 Fc | 2.60 | >95 | >90 |

### Example 24. Binding activity of MET-EGFR bispecific antibody to recombinant human MET antigen

Method:
1. The plate was coated with recombinant human MET antigen at 4°C overnight, and then washed twice with 200 µL of PBST (0.01% Tween-20);
2. Wells were blocked for 2 hours by adding 300 µL of 3% BSA solution per well, and then washed twice with 200 µL of PBST (0.01% Tween-20);
3. MET-EGFR bispecific antibody was added and incubated at room temperature for 1 hour, and then washed twice with 200 µL of PBST (0.01% Tween-20);
4. 100 µL of anti-Fc-HRP antibody dilution was added and incubated at 37°C for 0.5 hours, and then washed 4 times with 200 µL of PBST (0.01% Tween-20);
5. 100 µL of TMB substrate solution was added for color development for 5 minutes;
6. 50 µL of stop solution was added to terminate the reaction, and the absorbance value was read at 450 nm using a microplate reader.

As shown in Fig. 19 and Table 12, the experimental results indicate that the MET-EGFR bispecific antibody is capable of binding MET antigen.

**Table 12 EC₅₀ value of the MET-EGFR antibody binding to recombinant human MET antigen**

| **Antibody Name** | **EC50 (µg/mL)** | **Antibody Name** | **EC50 (µg/mL)** |
|---|---|---|---|
| BH01-BK58 | 0.005770 | BH01-BK5801 | 0.005053 |
| DH01-DK58 | 0.003316 | BH01-BK01-C58 | 0.007529 |
| BH01-DK58 | 0.005423 | 01-58-IgG1 | 0.007075 |
| BH01-BK0158 | <0.00061 | 01-IgG1-58 | 0.009844 |

### Example 25. Binding activity of MET-EGFR bispecific antibody to recombinant human EGFR antigen

Method:
1. The plate was coated with recombinant human EGFR antigen at 4°C overnight, and then washed twice with 200 µL of PBST (0.01% Tween-20);
2. Wells were blocked for 2 hours by adding 300 µL of 3% BSA solution per well, and then washed twice with 200 µL of PBST (0.01% Tween-20);
3. MET-EGFR bispecific antibody was added and incubated at room temperature for 1 hour, and then washed twice with 200 µL of PBST (0.01% Tween-20);
4. 100 µL of anti-Fc-HRP antibody dilution was added and incubated at 37°C for 0.5 hours, and then washed 4 times with 200 µL of PBST (0.01% Tween-20);
5. 100 µL of TMB substrate solution was added for color development for 5 minutes;
6. 50 µL of stop solution was added to terminate the reaction, and the absorbance value was read at 450 nm using a microplate reader.

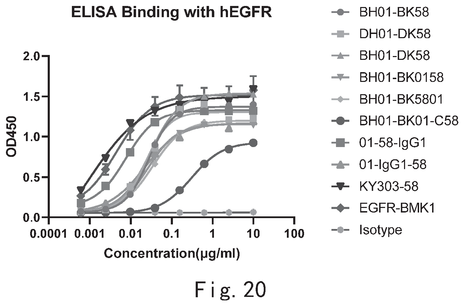

As shown in Fig. 20 and Table 13, the experimental results indicate that the MET-EGFR bispecific antibody is capable of binding EGFR antigen. Among them, BH01-BK58, DH01-DK58, BH01-DK58, and 01-58-IgG1 have strong binding ability to hEGFR.

**Table 13 EC₅₀ value of the MET-EGFR antibody binding to recombinant human EGFR antigen**

| **Antibody Name** | **EC50 (µg/mL)** | **Antibody Name** | **EC50 (µg/mL)** |
|---|---|---|---|
| BH01-BK58 | 0.02742 | BH01-BK5801 | 0.03424 |
| DH01-DK58 | 0.02320 | BH01-BK01-C58 | 0.3065 |
| BH01-DK58 | 0.02991 | 01-58-IgG1 | 0.007556 |
| BH01-BK0158 | 0.02656 | 01-IgG1-58 | 0.02139 |

### Example 26. Species cross-reactivity of the candidate MET-EGFR bispecific antibodies to MET and EGFR antigens

Method:
1. The plate was coated with recombinant human, mouse and cynomolgus MET or EGFR antigen at 4°C overnight, respectively, and then washed twice with 200 µL of PBST (0.01% Tween-20);
2. Wells were blocked for 2 hours by adding 300 µL of 3% BSA solution per well, and then washed twice with 200 µL of PBST (0.01% Tween-20);
3. MET-EGFR bispecific antibody was added and incubated at room temperature for 1 hour, and then washed twice with 200 µL of PBST (0.01% Tween-20);
4. 100 µL of anti-Fc-HRP antibody dilution was added and incubated at 37°C for 0.5 hours, and then washed 4 times with 200 µL of PBST (0.01% Tween-20);
5. 100 µL of TMB substrate solution was added for color development for 5 minutes;
6. 50 µL of stop solution was added to terminate the reaction, and the absorbance value was read at 450 nm using a microplate reader.

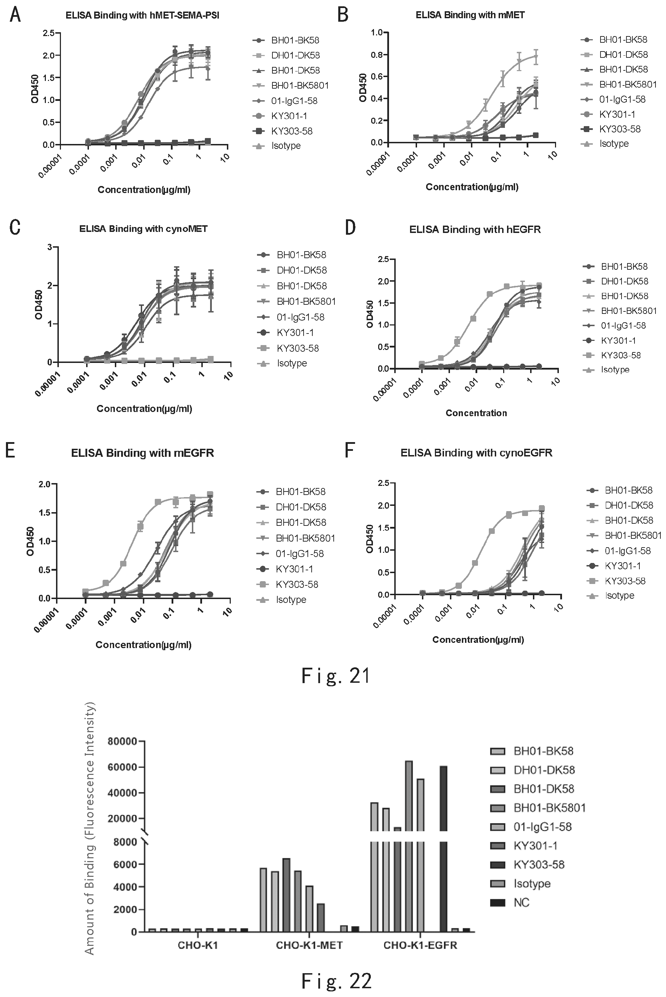

As shown in Fig. 21 and Tables 14-15, the experimental results indicate that the MET-EGFR bispecific antibody is capable of binding to human, mouse, and cynomolgus MET antigens, having the species-cross activity.

**Table 14 EC₅₀ values of MET-EGFR antibody binding to recombinant mouse and cynomolgus MET antigens (µg/mL)**

| **Antibody Name** | **mMET** | **cynoMET** |
|---|---|---|
| BH01-BK58 | 0.4434 | 0.008300 |
| DH01-DK58 | 0.3839 | 0.008959 |
| BH01-DK58 | 0.2269 | 0.009824 |
| BH01-BK5801 | 0.05034 | 0.008582 |
| 01-IgG1-58 | 0.07469 | 0.01104 |

**Table 15 EC₅₀ values of MET-EGFR antibody binding to recombinant mouse and cynomolgus EGFR antigens (µg/mL)**

| **Antibody Name** | **mEGFR** | **cynoEGFR** |
|---|---|---|
| BH01-BK58 | 0.08091 | 0.6687 |
| DH01-DK58 | 0.09397 | 0.8715 |
| BH01-DK58 | 0.06836 | 0.3915 |
| BH01-BK5801 | 0.05819 | 0.4461 |
| 01-IgG1-58 | 0.02834 | 0.3301 |

### Example 27. Cell-binding specificity of candidate MET - EGFR bispecific antibodies

Method:
1. CHO-K1, CHO-K1-EGFR cells (CHO-K1 cells expressing human EGFR protein) and CHO-K1-MET cells (CHO-K1 cells expressing human MET protein) were seeded in a 96-well transparent V-bottom plate at a density of 1E5 cells/well, with 100 µL cell suspension per well;
2. 100 µL of diluted antibody solution to be tested was added to each well and incubated at 4°C for 1 hour. The cells were washed twice with 200 µL of PBS containing 1% FBS at 4°C;
3. 100 µL of 1:100 diluted anti-Fc-PE antibody was added to each well, and incubated at 4°C for 0.5 hour;
4. The cells were washed twice with 100 µL of PBS containing 1% FBS, and resuspended in 100 µL of PBS. Fluorescence signals were detected in the PE fluorescence channel, and median fluorescence intensity was observed.

As shown in Fig. 22, the experimental results indicate that the MET-EGFR bispecific antibodies do not bind to CHO-K1 antigen-negative cells but bind to CHO-K1 antigen-positive cells (CHO-K1-EGFR and CHO-K1-MET), suggesting that the binding is specific at the cellular level.

### Example 28. Conjugation of MET-EGFR bispecific antibodies with MMAE

Method:
1. Histidine-acetate buffer (with a pH of 5.5), EDTA-2Na solution, TCEP solution, and EGFR antibody were sequentially added, so that the final concentration of MET-EGFR antibody was 2-3 mg/mL, the final concentration of EDTA-2Na was 5 mM, and the amount of TCEP substance was 8 times that of the EGFR antibody. The mixture was well-mixed and reacted with shaking at 37 °C for 4 h;
2.9 times the amount of the antibody substance of MMAE and DMSO solution were added, such that the total amount of DMSO accounted for 10% of the total reaction solution volume. The reaction was carried out with shaking at 4 °C for 2 h;
3. 7 times the amount of the antibody substance of L-cysteine was added and mixed well;
4. The above-mentioned reaction product was centrifuged. The supernatant was taken and transferred to a 10 KDa ultrafiltration tube, centrifuged, added with histidine-acetate buffer, and centrifuged again. This process was repeated 18 times to remove the free toxin that was not conjugated to the antibody.
5. The liquid in the ultrafiltration tube was taken out. The ultrafiltration tube was rinsed with histidine-acetate buffer. The rinsing liquid was mixed with the liquid taken-out previously. The absorbance was detected at 280 nm for calculation of antibody concentration.

### Example 29. MET-EGFR antibody-MMAE product is more effective than single-target antibody-MMAE in inhibiting the growth of HCT116 tumor cells

Method:
1. HCT116 cells were seeded into a 96-well plate with black wall and transparent bottom at 100 µL of cell suspension per well;
2. 10 µL of serially diluted ADC to be tested was added to each well, and the cells were cultured for 6 days;
3. 100 µL of CellTiter-Glo 2.0 Cell Viability Assay was added to each well, and the plate was shaken for mixing for 10 min. Then the fluorescence intensity was detected.

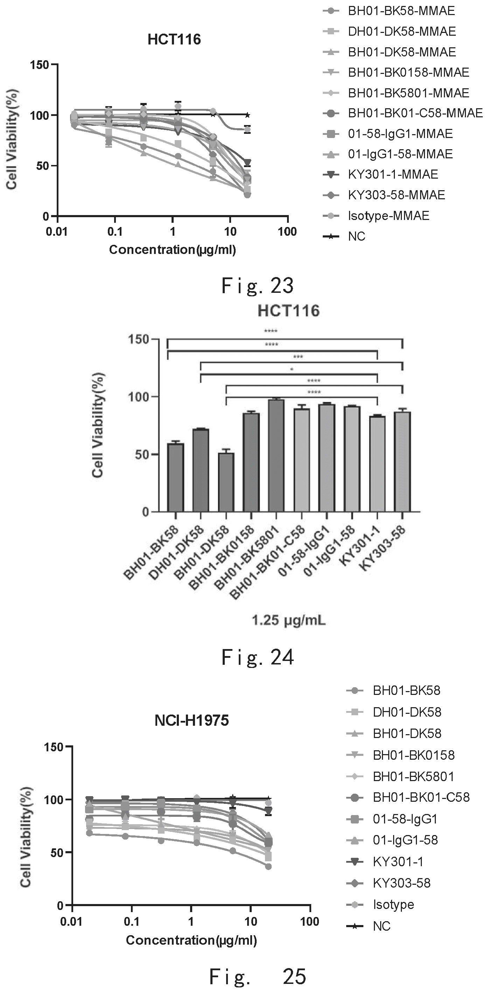

As shown in Fig. 23, the experimental results indicate that all MET-EGFR antibody-MMAEs can effectively inhibit the growth of HCT116 tumor cells. Among them, BH01-BK58-MMAE, DH01-DK58-MMAE, and BH01-DK58-MMAE (KY-0301) have better tumor-killing effects and can effectively inhibit tumor growth at a low concentration. As shown in Fig. 24 and Table 16, at the same dose, the bispecific MET-EGFR antibody conjugate BH01-DK58-MMAE (KY-0301) of the present invention has significantly better cell-killing performance against HCT116 target cells than the MET single-target ADC (KY301-01-MMAE) and the EGFR single-target ADC (KY303-58-MMAE), showing a synergistic effect.

**Table 16. Cell survival rate of HCT116 at an ADC concentration of 1.25 µg/mL**

| Antibody ADC (1.25 µg/mL) | Cell survival rate (%) |
|---|---|
| BH01-BK58 | 59.50 |
| DH01-DK58 | 72.04 |
| BH01-DK58 | 51.69 |
| BH01-BK0158 | 86.12 |
| BH01-BK5801 | 97.77 |
| BH01-BK01-C58 | 90.09 |
| 01-58-IgG1 | 93.77 |
| 01-IgG1-58 | 91.93 |
| KY301-01 | 83.42 |
| KY303-58 | 87.30 |

### Example 30. MET-EGFR antibody-MMAE product is more effective than single-target antibody-MMAE in inhibiting the growth of NCI-H1975 tumor cells

Method:
1. NCI-H1975 cells were seeded into a 96-well plate with black wall and transparent bottom at 100 µL of cell suspension per well;
2. 10 µL of serially diluted ADC to be tested was added to each well, and the cells were cultured for 6 days;
3. 100 µL of CellTiter-Glo 2.0 Cell Viability Assay was added to each well, and the plate was shaken for mixing for 10 min. Then the fluorescence intensity was detected.

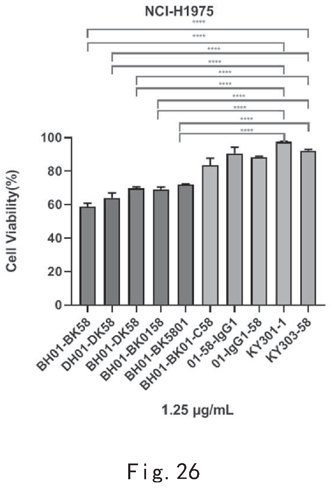

As shown in Fig. 25, the experimental results indicate that all MET-EGFR antibody-MMAEs can effectively inhibit the growth of HCT116 tumor cells. Among them, BH01-BK58-MMAE, DH01-DK58-MMAE, BH01-DK58-MMAE, BK01-BH0158-MMAE, BK01-BH5801-MMAE have better tumor-killing effects. As shown in Fig. 26 and Table 17, at the same dose, the bispecific MET-EGFR antibody conjugate BH01-DK58-MMAE (KY-0301) of the present invention has significantly better cell-killing performance against NCI-H1975 target cells than the MET single-target ADC (KY301-01-MMAE) and the EGFR single-target ADC (KY303-58-MMAE), showing a synergistic effect.

**Table 17. Cell survival rate of NCI-H1975 at an ADC concentration of 1.25 µg/mL**

| Antibody ADC (1.25 µg/mL) | Cell survival rate (%) |
|---|---|
| BH01-BK58-MMAE | 58.89 |
| DH01-DK58-MMAE | 64.17 |
| BH01-DK58-MMAE | 69.67 |
| BH01-BK0158-MMAE | 69.23 |
| BH01-BK5801-MMAE | 72.17 |
| BH01-BK01-C58-MMAE | 83.66 |
| 01-58-IgG1-MMAE | 90.52 |
| 01-IgG1-58-MMAE | 88.36 |
| KY301-1-MMAE | 97.44 |
| KY303-58-MMAE | 92.25 |

### Example 31. Binding affinity and kinetic constants of BH01-BK58 and BH01-DK58

The equilibrium dissociation constants (KD values) of MET and EGFR binding to MET-EGFR antibodies were determined using a real-time surface plasmon resonance biosensor assay on a Biacore 2000 instrument.

Method:
1. Antibody capture: 1×PBST (1×PBS solution containing 0.05% Tween 20, pH 7.4) buffer was used as the running buffer. Each antibody was diluted to 1 µg/mL with PBS buffer. The flow rate was set as 10 µL/min, and antibodies were continuously captured by a Protein A chip for 60 s;
2. Binding detecting: Human recombinant MET antigen was diluted with 1 ×PBST to different concentrations (0 nM, 31.25 nM, 62.5 nM, 125 nM, 250 nM, 500 nM, 1000 nM, 2000 nM) and injected onto the antibody-captured surface at a flow rate of 30 µL/min. The binding of the antigen to the antibody was detected for 120 s;
3. Dissociation detecting: the dissociation of the MET antigen was detected in the 1 ×PBST running buffer for 360 s;
4. Regeneration: regeneration was performed using Gly-HC1 buffer (pH 1.5) at a flow rate of 30 µL/min for 30 s, and then the chip was stabilized with 1 ×PBST for 60 s;
5. Antibody capture: 1×PBST (1×PBS solution containing 0.05% Tween 20, pH 7.4) buffer was used as the running buffer. Each antibody was diluted to 1 µg/mL with PBS buffer. The flow rate was set as 10 µL/min, and antibodies were continuously captured by a Protein A chip for 60 s;
6. Binding detecting: Human recombinant EGFR antigen was diluted with 1 ×PBST to different concentrations (0 nM, 31.25 nM, 62.5 nM, 125 nM, 250 nM, 500 nM, 1000 nM, and 2000 nM) and injected onto the antibody-captured surface at a flow rate of 30 µL/min. The binding of the antigen to the antibody was detected for 120 s;
7. Dissociation detecting: The dissociation of the MET antigen was detected in the 1 ×PBST running buffer for 360 s;
8. Regeneration: Regeneration was performed using Gly-HC1 buffer (pH 1.5) at a flow rate of 30 µL/min for 30 s;

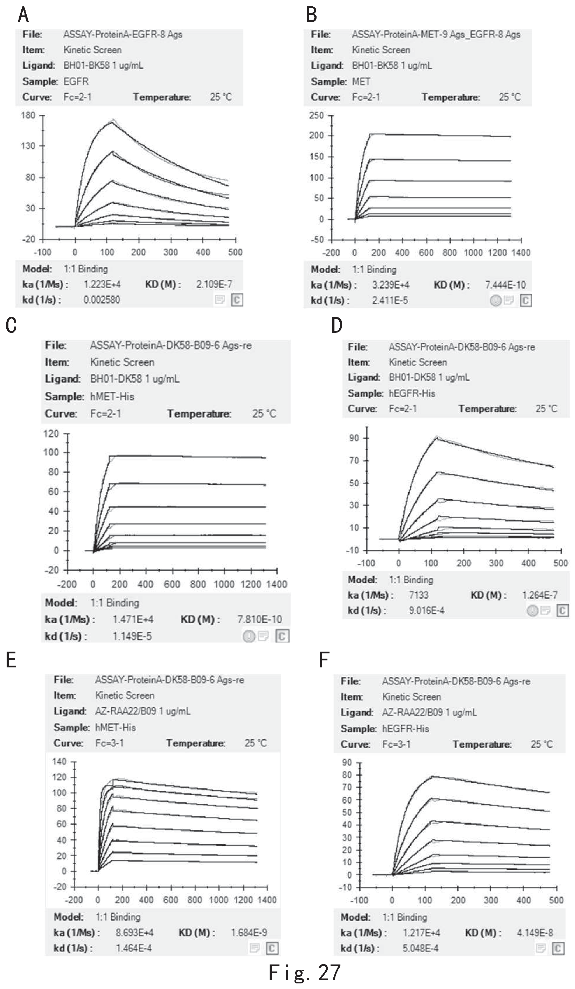

The association-dissociation kinetic curves of BH01-BK58, BH01-DK58, and the control antibody AZ-RAA22/B09 with the target antigens are shown in Fig. 27. The affinity constants are summarized in Table 18. The KD values of BH01-DK58 and BH01-BK58 for hMET are similar, which are twice the affinity of the MET antibody KY301-01, indicating that the affinity of BH01-DK58 and BH01-BK58 for hMET is weaker than that of KY301-01. Comparing the affinities for hEGFR, the order from strong to weak is: KY303-58, BH01-BK58, BH01-DK58. BH01-DK58 and BH01-BK58 have the same VHH sequence, and the difference in their affinities for hEGFR is due to the "short-arm" hinge design of DK58, which reduces the toxicity to normal tissue cells caused by targeting EGFR.

**Table 18 Binding affinities of MET-EGFR bispecific antibodies to recombinant human MET or EGFR antigen**

| Antigen | Antibody | ka (1/Ms) | kd (1/s) | KD (M) |
|---|---|---|---|---|
| hMET | KY301-1 | 6.63E+04 | 2.16E-05 | 3.26E-10 |
| hMET | BH01-DK58 | 1.47E+04 | 1.15E-05 | 7.81E-10 |
| hMET | BH01-BK58 | 3.24E+04 | 2.41E-05 | 7.44E-10 |
| hEGFR | KY303-58 | 1.60E+04 | 9.13E-04 | 5.69E-08 |
| hEGFR | BH01-DK58 | 7.13E+03 | 9.02E-04 | 1.26E-07 |
| hEGFR | BH01-BK58 | 1.22E+04 | 2.58E-03 | 2.11E-07 |

As shown in Tables 19 and 20, the affinity of BH01-DK58 for hMET and hEGFR differs by 161-fold., which is much greater than the difference in affinities of the control antibody AZ-RAA22/B09 for hMET and hEGFR (of which the affinity for hMET and hEGFR differs by 24.7-fold). The design of the difference in affinity for hMET and hEGFR aims to reduce the binding priority of the MET-EGFR antibody to EGFR, further reduce the toxic side effects caused by targeting EGFR, and improve safety.

**Table 19 Binding affinities of BH01-DK58 to recombinant human, mouse, and monkey MET or EGFR**

| Antigen | Antibody | ka (1/Ms) | kd (1/s) | KD (M) |
|---|---|---|---|---|
| hMET | | 1.47E+04 | 1.15E-05 | 7.81E-10 |
| mMET | | 1.07E+04 | 4.29E-05 | 4.01E-09 |
| cynoMET | BH01-DK58 | 7.78E+03 | 2.77E-05 | 3.56E-09 |
| hEGFR | | 7.13E+03 | 9.02E-04 | 1.26E-07 |
| mEGFR | | 1.72E+04 | 1.81E-03 | 1.05E-07 |
| cynoEGFR | | 4.34E+03 | 4.49E-05 | 1.03E-08 |

**Table 20 Binding affinities of the control MET-EGFR bispecific antibody (AZ-RAA22/B09) to recombinant human, mouse, and monkey MET or EGFR**

| Sample | Ligand | ka (1/Ms) | kd (1/s) | KD (M) |
|---|---|---|---|---|
| hMET | | 8.69E+04 | 1.46E-04 | 1.68E-09 |
| mMET | | 1.48E+04 | 3.33E-05 | 2.25E-09 |
| cynoMET | AZ-RAA22/B09 | 6.42E+04 | 1.39E-04 | 2.17E-09 |
| hEGFR | | 1.22E+04 | 5.05E-04 | 4.15E-08 |
| mEGFR | | 1.73E+04 | 2.24E-03 | 1.30E-07 |
| cynoEGFR | | 2.34E+04 | 2.93E-03 | 1.25E-07 |

### Example 32. Conjugation of MMAE to BH01-BK58 and BH01-DK58 bispecific antibodies

Method:
1. Histidine-acetate buffer (with a pH of 5.5), EDTA-2Na solution, TCEP solution, and EGFR antibody were sequentially added, so that the final concentration of MET-EGFR antibody was 2-3 mg/mL, the final concentration of EDTA-2Na was 5 mM, and the amount of TCEP substance was 8 times that of the EGFR antibody. The mixture was well-mixed and reacted with shaking at 37 °C for 4 h;
2. 9 times the amount of the antibody substance of MMAE and DMSO solution were added, such that the total amount of DMSO accounted for 10% of the total reaction solution volume. The reaction was carried out with shaking at 4 °C for 2 h;
3. 7 times the amount of the antibody substance of L-cysteine was added and mixed well;
4. The above-mentioned reaction product was centrifuged. The supernatant was taken and transferred to a 10 KDa ultrafiltration tube, centrifuged, added with histidine-acetate buffer, and centrifuged again. This process was repeated 18 times to remove the free toxin that was not conjugated to the antibody.
5. The liquid in the ultrafiltration tube was taken out. The ultrafiltration tube was rinsed with histidine-acetate buffer. The rinsing liquid was mixed with the liquid taken-out previously. The absorbance was detected at 280 nm for calculation of antibody concentration.

### Example 33. Detection of DAR values and free toxins in the BH01-BK58 and BH01-DK58 bispecific antibody-MMAE conjugates

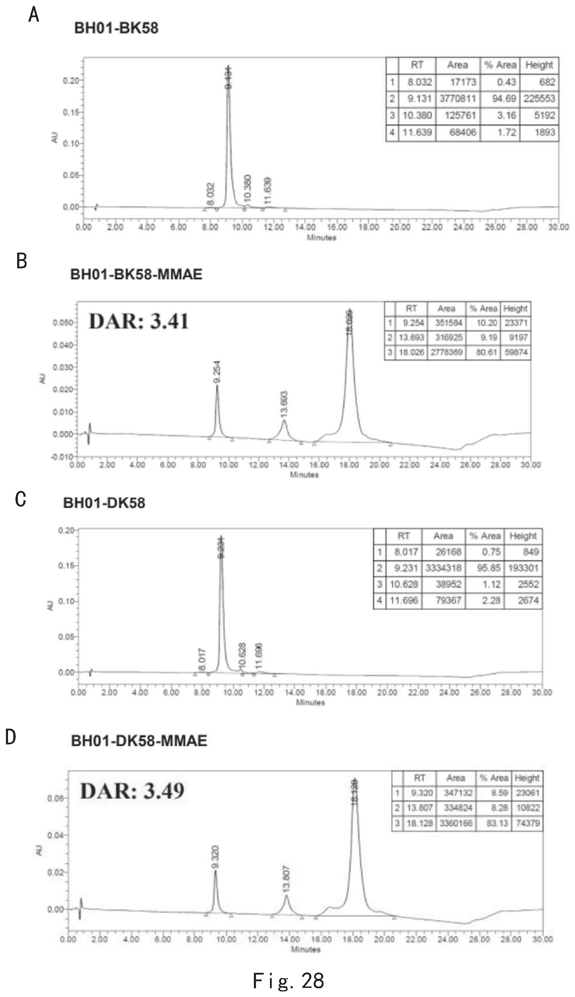

Method:
Detection of DAR values:
50 µg of BH01-BK58 and BH01-DK58 bispecific antibodies MMAE-conjugate products were injected into a high performance liquid chromatography column, and eluted with different mobile phases. The chromatogram was recorded simultaneously (Fig. 28). The DAR value was calculated according to the formula: DAR = Σ(relative peak area × number of loaded drugs)/100;
Detection of free toxins:
80 µg of the conjugate sample was mixed with 3 µL of DMSO sample, and then was mixed well with 60 µL of a mixture of anhydrous methanol-acetonitrile containing sodium chloride. 20 µL of the mixture was taken and injected into high-performance liquid chromatography for elution, and the chromatogram was recorded. According to the peak area values of the toxin standard at different concentrations, a regression equation was fitted to calculate the content of free toxins in the sample to be tested: Free Drug (mol/mol%) = molar concentration of residual small molecules/molar concentration of antibody × 100.

**Table 21 DAR values and free toxin contents of MET-EGFR antibody-MMAE products**

| Name | DAR | Free-Drug(linker-payload) | | Free-Drug(Total) | |
|---|---|---|---|---|---|
| | | mol/mol % | × 10⁻³mg/ml | mol/mol % | × 10⁻³mg/ml |
| BH01-BK58-MMAE | 3.41 | 9.603 | 3.41 | 2.26 | 3.39 |
| BH01-DK58-MMAE | 3.49 | 10.223 | 3.49 | 2.19 | 3.48 |

**Table 22 DAR values and product contents of MET-EGFR antibody-MMAE products**

| Name | Naked antibody HIC purity (%) | | DAR | HIC purity (%) | |
|---|---|---|---|---|---|
| BH01-BK58-MMAE | Peak 1 | 0.43 | 3.41 | DAR1 | 0 |
| | Peak 2 | 94.69 | | DAR2 | 9.19 |
| | Peak 3 | 3.16 | | DAR3 | 0 |
| | Peak 4 | 1.72 | | DAR4 | 80.61 |
| BH01-DK58-MMAE | Peak 1 | 0.75 | 3.49 | DAR1 | 0 |
| | Peak 2 | 95.85 | | DAR2 | 8.28 |
| | Peak 3 | 1.12 | | DAR3 | 0 |
| | Peak 4 | 2.28 | | DAR4 | 83.13 |

### Example 34. Killing ability of BH01-BK58-MMAE and BH01-DK58-MMAE against tumor cells

Method:
1. BxPC-3/ HT29/HS746T/SNU-5 cells were seeded into a 96-well plate with black wall and transparent bottom at 100 µL of cell suspension per well;
2. 10 µL of serially diluted antibody to be tested was added to each well, and the cells were cultured for 6 days;
3. 100 µL of CellTiter-Glo 2.0 Cell Viability Assay was added to each well, and the plate was shaken for mixing for 10 min. Then the fluorescence intensity was detected.

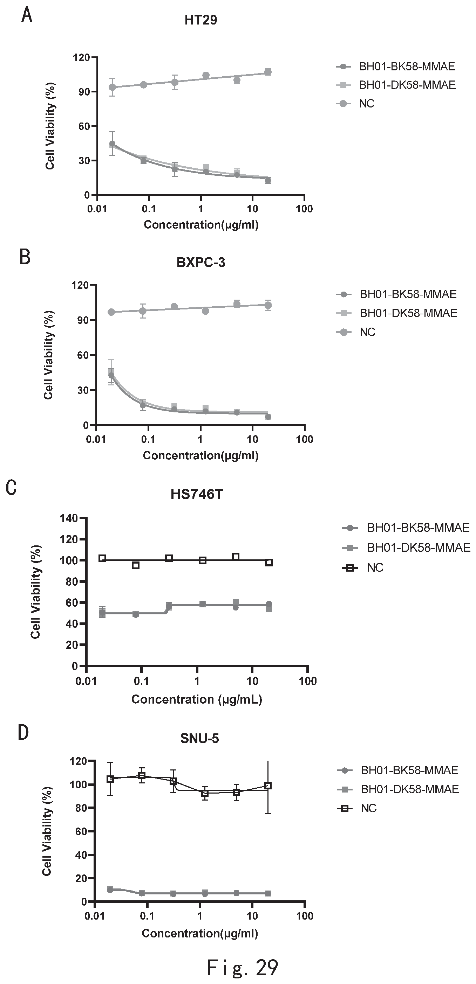

As shown in Figure 29, both BH01-BK58-MMAE and BH01-DK58-MMAE can effectively kill 4 types of tumor cells BxPC-3/HT29/HS746T/SNU-5, and the killing effects are similar.

### Example 35. The anti-tumor efficacy of BH01-DK58-MMAE and BH01-BK58-MMAE against HCT116 xenograft tumors is superior

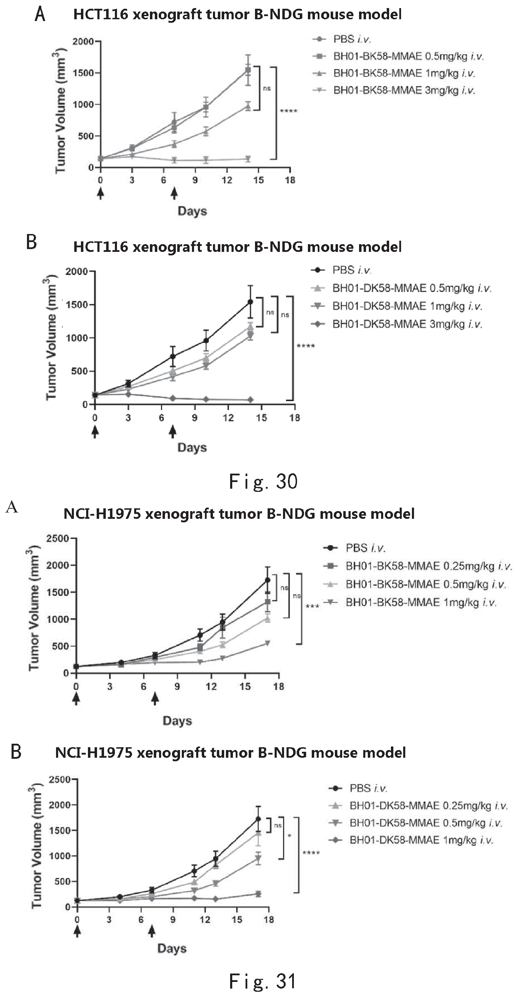

Experimental mice: 36 female B-NDG mice, 7-week-old at the time of the experiment;
Experimental cells: human colon cancer cells HCT116 in the logarithmic growth phase were collected, the culture medium was removed, and the cells were washed twice with PBS before inoculation;
Inoculation volume: 5×10⁶ cells/100 µL/mouse;
Inoculation site: right back area;
Grouping and administration: when the average tumor volume grew to about 100-150 mm³, the tumor-bearing mice were randomly grouped, and drugs were administered on the day of grouping, which was defined as Day 0. The drugs were administered through intravenous injection once a week for a total of two times. During the experiment, tumor volume and body weight were measured twice a week, and the relationship between the changes in body weight and tumor volume of tumor-bearing mice and the administration time was recorded. Tumor volume (mm³) was calculated as (length × width²)/2. Tumor growth inhibition rate (TGI%) = (1 - change in tumor volume in the drug-treated group / change in tumor volume in the control group) × 100%.
Administration doses: 0.5 mg/kg, 1 mg/kg, 3 mg/kg;
As shown in Fig. 30 and Table 23, BH01-BK58-MMAE and BH01-DK58-MMAE can significantly inhibit the growth of human colon cancer cells HCT116 in a dose-dependent manner. At the administration dose of 3 mg/kg, the tumor inhibition rates of BH01-BK58-MMAE and BH01-DK58-MMAE on Day 14 were 91.34% and 95.52%, respectively. In addition, BH01-DK58-MMAE has a relatively low effective dose and shows tumor-inhibiting effects even at a dose of 0.5 mg/kg (with TGI% of 24.11%), indicating that BH01-DK58-MMAE has a large therapeutic window.

**Table 23 Tumor inhibition rates of BH01-BK58-MMAE and BH01-DK58-MMAE against HCT116 xenograft tumors on Day 14**

| Group | Treatment group | Dose (mg/kg) | Average tumor volume (mm³) | Tumor growth inhibition rate (TGI%) |
|---|---|---|---|---|
| G1 | PBS | - | 1544.76 | - |
| G2 | BH01-BK58-MMAE | 0.5 | 1550.60 | -0.38% |
| G3 | BH01-BK58-MMAE | 1 | 976.28 | 36.80% |
| G4 | BH01-BK58-MMAE | 3 | 133.83 | 91.34% |
| G5 | BH01-DK58-MMAE | 0.5 | 1172.34 | 24.11% |
| G6 | BH01-DK58-MMAE | 1 | 1029.39 | 33.36% |
| G7 | BH01-DK58-MMAE | 3 | 69.16 | 95.52% |

### Example 36. The anti-tumor efficacy of BH01-DK58-MMAE and BH01-BK58-MMAE against NCI-H1975 xenograft tumors

Experimental mice: 35 female B-NDG mice, 7-week-old at the time of the experiment
Experimental cells: human lung cancer cells NCI-H1975 in the logarithmic growth phase were collected, the culture medium was removed, and the cells were washed twice with PBS before inoculation;
Inoculation volume: 5×10⁶ cells/100 µL/mouse;
Inoculation site: right back area;
Grouping and administration: when the average tumor volume grew to about 100-150 mm³, the tumor-bearing mice were randomly grouped, and drugs were administered on the day of grouping, which was defined as Day 0. The drugs were administered through intravenous injection once a week for a total of two times. During the experiment, tumor volume and body weight were measured twice a week, and the relationship between the changes in body weight and tumor volume of tumor-bearing mice and the administration time was recorded. Tumor volume (mm³) was calculated as (length × width²)/2. Tumor growth inhibition rate (TGI%) = (1 - change in tumor volume in the drug-treated group / change in tumor volume in the control group) × 100%.
Administration doses: 0.25 mg/kg, 0.5 mg/kg, 1 mg/kg.
As shown in Fig. 31 and Table 24, BH01-BK58-MMAE and BH01-DK58-MMAE can significantly inhibit the growth of human lung cancer cells NCI-H1975 in a dose-dependent manner. On Day 17, both BH01-DK58-MMAE and BH01-BK58-MMAE at a dose of 1 mg/kg showed significant tumor-inhibiting effects (with TGI% of 72.98% and 61.47%, respectively). In addition, BH01-DK58-MMAE has a relatively low effective dose and shows tumor-inhibiting effects even at a dose of 0.25mg/kg ( with TGI% of 15.24%). At a dose of 0.5 mg/kg, it had shown significant tumor inhibition compared with that of the control group on Day 17.

**Table 24 Tumor inhibition rates of BH01-BK58-MMAE and BH01-DK58-MMAE against NCI-H1975 xenograft tumors on Day 20**

| Group | Treatment group | Dose (mg/kg) | Average tumor volume (mm³) | Tumor growth inhibition rate (TGI%) |
|---|---|---|---|---|
| G1 | PBS | - | 2055.02 | - |
| G2 | BH01-BK58-MMAE | 0.25 | 2071.98 | -0.83% |
| G3 | BH01-BK58-MMAE | 0.5 | 1463.04 | 28.81% |
| G4 | BH01-BK58-MMAE | 1 | 791.73 | 61.47% |
| G5 | BH01-DK58-MMAE | 0.25 | 1741.75 | 15.24% |
| G6 | BH01-DK58-MMAE | 0.5 | 1417.17 | 31.04% |
| G7 | BH01-DK58-MMAE | 1 | 555.19 | 72.98% |

### Example 37. C57BL/6 mouse model has good tolerance to BH01-BK58-MMAE and BH01-DK58-MMAE

Experimental mice: 48 female and 48 male C57BL/6 mice, 6-week-old.

Grouping and administration: mice were randomly grouped according to their body weights, and drugs were administered on the day of grouping, which was defined as Day 0. The drugs were administered through intravenous injection every two weeks for a total of two times. During the experiment, the body weights were measured twice a week, and the relationship between the changes in the mice's body weights and the administration time was recorded.

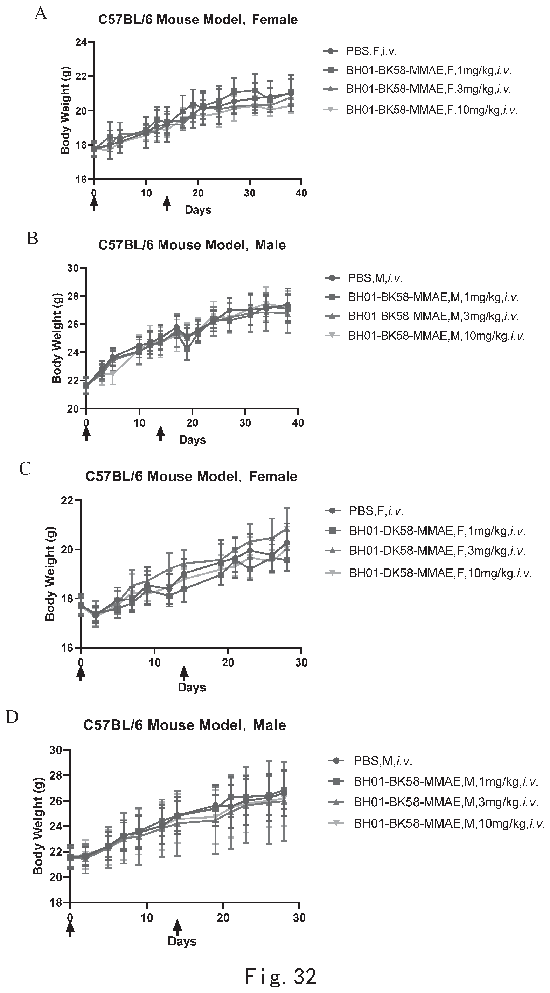

As shown in Fig. 32 and Tables 25 and 26, the experimental results indicate that the average body weights of animals in different dose groups and of different genders all increased, and there was no obvious weight loss caused by drug toxicity. This indicates that C57BL/6 mice have good tolerance to BH01-BK58-MMAE and BH01-DK58-MMAE at a dose of 10 mg/kg or less.

**Table 25 Changes in the body weights of C57BL/6 mice injected intravenously with BH01-BK58-MMAE on Day 27**

| Group | Treatment group | Gender | Dose (mg/kg) | Average body weight on Day 27 (g) | Average body weight on Day 0 (g) | Rate of weight change |
|---|---|---|---|---|---|---|
| G1 | PBS | Female | - | 20.53 | 17.77 | 15.53% |
| G2 | | Male | | 26.98 | 21.63 | 24.73% |
| G3 | BH01-BK5 8-MMAE | Female | 1 | 21.07 | 17.75 | 18.70% |
| G4 | | Male | | 26.27 | 21.65 | 21.34% |
| G5 | BH01-BK5 8-MMAE | Female | 3 | 20.23 | 17.77 | 13.84% |
| G6 | | Male | | 26.48 | 21.63 | 22.42% |
| G7 | BH01-BK5 | Femal | 10 | 20.13 | 17.77 | 13.28% |
| | 8-MMAE | e | | | | |
| G8 | | Male | | 26.47 | 21.63 | 22.38% |

**Table 26 Changes in the body weights of C57BL/6 mice injected intravenously with BH01-DK58-MMAE on Day 28**

| Group | Treatment group | Gender | Dose (mg/kg) | Average body weight on Day 28 (g) | Average body weight on Day 0 (g) | Rate of weight change |
|---|---|---|---|---|---|---|
| G1 | PBS | Female | - | 20.26 | 17.73 | 14.27% |
| G2 | | Male | | 26.61 | 21.58 | 23.31% |
| G3 | BH01-DK5 8-MMAE | Female | 1 | 19.56 | 17.72 | 10.38% |
| G4 | | Male | | 26.85 | 21.56 | 24.54% |
| G5 | BH01-DK5 8-MMAE | Female | 3 | 20.85 | 17.72 | 17.66% |
| G6 | | Male | | 25.96 | 21.55 | 20.46% |
| G7 | BH01-DK5 8-MMAE | Female | 10 | 20.03 | 17.71 | 13.10% |
| G8 | | Male | | 26.19 | 21.57 | 21.42% |

### Example 38. The toxicity of high-dose BH01-DK58-MMAE to female and male C57BL/6 mice is lower than that of BH01-BK58-MMAE at the same dose

Experimental mice: 6-week-old C57BL/6 mice, half male and half female (48 female and 48 male).

Grouping and administration: mice were randomly grouped according to their body weights, and drugs were administered on the day of grouping, which was defined as Day 0. The drugs were administered through a single intravenous injection. During the experiment, the body weights were measured twice a week, and the relationship between the changes in the mice's body weights and the administration time was recorded.

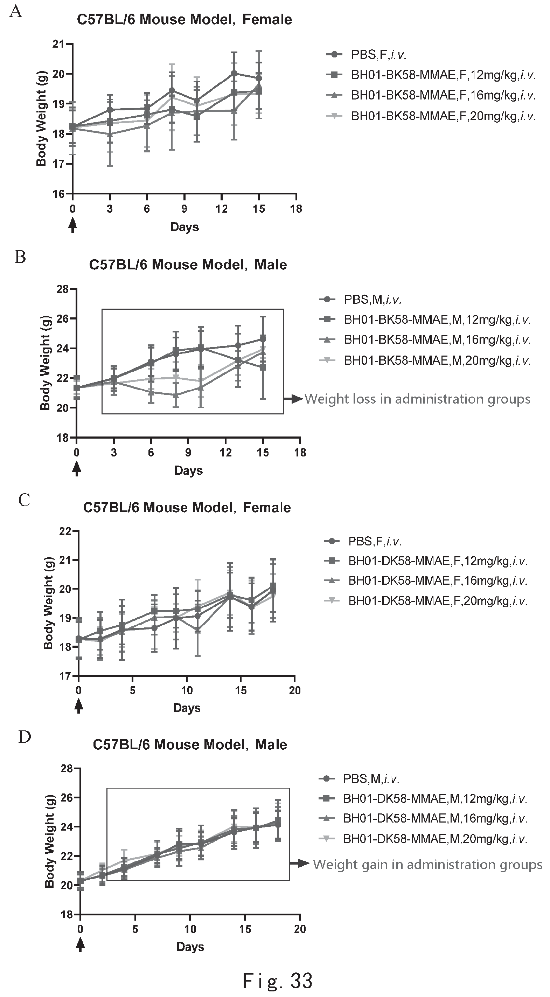

As shown in Fig. 33 and Tables 27 and 28, after intravenous injection of 12-20 mg/kg BH01-BK58-MMAE, the body weight change range of female mice on Day 8 was 2.86%-6.64%, with no significant difference, while the body weight change range of male mice was -2.30%-11.86%, wherein the body weight increase at doses of 16 mg/kg and 20 mg/kg was significantly lower than that at a dose of 12 mg/kg and of the PBS group, indicating that BH01-BK58-MMAE has certain toxicity to male mice. After intravenous injection of 2-20 mg/kg BH01-DK58-MMAE, the body weight change range of female mice on Day 7 was 1.97%-5.37%, and that of male mice was 7.84%-9.22%, with no significant difference, indicating that mice have better tolerance to BH01-DK58-MMAE.

The above results suggest that the strategy of controlling the affinity of the EGFR-binding domain and truncating the hinge region to reduce EGFR targeting is beneficial for safety, significantly reducing the toxicity to normal tissue cells and resulting in lower side-effects of the prepared bispecific antibodies and ADCs.

**Table 27 Changes in the body weights of C57BL/6 mice injected intravenously with BH01-BK58-MMAE on Day 15**

| Group | Treatment group | Gender | Dose (mg/ kg) | Average body weight on Day 15 (g) | Average body weight on Day 8 (g) | Average body weight on Day 0 (g) | Rate of weight change | |
|---|---|---|---|---|---|---|---|---|
| | | | | | | | D15 Vs D0 | D8 Vs D0 |
| G1 | PBS | Female | - | 19.86 | 19.44 | 18.23 | 8.94% | 6.64% |
| G2 | | Male | | 24.62 | 23.61 | 21.36 | 15.26% | 10.53% |
| G3 | BH01-BK5 8-MMAE | Female | 12 | 19.43 | 18.81 | 18.24 | 6.52% | 3.13% |
| G4 | | Male | | 22.73 | 23.86 | 21.33 | 6.56% | 11.86% |
| G5 | BH01-BK5 8-MMAE | Female | 16 | 19.64 | 18.70 | 18.18 | 8.03% | 2.86% |
| G6 | | Male | | 23.75 | 20.85 | 21.34 | 11.29% | -2.30% |
| G7 | BH01-BK5 8-MMAE | Female | 20 | 19.34 | 19.22 | 18.21 | 6.21% | 5.55% |
| G8 | | Male | | 23.95 | 22.02 | 21.36 | 12.13% | 3.09% |

**Table 28 Changes in the body weights of C57BL/6 mice injected intravenously with BH01-DK58-MMAE on Day 14**

| Group | Treatment group | Gender | Dose (mg/ kg) | Average body weight on Day 14 (g) | Average body weight on Day 7 (g) | Average body weight on Day 0 (g) | Rate of weight change | |
|---|---|---|---|---|---|---|---|---|
| | | | | | | | D14 Vs D0 | D7 Vs D0 |
| G1 | PBS | Female | - | 19.73 | 18.65 | 18.29 | 7.87% | 1.97% |
| G2 | | Male | | 23.63 | 22.04 | 20.29 | 16.46% | 8.62% |
| G3 | BH01-DK5 8-MMAE | Female | 12 | 19.79 | 19.23 | 18.25 | 8.44% | 5.37% |
| G4 | | Male | | 23.83 | 22.16 | 20.29 | 17.45% | 9.22% |
| G5 | BH01-DK5 8-MMAE | Female | 16 | 19.73 | 19.01 | 18.27 | 8.00% | 4.05% |
| G6 | | Male | | 23.80 | 21.88 | 20.29 | 17.30% | 7.84% |
| G7 | BH01-DK5 8-MMAE | Female | 20 | 19.87 | 18.65 | 18.28 | 8.70% | 2.02% |
| G8 | | Male | | 24.03 | 22.16 | 20.32 | 18.26% | 9.06% |

### Example 39. The internalization of BH01-DK58 antibody is stronger than that of single-target antibodies and the control antibodies

Method:
1. The target cells were digested with Accutase and collected, and were resuspended in the experimental buffer (the corresponding complete medium).
2. The density of the target cells was adjusted to 1E5 cells/mL with the experimental buffer, and the cell suspension was transferred to corresponding wells (50 µL/well, 5000 cells/well) of a 96-well experimental plate (Corning, Cat. #: 3595).
3.The experimental plate was placed in a cell culture incubator (37°C/5% CO₂) overnight for culture to allow the target cells to adhere to the plate.
4. The test sample/control sample working solutions (4×) and the labeling reagent (Incucyte^{®} Human Fabfluor-pH Red Antibody Labeling Reagent, Sartorius, Cat. #: 4722) working solution were prepared with experimental buffer.
5. The test sample/control sample working solutions and the labeling reagent working solution were mixed at a molar ratio of 1:3 and a volume ratio of 1:1, and were incubated in a cell culture incubator (37°C/5% CO2) for 15 minutes to ensure full conjugation between them.
6. According to the experimental protocol, the conjugated mixture working solution was transferred to corresponding wells of the 96-well experimental plate (50 µL/well).
7. The experimental plate was incubated in an Incucyte^{®} S3 live-cell analyzer imaging system (37°C/5% CO₂). The Incucyte software was used to set up the imaging program (imaging channels: Phase and Red; objective lens: 10×; imaging time interval: 2 hours; imaging end time: 48 hours). Pictures were taken by the data processing system Controller at appropriate detection wavelengths and specified time points.
8. Data analysis of the antibody internalization experiment (Incucyte method): the original experimental data and results were analyzed and exported by the Incucyte^{®} Live-Cell Analysis System, the data processing system of the live-cell analyzer. The analysis result parameter was expressed as: Total Red Object Area (µm²/Image). The exported original data could be further analyzed using Microsoft Office Excel 2016 and GraphPad Prism 6 software.

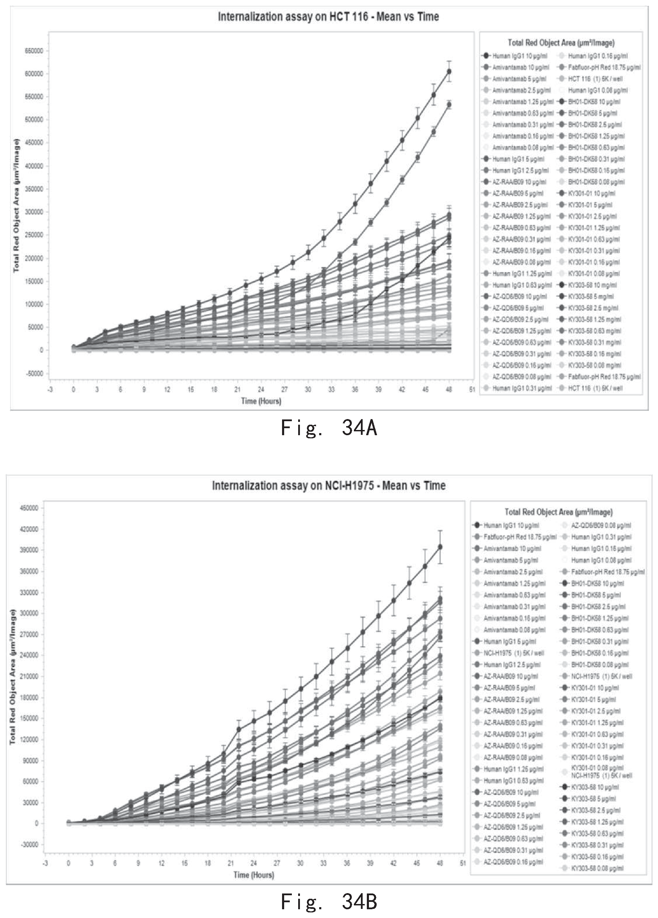

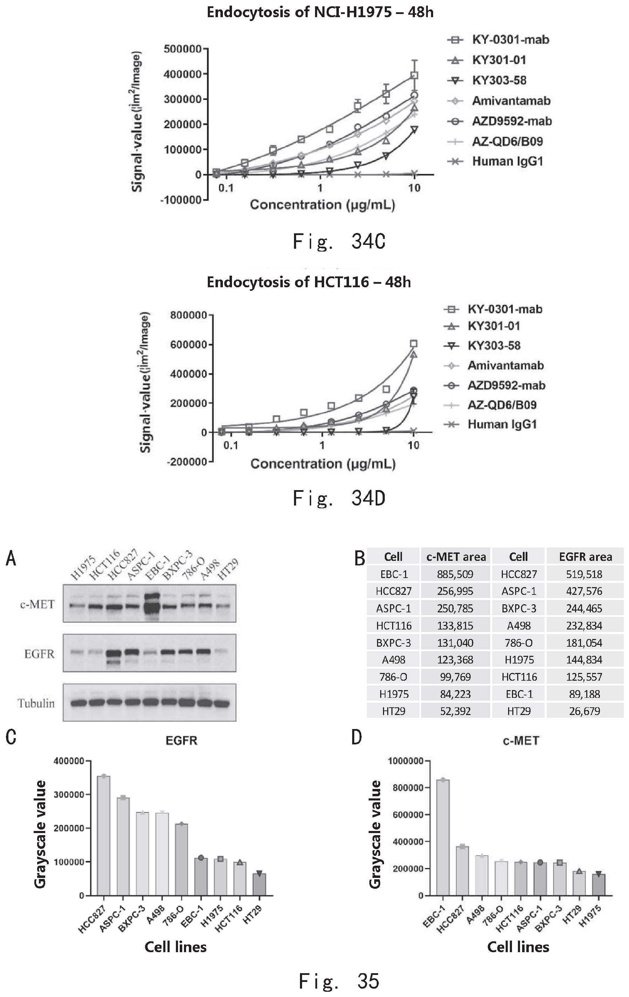

As shown in Fig. 34, in both HCT116 and NCI-H1975 cells, the internalization of BH01-DK58 was stronger than that of the single-target antibodies KY301-01 and KY303-58, and the control antibodies Amivantamab, AZ-RAA/B09, and AZ-QD6/B09.

### Example 40. Protein levels of MET and EGFR in tumor cells

Method:
1. Preparation of protein samples: cells were taken out and placed on ice, centrifuged at 300×g for 5 min at 4°C, washed twice with pre-cooled PBS and centrifuged to remove the supernatant. RIPA lysis buffer was added to the cell pellet, and the mixture was thoroughly shaken and lysed. Protein quantification was performed using the BCA method.
2. Electrophoresis: A 4-12% pre-cast gel was used. 5 µL of protein marker and 18 µL of protein sample were added in sequence. The power plug was inserted, and the voltage was adjusted to 80-90 V. After 15-30 min, when the bromophenol blue entered the lower gel, the voltage was adjusted to 120-130 V. The electrophoresis was terminated when the bromophenol blue just ran out of the gel.
3. Membrane transfer: A piece of PVDF membrane was cut with scissors to the same size and shape as the gel. The cut PVDF membrane was soaked in methanol for 15 seconds for activation before use. The activated PVDF membrane was completely covered on the gel block. The glass plate was removed, the gel was appropriately cut and buckled on the membrane. There should be no air bubbles between the membrane and the gel block. Then, filter paper and sponge soaked in the membrane transfer solution were covered on the membrane. The whole was placed in the membrane transfer device and then in the membrane transfer tank. The membrane side was placed on the positive electrode, and the gel side was placed on the negative electrode. An ice box was placed in the tank and filled with membrane transfer buffer. The voltage was adjusted, and the appropriate membrane transfer time was set according to the molecular weight of the target protein for membrane transfer.
4. Blocking: the PVDF membrane was washed 2-3 times with 0.1% TBST for 5 min each time. The prepared 5% skim milk was added, and the membrane was blocked on a shaker at room temperature for 60 min;
5. Washing: the blocked PVDF membrane was rinsed 3 times with 0.1% TBST for 5 min each time;
6. Incubation with the primary antibody: the membrane was blocked overnight on a shaker at 4°C;
7. Washing: the blocked PVDF membrane was rinsed 3 times with 0.1% TBST for 5 min each time;
8. Incubation with the secondary antibody: the HRP-labeled secondary antibody of the corresponding species was diluted with 0.1% TBST and incubated on a shaker at room temperature for 60 min;
9. Washing: the NC membrane was rinsed 3 times with 0.1% TBST for 10 min each time;
10. Color development: Solution A and Solution B in the ECL luminescent agent were mixed in a 1:1 ratio and dropped onto the PVDF membrane. Exposure, development, fixation, and film scanning were performed in a darkroom.

As shown in Fig. 35, MET and EGFR proteins are expressed in 9 types of cells, NCI-H1975, HCT116, HCC827, ASPC-1, EBC-1, BxPC-3, 786-O, A498, and HT29.

### Example 41. KY-0301 (BH01-DK58-MMAE) down-regulates the protein levels of MET and EGFR

Method:
1. Preparation of protein samples: cells were taken out and placed on ice, centrifuged at 300×g for 5 min at 4°C, washed twice with pre-cooled PBS and centrifuged to remove the supernatant. RIPA lysis buffer was added to the cell pellet, and the mixture was thoroughly shaken and lysed. Protein quantification was performed using the BCA method.
2. Electrophoresis: A 4-12% pre-cast gel was used. 5 µL of protein marker and 18 µL of protein sample were added in sequence. The power plug was inserted, and the voltage was adjusted to 80-90 V. After 15-30 min, when the bromophenol blue entered the lower gel, the voltage was adjusted to 120-130 V. The electrophoresis was terminated when the bromophenol blue just ran out of the gel.
3. Membrane transfer: A piece of PVDF membrane was cut with scissors to the same size and shape as the gel. The cut PVDF membrane was soaked in methanol for 15 seconds for activation before use. The activated PVDF membrane was completely covered on the gel block. The glass plate was removed, the gel was appropriately cut and buckled on the membrane. There should be no air bubbles between the membrane and the gel block. Then, filter paper and sponge soaked in the membrane transfer solution were covered on the membrane. The whole was placed in the membrane transfer device and then in the membrane transfer tank. The membrane side was placed on the positive electrode, and the gel side was placed on the negative electrode. An ice box was placed in the tank and filled with membrane transfer buffer. The voltage was adjusted, and the appropriate membrane transfer time was set according to the molecular weight of the target protein for membrane transfer.
4. Blocking: the PVDF membrane was washed 2-3 times with 0.1% TBST for 5 min each time. The prepared 5% skim milk was added, and the membrane was blocked on a shaker at room temperature for 60 min;
5. Washing: the blocked PVDF membrane was rinsed 3 times with 0.1% TBST for 5 min each time;
6. Incubation with the primary antibody: the membrane was blocked overnight on a shaker at 4°C;
7. Washing: the blocked PVDF membrane was rinsed 3 times with 0.1% TBST for 5 min each time;
8. Incubation with the secondary antibody: the HRP-labeled secondary antibody of the corresponding species was diluted with 0.1% TBST and incubated on a shaker at room temperature for 60 min;
9. Washing: the NC membrane was rinsed 3 times with 0.1% TBST for 10 min each time;
10. Color development: Solution A and Solution B in the ECL luminescent agent were mixed in a 1:1 ratio and dropped onto the PVDF membrane. Exposure, development, fixation, and film scanning were performed in a darkroom.

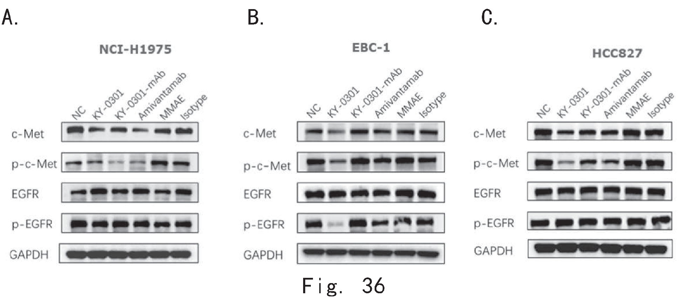

As shown in Fig. 36, after treating NCI-H1975, EBC-1, and HCC827 cells with 1 mg/mL KY-0301 for 24 hours, the protein levels of MET and EGFR were significantly reduced, and the levels of phosphorylated MET and phosphorylated EGFR also decreased. This indicates that KY-0301 can promote the internalization of MET and EGFR and inhibit the activation of MET and EGFR.

### Example 42. Killing ability of KY-0301 (BH01-DK58-MMAE) against tumor cells is superior to that of single-target-MMAEs

Method:
1. 786-O/ASPC-1/A498/BxPC-3/EBC-1/HCC827/NCI-H1975/HCT116/HT2 9 cells were seeded into a 96-well plate with black wall and transparent bottom at 100 µL of cell suspension per well;
2. 10 µL of serially diluted ADC to be tested was added to each well, and the cells were cultured for 6 days;
3. 100 µL of CellTiter-Glo 2.0 Cell Viability Assay was added to each well, and the plate was shaken for mixing for 10 min. Then the fluorescence intensity was detected

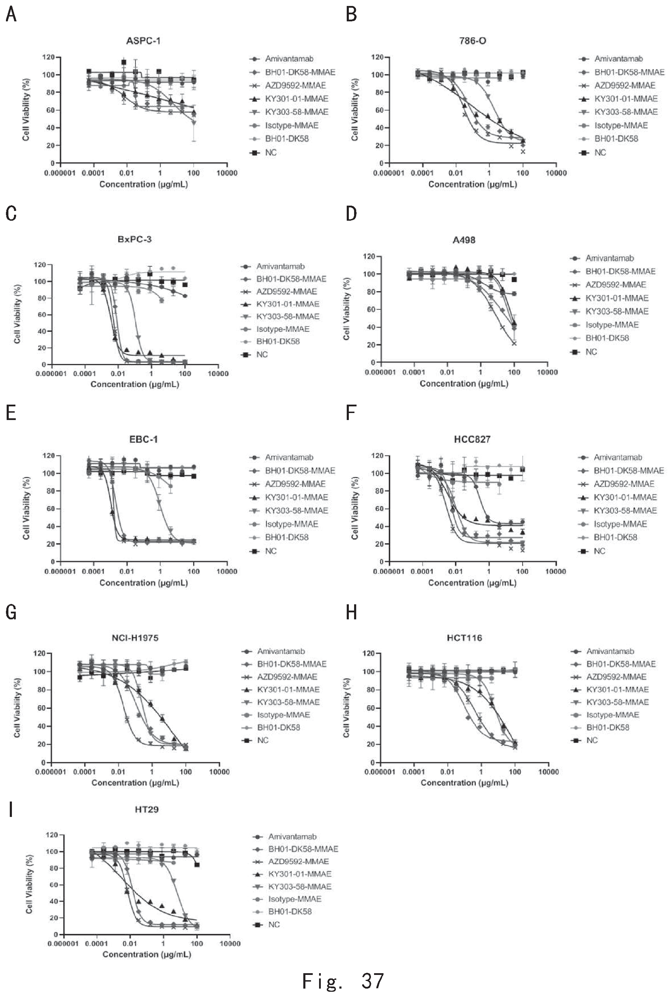

As shown in Figure 37, BH01-DK58-MMAE, the control antibody AZD9592-MMAE, KY301-01-MMAE, and KY303-58-MMAE can all effectively kill tumor cells with medium, high, and low expression levels of MET and EGFR (such as 786-O/ASPC-1/A498/BxPC-3/EBC-1/HCC827/NCI-H1975/HCT116/HT29). Among them, the killing effect of BH01-DK58-MMAE was stronger than that of the single-target ADCs (KY301-01-MMAE and KY303-58-MMAE), and is similar to that of AZD9592-MMAE (the IC50 values are shown in Table 29).

**Table 29 IC50 values of BH01-DK58-MMAE and AZD9592-MMAE against different tumor cells**

| IC50 (µg/mL) | BH01-DK58-MMAE | AZD9592-MMAE |
|---|---|---|
| 786-O | 0.0579 | 0.0271 |
| A498 | 30.9200 | 7.9070 |
| ASPC-1 | ~ 0.03064 | 0.0065 |
| BXPC-3 | 0.0082 | 0.0051 |
| EBC-1 | 0.0021 | ~ 0.001583 |
| HCC827 | 0.0050 | 0.0026 |
| NCI-H1975 | 0.1119 | 0.0196 |
| HCT116 | 0.0992 | 0.4183 |
| HT29 | 0.0133 | 0.0068 |

### Example 43. The toxicity of KY-0301 (BH01-DK58-MMAE) to normal cells is weaker than that of the control antibody AZD9592-MMAE

Method:
1. HFL-1/MIHA/MRC-5/BEAS-2B cells were seeded into a 96-well plate with black wall and transparent bottom at 100 µL of cell suspension per well;
2. 10 µL of serially diluted ADC to be tested was added to each well, and the cells were cultured for 6 days;
3. 100 µL of CellTiter-Glo 2.0 Cell Viability Assay was added to each well, and the plate was shaken for mixing for 10 min. Then the fluorescence intensity was detected

As shown in Fig. 37, BH01-DK58-MMAE exhibited toxicity to normal cells such as HFL-1, MRC-5, and BEAS-2B at a high concentration (20 µg/mL), and showed almost no toxicity to MIHA cells. In contrast, the control antibody AZD9592-MMAE killed FL-1/MRC-5/BEAS-2B normal cells at a relatively low dose of 0.8 µg/mL, and killed MIHA cells at an extremely high dose of 100 µg/mL. The results indicate that the toxicity of BH01-DK58-MMAE to normal cells is significantly weaker than that of the control antibody AZD9592-MMAE, suggesting that the safety of BH01-DK58-MMAE is better than that of AZD9592-MMAE.

### Example 44. KY-0301 (BH01-DK58-MMAE) causes cell cycle arrest in tumor cells

Method:
1. The HCT116 cells treated with BH01-DK58-MMAE for 48 hours were digested to prepare a cell suspension;
2. 50 µL of the cell suspension was taken, and 1 mL of pre-cooled PBS was added to resuspend the cells. After resuspension and centrifugation, the supernatant was removed while 50 µL of the supernatant was retained. The bottom of the tube was flicked gently to disperse the cells;
3. 1 mL of pre-cooled 70% ethanol was added, mixed gently, and the cells were fixed at 4 °C for more than 30 min. Then, centrifugation was carried out to retain the precipitate;
4. 1 mL of pre-cooled PBS solution was added to resuspend the cells, and centrifugation was carried out;
5. 0.5 mL of propidium iodide staining solution was added. The cells were slowly and thoroughly resuspended, incubated at 37 °C for 30 min, and stored on ice;
6. Detection was carried out using a flow cytometer;

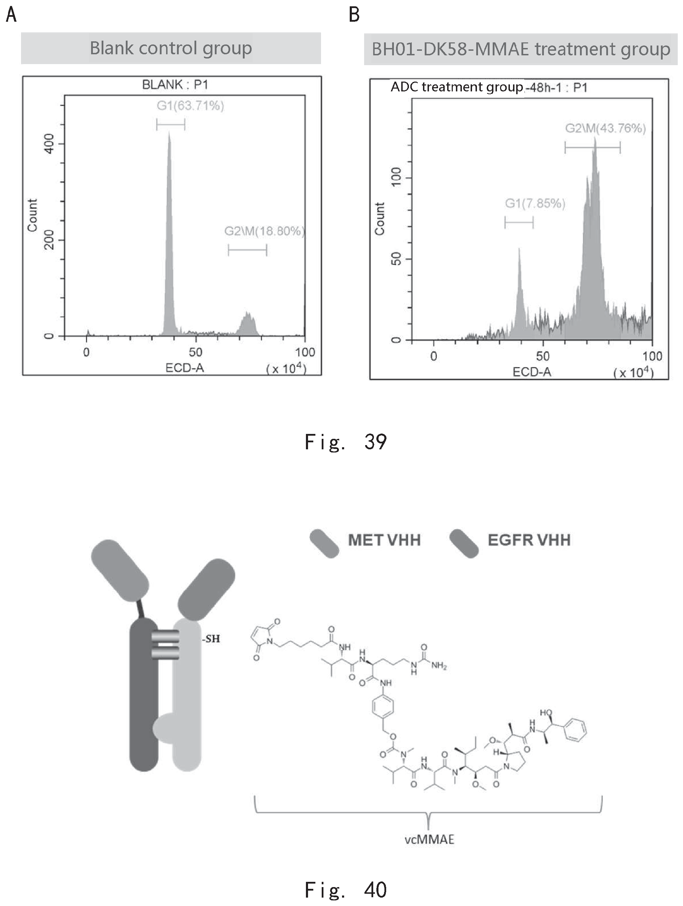

As shown in Fig. 39, after treatment with BH01-DK58-MMAE for 48 h, the proportion of G2/M phase cells in HCT116 cells was 43.76%, which was much higher than 18.8% in the blank control group, indicating that BH01-DK58-MMAE can induce G2/M phase arrest in HCT116 cells.

### Example 45. KY-0301 has significant anti-tumor efficacy against EBC-1 xenograft tumors

Experimental mice: female Balb/C Nude mice
Experimental cells: human lung cancer cells EBC-1 in the logarithmic growth phase were collected, the culture medium was removed, and the cells were washed twice with PBS before inoculation;
Inoculation volume: 5×10⁶ cells/100 µL/mouse;
Inoculation site: right back area;
Grouping and administration: when the average tumor volume grew to about 100-150 mm³, the tumor-bearing mice were randomly grouped, and drugs were administered on the day of grouping, which was defined as Day 0. The drugs were administered through intravenous injection once a week for a total of two times. During the experiment, tumor volume and body weight were measured twice a week, and the relationship between the changes in body weight and tumor volume of tumor-bearing mice and the administration time was recorded. Tumor volume (mm³) was calculated as (length × width²)/2. Tumor growth inhibition rate (TGI%) = (1 - change in tumor volume in the drug-treated group / change in tumor volume in the control group) × 100%.
Administration doses: 1 mg/kg, 2 mg/kg, 4 mg/kg.

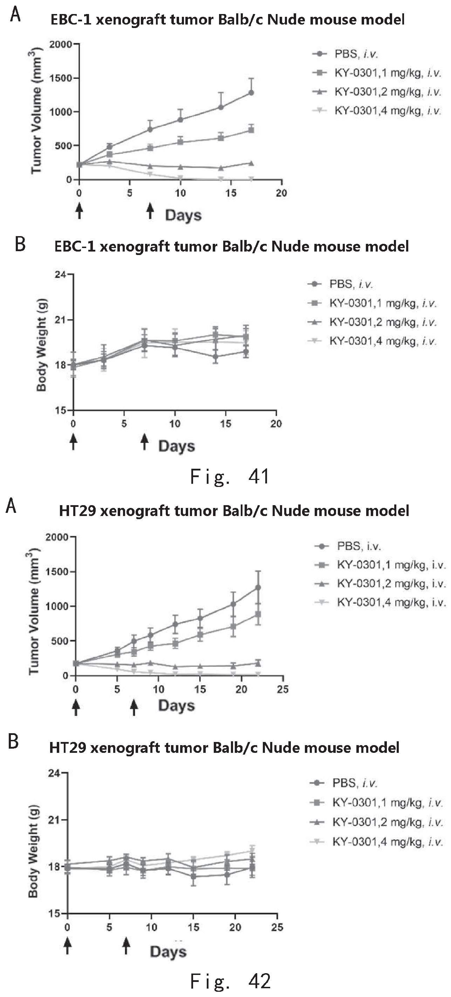

The structural schematic diagram of KY-0301, namely the BH01-DK58-MMAE mentioned above, is shown in Fig. 40. As shown in Fig. 41, KY-0301 can significantly inhibit the growth of human lung cancer cells EBC-1 in a dose-dependent manner. On Day 17, the tumor inhibition rate of 4 mg/kg KY-0301 was 100%.

### Example 46. KY-0301 has significant anti-tumor efficacy against HT29 xenograft tumors

Experimental mice: female Balb/C Nude mice
Experimental cells: human colorectal cancer cells HT29 in the logarithmic growth phase were collected, the culture medium was removed, and the cells were washed twice with PBS before inoculation;
Inoculation volume: 5×10⁶ cells/100 µL/mouse;
Inoculation site: right back area;
Grouping and administration: when the average tumor volume grew to about 100-150 mm³, the tumor-bearing mice were randomly grouped, and drugs were administered on the day of grouping, which was defined as Day 0. The drugs were administered through intravenous injection once a week for a total of two times. During the experiment, tumor volume and body weight were measured twice a week, and the relationship between the changes in body weight and tumor volume of tumor-bearing mice and the administration time was recorded. Tumor volume (mm³) was calculated as (length × width²)/2. Tumor growth inhibition rate (TGI%) = (1 - change in tumor volume in the drug-treated group / change in tumor volume in the control group) × 100%.

Administration doses: 1 mg/kg, 2 mg/kg, 4 mg/kg.

As shown in Fig. 42, KY-0301 can significantly inhibit the growth of human colorectal cancer cells HT29 in a dose-dependent manner. On Day 22, the tumor inhibition rate of 4 mg/kg KY-0301 was 98%.

### Example 47. KY-0301 has significant anti-tumor efficacy in high-tumor-burden models and relapse models of NCI-H1975 xenograft tumor

Experimental mice: female Balb/C Nude mice
Experimental cells: human lung cancer cells NCI-H1975 in the logarithmic growth phase were collected, the culture medium was removed, and the cells were washed twice with PBS before inoculation;
Inoculation volume: 5×10⁶ cells/100 µL/mouse;
Inoculation site: right back area;
Administration for high-tumor-burden model group: when the average tumor volume in the PBS group grew to about 3000 mm³, the tumor-bearing mice were randomly grouped. Drugs were administered twice on D26 and D33 respectively, with a dosage of 5 mg/kg. During the experiment, tumor volume and body weight were measured twice a week, and the relationship between the changes in body weight and tumor volume of tumor-bearing mice and the administration time was recorded. Tumor volume (mm³) was calculated as (length × width²)/2. Tumor growth inhibition rate (TGI%) = (1 - change in tumor volume in the drug-treated group / change in tumor volume in the control group) × 100%.

Administration for relapse model group: when the average tumor volume grew to about 100-150 mm³, the tumor-bearing mice were randomly grouped, and drugs were administered on the day of grouping, which was defined as Day 0. The drugs were administered through intravenous injection once a week for a total of two times, with a dosage of 1.5 mg/kg. By D30, the tumors resumed growth. By D47, the tumor volume grew to about 1600 mm³. Drugs were administered again at a dosage of 1.5 mg/kg on D47 and D54 to test the killing effect of KY-0301 on the regrown tumors. During the experiment, tumor volume and body weight were measured twice a week, and the relationship between the changes in body weight and tumor volume of tumor-bearing mice and the administration time was recorded. Tumor volume (mm³) was calculated as (length × width²)/2. Tumor growth inhibition rate (TGI%) = (1 - change in tumor volume in the drug-treated group / change in tumor volume in the control group) × 100%.

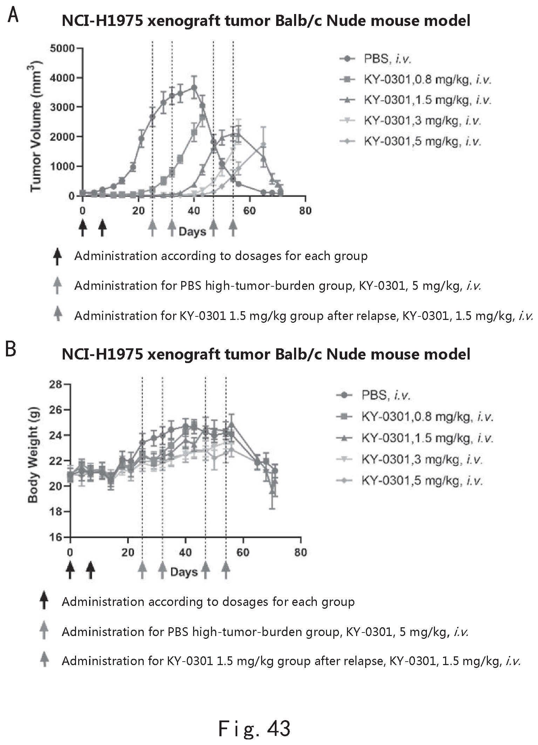

As shown in Fig. 43A, KY-0301 can significantly inhibit the growth of human lung cancer NCI-H1975 in high-tumor-burden model, and the tumor volume decreased from about 3500 mm³ to complete clearance.

As shown in Fig. 43B, KY-0301 can significantly inhibit the growth of human lung cancer NCI-H1975 in relapse model, and the tumor volume decreased from about 2000 mm³ to complete clearance.

### Example 48. KY-0301 effectively inhibits tumor growth in a lung cancer PDX mouse model

1. 45 female B-NDG mice (8-10 weeks old) were prepared;
2. Non-small cell lung cancer tissues were cut into pieces of 2×2×2 mm and inoculated subcutaneously in the right anterior flank of B-NDG mice;
3. When the tumors grew to 200-300 mm³, the B-NDG mice were grouped into 3 groups with 6 mice in each group. The grouping and administration method are shown in the following table (Table 30):

**Table 30 Animal grouping and administration**

| **Group** | **Experimental Group** | **Number of Animals** | **Dosage** | **Administration Route** | **Administration Frequency** |
|---|---|---|---|---|---|
| 1 | PBS | 6 | NA | i.v. | QW×2 |
| 2 | KY-0301 | 6 | 1.5 mg/kg | i.v. | QW×2 |
| 3 | KY-0301 | 6 | 3 mg/kg | i.v. | QW×2 |

4. The mice were administered with PBS, KY-0301 at a dose of 1.5 mg/kg, and KY-0301 at a dose of 3 mg/kg via tail vein injection once a week for two consecutive weeks;
5. Tumor volume and body weight were measured twice a week, and the relationship between the changes in body weight and tumor volume of tumor-bearing mice and the administration time was recorded;
6. The tumor volume and tumor growth inhibition rate were calculated $Tumor volume \left(V\right) was calculated as \left(length\times {width}^{2}\right) / 2$ Tumor growth inhibition rate (TGI%) was calculated using the following formula: 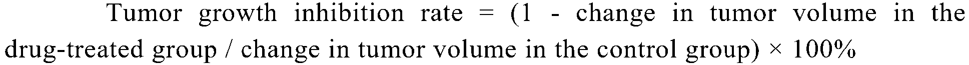 Tumor growth inhibition rate = (1 - change in tumor volume in the drug-treated group / change in tumor volume in the control group) × 100%

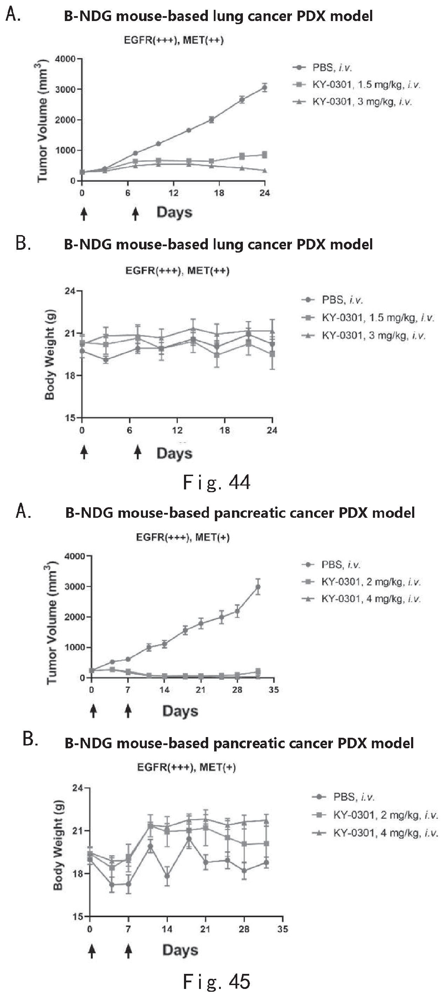

The experimental results (Fig. 44) indicate that in the non-small cell lung cancer PDX B-NDG mouse model, both 1.5 mg/kg and 3 mg/kg of KY-0301 could effectively inhibit the growth and proliferation of tumor tissues in mice. The tumor inhibition rates on Day 24 were 79.5% and 97.9% respectively, which is dose-dependent. The above results indicate that KY-0301 at a dose of 1.5 mg/kg or more can effectively inhibit the growth and proliferation of non-small cell lung cancer tissues in B-NDG mice, and the effect is dose-dependent.

### Example 49. KY-0301 effectively inhibits tumor growth in a pancreatic cancer PDX mouse model

1. 45 female B-NDG mice (8-10 weeks old) were prepared;
2. Pancreatic cancer tissues were cut into pieces of 3×3×3 mm and inoculated subcutaneously in the right anterior flank of B-NDG mice;
3. When the tumors grew to 200-300 mm³, the B-NDG mice were grouped into 3 groups with 6 mice in each group. The grouping and administration method are shown in the following table (Table 31):

**Table 31 Animal grouping and administration**

| **Group** | **Experimental Group** | **Number of Animals** | **Dosage** | **Administration Route** | **Administration Frequency** |
|---|---|---|---|---|---|
| 1 | PBS | 6 | NA | i.v. | QW×2 |
| 2 | KY-0301 | 6 | 2 mg/kg | i.v. | QW×2 |
| 3 | KY-0301 | 6 | 4 mg/kg | i.v. | QW×2 |

4. The mice were administered with PBS, KY-0301 at a dose of 2 mg/kg, and KY-0301 at a dose of 4 mg/kg via tail vein injection once a week for two consecutive weeks;
5. Tumor volume and body weight were measured twice a week, and the relationship between the changes in body weight and tumor volume of tumor-bearing mice and the administration time was recorded.
6. The tumor volume and tumor growth inhibition rate were calculated $Tumor volume \left(V\right) was calculated as \left(length\times {width}^{2}\right) / 2$ Tumor growth inhibition rate (TGI%) was calculated using the following formula: 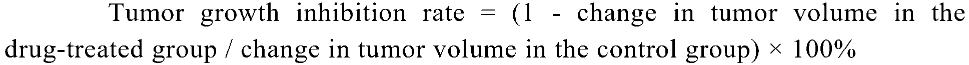 Tumor growth inhibition rate = (1 - change in tumor volume in the drug-treated group / change in tumor volume in the control group) × 100%

The experimental results (Fig. 45) indicate that in the pancreatic cancer PDX B-NDG mouse model, both 2 mg/kg and 4 mg/kg of KY-0301 could effectively inhibit the growth and proliferation of tumor tissues in mice. The tumor inhibition rates on Day 32 were 93.7% and 98.8% respectively, which is dose-dependent. The above results indicate that KY-0301 at a dose of 2 mg/kg or more can effectively inhibit the growth and proliferation of pancreatic cancer tissues in B-NDG mice, and the effect is dose-dependent.

### Example 50. Tumor-inhibiting effect of KY-0301 in the HCT116 xenograft tumor mouse model is better than that of the control product AZD9592-MMAE

1. 40 female Balb/c nude mice (4-5 weeks old) were prepared;
2. Human colon cancer HCT116 cells were inoculated subcutaneously into the right anterior flank of mice at a dose of 5×10⁶ cells per mouse;
3. When the tumors grew to 100-150 mm³, the Balb/c nude mice were grouped into 5 groups with 5 mice in each group. The grouping and administration method are shown in the following table (Table 32):

**Table 32 Animal grouping and administration**

| **Group** | **Experimental Group** | **Number of Animals** | **Dosage** | **Administration Route** | **Administration Frequency** |
|---|---|---|---|---|---|
| 1 | PBS | 5 | NA | i.v. | QW×2 |
| 2 | KY-0301 | 5 | 1.5 mg/kg | i.v. | QW×2 |
| 3 | KY-0301 | 5 | 3 mg/kg | i.v. | QW×2 |
| 4 | AZD9592-MMAE | 5 | 2.8 mg/kg | i.v. | QW×2 |
| 5 | AZD9592-MMAE | 5 | 5.6 mg/kg | i.v. | QW×2 |

4. The mice were administered with PBS, KY-0301 at a dose of 1.5mg/kg, KY-0301 at a dose of 3mg/kg, AZD9592-MMAE at a dose of 2.8mg/kg, and AZD9592-MMAE at a dose of 5.6mg/kg via tail vein injection once a week for two consecutive weeks;
5. Tumor volume and body weight were measured twice a week, and the relationship between the changes in body weight and tumor volume of tumor-bearing mice and the administration time was recorded;
6. The tumor volume and tumor growth inhibition rate were calculated $Tumor volume \left(V\right) was calculated as \left(length\times {width}^{2}\right) / 2$ Tumor growth inhibition rate (TGI%) was calculated using the following formula: 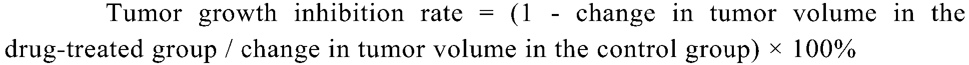 Tumor growth inhibition rate = (1 - change in tumor volume in the drug-treated group / change in tumor volume in the control group) × 100%

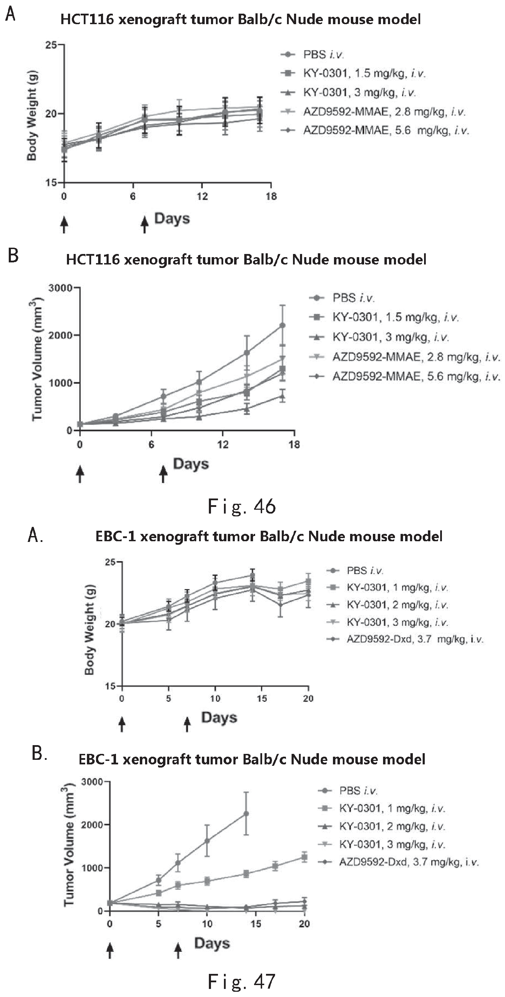

The experimental results indicate (Fig. 46) that in the Balb/c nude mouse model of colon cancer HCT116 xenograft, 1.5 mg/kg and 3 mg/kg of KY-0301, as well as 2.8 mg/kg and 5.6 mg/kg of AZD9592-MMAE could effectively inhibit the growth and proliferation of tumor cells in mice. The tumor inhibition rates on Day 17 were 41.3%, 67.0%, 32.2%, and 45.7% respectively. Both KY-0301 and AZD9592-MMAE exhibited dose-dependence. The molar dose of 1.5 mg/kg of KY-0301 (DAR = 4) is equal to that of 2.8 mg/kg of AZD9592-MMAE (DAR = 4), and the molar dose of 3 mg/kg of KY-0301 (DAR = 4) is equal to that of 5.6 mg/kg of AZD9592-MMAE (DAR = 4). The tumor inhibition rate of low-dose KY-0301 was higher than that of low-dose AZD9592-MMAE (41.3% > 32.2%), and the tumor inhibition rate of high-dose KY-0301 was higher than that of high-dose AZD9592-MMAE (67.0% > 45.7%).

The above results indicated that the growth and proliferation of colon cancer cells in mice is effectively inhibited by KY-0301 at a dose of 1.5 mg/kg or more in a dose-dependent manner. At equal molar doses and equal payloads, the tumor-inhibiting effect of KY-0301 is better than that of AZD9592-MMAE.

### Example 51. Tumor-inhibiting effect of KY-0301 in the EBC-1 xenograft tumor mouse model is better than that of the control product AZD9592-Dxd

1. 40 female Balb/c nude mice (4-5 weeks old) were prepared;
2. Human non-small cell lung cancer tissues were inoculated subcutaneously into the right anterior flank of mice at a dose of 5×10⁶ cells per mouse;
3. When the tumors grew to 100-200 mm³, the Balb/c nude mice were grouped into 5 groups with 5 mice in each group. The grouping and administration method are shown in the following table (Table 33):

**Table 33 Animal grouping and administration**

| **Group** | **Experimental Group** | **Number of Animals** | **Dosage** | **Administration Route** | **Administration Frequency** |
|---|---|---|---|---|---|
| 1 | PBS | 5 | NA | i.v. | QW×2 |
| 2 | KY-0301 | 5 | 1 mg/kg | i.v. | QW×2 |
| 3 | KY-0301 | 5 | 2 mg/kg | i.v. | QW×2 |
| 4 | KY-0301 | 5 | 3 mg/kg | i.v. | QW×2 |
| 5 | AZD9592-DXd | 5 | 3.7 mg/kg | i.v. | QW×2 |

4. The mice were administered with PBS, KY-0301 at a dose of 1 mg/kg, KY-0301 at a dose of 2 mg/kg, KY-0301 at a dose of 3 mg/kg, and AZD9592-DXd at a dose of 3.7 mg/kg via tail vein injection once a week for two consecutive weeks;
5. Tumor volume and body weight were measured twice a week, and the relationship between the changes in body weight and tumor volume of tumor-bearing mice and the administration time was recorded;
6. The tumor volume and tumor growth inhibition rate were calculated $Tumor volume \left(V\right) was calculated as \left(length\times {width}^{2}\right) / 2$ Tumor growth inhibition rate (TGI%) was calculated using the following formula: 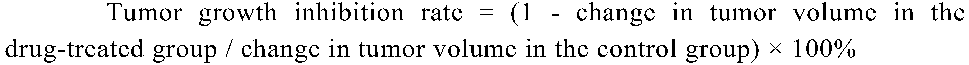 Tumor growth inhibition rate = (1 - change in tumor volume in the drug-treated group / change in tumor volume in the control group) × 100%

The experimental results indicate (Figure 47) that in the Balb/c nude mouse model of lung cancer EBC-1 xenograft, 1 mg/kg, 2 mg/kg, and 3 mg/kg of KY-0301, as well as a dose of 3.7 mg/kg of AZD9592-Dxd could effectively inhibit the growth and proliferation of tumor cells in mice. The tumor inhibition rates on Day 14 were 66.3%, 97.3%, 100.0%, and 96.3% respectively. The tumor-inhibiting effect of KY-0301 was dose-dependent. The molar dose of 2 mg/kg of KY-0301 (DAR = 4) is equal to that of 3.7 mg/kg of AZD9592-Dxd (DAR = 6). At the equal molar dose on Day 20, the average tumor volume in the 2 mg/kg of KY-0301 (DAR = 4) group was 116.0 mm³, showing a -35.8% growth compared with the initial tumor volume. While the average tumor volume in the 3.7 mg/kg of AZD9592-Dxd (DAR = 6) group was 221.5 mm³, showing a 22.0% growth compared with the initial tumor volume. The above results indicate that the growth and proliferation of non-small cell lung cancer cells in mice could is effectively inhibited KY-0301 at a dose of 1 mg/kg or more in a dose-dependent manner. At equal molar doses, the tumor-inhibiting effect of KY-0301 is better than that of AZD9592-Dxd.

### Example 52. KY-0301 demonstrates good safety in the pre-toxicological experiment on primates

Experimental animals: 4 rhesus monkeys, two males and two females
Grouping and administration: The 4 rhesus monkeys were randomly divided into two groups (two monkeys in each group, one male and one female). The groups include a low-dose group of KY-0301 (1/6 mg/kg, the dose was increased starting from D29) and a high-dose group (3 mg/kg)., the drug was administered once on D1 and D15 in low-and high-dose groups, with the administration doses being 1 mg/kg and 3 mg/kg respectively. The dose for the low-dose group was increased on D29 (from 1 mg/kg to 6 mg/kg), and the drug was administered once on D29 and D43 respectively;
Experimental results: No unscheduled death of the animals was observed during the experiment. No obvious abnormalities related to KY-0301 were found in clinical observations, body weight, food intake, coagulation, serum biochemistry, and immune function tests. The main abnormalities related to KY-0301 were as follows: at a dose of ≥ 3 mg/kg, a decrease in RBC, HGB, HCT, #RET, RET%, #NEU, #LYMP, and/or #MONO was observed in both male and female animals one week after drug administration (D7 or D35 or D49). In addition, an increase in WBC, #LYMP, and #MONO was also observed in both male and female animals two weeks after drug administration (D14 or D42). All the above-mentioned abnormalities were shown to have recovery or a recovery trend by the end of the observation period (D71). The highest non-severely toxic dose (HNSTD) of KY-0301 for both male and female animals administered by intravenous injection was determined to be 6 mg/kg. After drug administration on D1 (or D29), no gender difference was found in ADC and total antibody exposure (measured by AUC (0-t) and Cmax) within the dose range of 1-6 mg/kg.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

## Claims

1. A nanobody targeting MET, wherein the VHH chain of the nanobody comprises the following complementarity determination regions CDR1, CDR2 and CDR3:
a CDR1 as shown in SEQ ID NO: 13,
a CDR2 as shown in SEQ ID NO: 14, and
a CDR3 as shown in SEQ ID NO: 15.

2. The nanobody according to claim 1, wherein the VHH chain of the nanobody targeting MET has an amino acid sequence having ≥85%, ≥90%, ≥95%, ≥96%, ≥97%, ≥98%, or ≥99% homology to the amino acid sequence shown in SEQ ID NO: 1, or has an amino acid sequence as shown in SEQ ID NO: 1.

3. An antibody targeting MET, which comprises one or more VHH chain(s) of the nanobody targeting MET according to claim 1.

4. A nanobody targeting EGFR, wherein the VHH chain of the nanobody comprises the following complementarity determination regions CDR1, CDR2 and CDR3:
a CDR1 as shown in SEQ ID NO: 16,
a CDR2 as shown in SEQ ID NO: 17, and
a CDR3 as shown in SEQ ID NO: 18.

5. The nanobody according to claim 4, wherein the VHH chain of the nanobody targeting EGFR has an amino acid sequence having ≥85%, ≥90%, ≥95%, ≥96%, ≥97%, ≥98%, or ≥99% homology to the amino acid sequence shown in SEQ ID NO: 3, or has an amino acid sequence as shown in SEQ ID NO: 3.

6. An antibody targeting EGFR, which comprises one or more VHH chain(s) of the nanobody targeting EGFR according to claim 4.

7. A multi-specific antibody which comprises the VHH chain of the nanobody targeting MET according to claim 1 and the VHH chain of the nanobody targeting EGFR according to claim 4.

8. The multi-specific antibody according to claim 7, wherein the multi-specific antibody is a bispecific antibody targeting MET and EGFR, which has the structure shown in Formula II from N-terminus to C-terminus:
V1-L1-V2-H1-Fc1-L2-V3 II
V4-L3-V5-H2-Fc2-L4-V6 (II);
wherein,
V1, V2, V3, V4, V5, and V6 are each independently absent, the VHH chain of the nanobody targeting MET, or the VHH chain of the nanobody targeting EGFR,
at least one of V1, V2, and V3 is the VHH chain of the nanobody targeting MET, and at least one of V4, V5, and V6 is the VHH chain of the nanobody targeting EGFR;
L1, L2, L3, L4, L5, and L6 are each independently absent or a linker peptide;
H1 and H2 are each independently absent or an immunoglobulin hinge region;
Fc1 and Fc2 are each independently an immunoglobulin Fc fragment;
"∥" represents a disulfide bond or a knob-into-hole connection;
"-" represents a peptide bond.

9. The multi-specific antibody according to claim 8, wherein the bispecific antibody has the structure shown in the following formula IIa from N-terminus to C-terminus:
V1-L1-V2-H1-Fc1 I
V4-L3-V5-H2-Fc2 (IIa)
wherein,
one of V1 and V2 is the VHH chain of the nanobody targeting MET, and the other is absent or the VHH chain of the nanobody targeting EGFR;
one of V4 and V5 is the VHH chain of the nanobody targeting EGFR, and the other is absent or the VHH chain of the nanobody targeting MET;
the remaining elements are as defined in formula (II).

10. The multi-specific antibody according to claim 8, which comprises a first antigen-binding chain and a second antigen-binding chain, wherein
the amino acid sequence of the first antigen-binding chain is as shown in any one of SEQ ID NOs: 2, 6, 11 and 12; and/or
the amino acid sequence of the second antigen-binding chain is as shown in any one of SEQ ID NOs: 5 and 7-12.

11. The multi-specific antibody according to claim 10, wherein the bispecific antibody comprises a first antigen-binding chain shown in SEQ ID NO: 2 and/or a second antigen-binding chain shown in SEQ ID NO: 5 or 7.

12. An immunoconjugate which comprises:
(a) an antibody moiety, which is the nanobody targeting MET according to claim 1, the antibody targeting MET according to claim 3, the nanobody targeting EGFR according to claim 4, the antibody targeting EGFR according to claim 6, or the multi-specific antibody according to claim 7; and
(b) a coupling moiety coupled to the nanobody moiety, which is selected from the group consisting of a detectable label, a drug, and a combination thereof.

13. The immunoconjugate according to claim 12, wherein the coupling moiety is MMAE.

14. A pharmaceutical composition which comprises:
(a) the nanobody targeting MET according to claim 1, the antibody targeting MET according to claim 3, the nanobody targeting EGFR according to claim 4, the antibody targeting EGFR according to claim 6, the multi-specific antibody according to any one of claims 7-11, or the immunoconjugate according to claim 14;
(ii) a pharmaceutically acceptable carrier.
